# FASCICULE DE BREVET EUROPEEN

(11) **EP 1 732 938 B1**
(45) Date de publication et mention de la délivrance du brevet: **06.05.2009**
(21) Numéro de dépôt: 04805706.1
(22) Date de dépôt: 15.12.2004
(51) Int. Cl.: C07F 9/38, C07F 9/44, A61P 35/00

(54) **DERIVES ORGANOPHOSPHORES DES INDAZOLES ET LEUR UTILISATION COMME INHIBITEURS DES PROTEINES KINASE**
PHOSPHORORGANISCHE INDAZOLDERIVATE UND DEREN VERWENDUNG ALS PROTEINKINASEINHIBITOREN
ORGANOPHOSPHOROUS INDAZOLE DERIVATIVES AND USE THEREOF AS PROTEIN KINASE INHIBITORS

(30) Priorité: 17.12.2003 FR 0314778
(43) Date de publication de la demande: 20.12.2006
(73) Titulaire: Aventis Pharma S.A., 92160 Antony (FR)
(72) Inventeur: CHERRIER, Marie-Pierre, F-94200 IVRY SUR SEINE (FR); CLERC, François, F-92160 ANTONY (FR); COMMERÇON, Alain, F-94400 VITRY SUR SEINE (FR); MAILLIET, Patrick, F-94120 FONTENAY SOUS BOIS (FR); MINOUX, Hervé, F-94320 THIAIS (FR); FILOCHE-ROMMÉ, Bruno, F-94000 CRETEIL (FR)
(74) Mandataire: Rousseau, Pierrick Edouard
(86) Numéro de dépôt international: PCT/FR2004/003225
(87) Numéro de publication internationale: WO 2005/058923

(56) Documents cités:
- EP-A- 1 256 574
- WO-A-93/18008
- WO-A-03/064397
- WO-A-03/078402

## Description

La présente invention concerne notamment de nouveaux composés chimiques, particulièrement de nouveaux dérivés organophosphorés des indazoles, des compositions les contenant, et leur utilisation comme médicaments.

Plus particulièrement, l'invention concerne de nouveaux indazoles spécifiques présentant une activité anticancéreuse, via la modulation de l'activité de protéines, en particulier des kinases.

A ce jour, la plupart des composés commerciaux utilisés en chimiothérapie sont des cytotoxiques qui posent des problèmes importants d'effets secondaires et de tolérance par les patients. Ces effets pourraient être limités dans la mesure où les médicaments utilisés agissent sélectivement sur les cellules cancéreuses, à l'exclusion des cellules saines. Une des solutions pour limiter les effets indésirables d'une chimiothérapie peut donc consister en l'utilisation de médicaments agissant sur des voies métaboliques ou des éléments constitutifs de ces voies, exprimés majoritairement dans les cellules cancéreuses, et qui ne seraient pas ou peu exprimés dans les cellules saines.

Les protéines kinase sont une famille d'enzyme qui catalysent la phosphorylation de groupes hydroxy de résidus spécifiques de protéines tels que des résidus tyrosine, sérine ou thréonine. De telles phosphorylations peuvent largement modifier la fonction des protéines ; ainsi, les protéines kinases jouent un rôle important dans la régulation d'une grande variété de processus cellulaires, incluant notamment le métabolisme, la prolifération cellulaire, la différentiation cellulaire, la migration cellulaire ou la survie cellulaire. Parmi les différentes fonctions cellulaires dans lesquelles l'activité d'une protéine kinase est impliquée, certains processus représentent des cibles attractives pour traiter les maladies cancéreuses ainsi que d'autres maladies.

Ainsi, un des objets de la présente invention est de proposer des compositions ayant une activité anticancéreuse, en agissant en particulier vis-à-vis de kinases. Parmi les kinases pour lesquelles une modulation de l'activité est recherchée, Aurora 2 et Tie2 sont préférées.

De nombreuses protéines impliquées dans la ségrégation des chromosomes et l'assemblage du fuseau ont été identifiées dans la levure et la drosophile. La désorganisation de ces protéines conduit à la non-ségrégation des chromosomes et à des fuseaux monopolaires ou désorganisés. Parmi ces protéines, certaines kinases, dont **Aurora** et Ipl1, provenant respectivement de drosophile et de *S*. *cerevisiae*, sont nécessaires pour la ségrégation des chromosomes et la séparation du centrosome. Un analogue humain de Ipl1 de levure a été récemment cloné et caractérisé par différents laboratoires. Cette kinase, nommée aurora2, STK15 ou BTAK appartient à la famille des kinases à sérine/thréonine. Bischoff et al. ont montré que Aurora2 est oncogène, et est amplifié dans les cancers colorectaux humains (EMBO J, 1998, 17, 3052-3065). Cela a également été exemplifié dans des cancers impliquant des tumeurs épithéliales telles que le cancer du sein.

Parmi les autres kinases sur lesquelles des produits de l'invention peuvent agir, on peut citer FAK, KDR, Src, Tie2 et les kinases dépendant de cyclines (CDK).

**FAK** est une tyrosine kinase cytoplasmique jouant un rôle important dans la transduction du signal transmis par les intégrines, famille de récepteurs hétérodimériques de l'adhésion cellulaire. FAK et les intégrines sont localisés dans des structures périmembranaires appelées plaques d'adhérence. Il a été montré dans de nombreux types cellulaires que l'activation de FAK ainsi que sa phosphorylation sur des résidus tyrosine et en particulier son autophosphorylation sur la tyrosine 397 étaient dépendantes de la liaison des intégrines à leurs ligands extracellulaires et donc induites lors de l'adhésion cellulaire [Kornberg L, et al. J. Biol. Chem. 267(33): 23439-442. (1992)]. L'autophosphorylation sur la tyrosine 397 de FAK représente un site de liaison pour une autre tyrosine kinase, Src, via son domaine SH2 [Schaller et al. Mol. Cell. Biol. 14 :1680-1688.1994; Xing et al. Mol. Cell. Biol. 5 :413-421. 1994]. Src peut alors phosphoryler FAK sur la tyrosine 925, recrutant ainsi la protéine adaptatrice Grb2 et induisant dans certaines cellules l'activation de la voie ras et MAP Kinase impliquée dans le contrôle de la prolifération cellulaire [Schlaepfer et al. Nature; 372:786-791. 1994; Schlaepfer et al. Prog. Biophy. Mol. Biol. 71:435-478. 1999; Schlaepfer and Hunter, J. Biol. Chem. 272:13189-13195. 1997]. L'activation de FAK peut aussi induire la voie de signalisation jun NH2-terminal kinase (JNK) et résulter dans la progression des cellules vers la phase G1 du cycle cellulaire [Oktay et al., J. Cell. Biol.145 :1461-1469. 1999]. Phosphatidylinositol-3-OH kinase (P13-kinase) se lie aussi à FAK sur la tyrosine 397 et cette interaction pourrait être nécessaire à l'activation de P13-kinase [Chen and Guan, Proc. Nat. Acad. Sci. USA. 91: 10148-10152. 1994; Ling et al. J. Cell. Biochem. 73 :533-544. 1999]. Le complexe FAK/Src phosphoryle différents substrats comme la paxilline et p130CAS dans les fibroblastes [Vuori et al. Mol. Cell. Biol. 16: 2606-2613. 1996].

Les résultats de nombreuses études soutiennent l'hypothèse que les inhibiteurs de FAK pourraient être utiles dans le traitement du cancer. Des études ont suggéré que FAK puisse jouer un rôle important dans la prolifération et/ou la survie cellulaire *in vitro.* Par exemple, dans les cellules CHO, certains auteurs ont démontré que la surexpression de p125FAK conduit à une accélération de la transition G1 à S, suggérant que p125FAK favorise la prolifération cellulaire [Zhao J.-H et al. J. Cell Biol. 143:1997-2008. 1998]. D'autres auteurs ont montré que des cellules tumorales traitées avec des oligonucléotides anti-sens de FAK perdent leur adhésion et entrent en apoptose (Xu et al, Cell Growth Differ. 4:413-418. 1996). Il a également été démontré que FAK promeut la migration des cellules *in vitro.* Ainsi, des fibroblastes déficients pour l'expression de FAK (souris « knockout » pour FAK) présentent une morphologie arrondie, des déficiences de migration cellulaire en réponse à des signaux chimiotactiques et ces défauts sont supprimés par une réexpression de FAK [DJ. Sieg et al., J. Cell Science. 112:2677-91. 1999]. La surexpression du domaine C-terminal de FAK (FRNK) bloque l'étirement des cellules adhérentes et réduit la migration cellulaire *in vitro* [Richardson A. and Parsons J.T. Nature. 380:538-540. 1996]. La surexpression de FAK dans des cellules CHO, COS ou dans des cellules d'astrocytome humain favorise la migration des cellules. L'implication de FAK dans la promotion de la prolifération et de la migration des cellules dans de nombreux types cellulaires *in vitro,* suggère le rôle potentiel de FAK dans les processus néoplasiques. Une étude récente a effectivement démontré l'augmentation de la prolifération des cellules tumorales *in vivo* après induction de l'expression de FAK dans des cellules d'astrocytome humain [Cary L.A. et al. J. Cell Sci. 109:1787-94. 1996; Wang D et al. J. Cell Sci. 113:4221-4230. 2000]. De plus, des études immunohistochimiques de biopsies humaines ont démontré que FAK était surexprimé dans les cancers de la prostate, du sein, de la thyroïde, du colon, du mélanome, du cerveau et du poumon, le niveau d'expression de FAK étant directement corrélé aux tumeurs présentant le phénotype le plus agressif [Weiner TM, et al. Lancet. 342(8878):1024-1025. 1993 ; Owens et al. Cancer Research. 55:2752-2755. 1995; Maung K. et al. Oncogene. 18:6824-6828. 1999; Wang D et al. J. Cell Sci. 113:4221-4230. 2000].

**KDR** (Kinase insert Domain Receptor) aussi appelée VEGF-R2 (Vascular Endothelial Growth Factor Receptor 2), est exprimé uniquement dans les cellules endothéliales. Ce récepteur se fixe au facteur de croissance angiogénique VEGF, et sert ainsi de médiateur à un signal transductionnel via l'activation de son domaine kinase intracellulaire. L'inhibition directe de l'activité kinase de VEGF-R2 permet de réduire le phénomène d'angiogénèse en présence de VEGF exogène (Vascular Endothelial Growth Factor: Facteur de croissance vasculaire endothélial) (Strawn et al., Cancer Research, 1996, vol. 56, p.3540-3545). Ce processus a été démontré notamment à l'aide de mutants VEGF-R2 (Millauer et al., Cancer Research, 1996, vol. 56, p.1615-1620). Le récepteur VEGF-R2 semble n'avoir aucune autre fonction chez l'adulte que celle liée à l'activité angiogénique du VEGF. Par conséquent, un inhibiteur sélectif de l'activité kinase du VEGF-R2 ne devrait démontrer que peu de toxicité.

En plus de ce rôle central dans le processus dynamique angiogénique, des résultats récents suggèrent que l'expression de VEGF contribue à la survie des cellules tumorales après des chimio- et radio-thérapies, soulignant la synergie potentielle d'inhibiteurs de KDR avec d'autres agents (Lee et al. Cancer Research, 2000, vol. 60, p.5565-5570).

**Tie-2** (TEK) est un membre d'une famille de récepteurs à tyrosine kinase, spécifique des cellules endothéliales. Tie2 est le premier récepteur à activité tyrosine kinase dont on connaît à la fois l'agoniste (angiopoïetine 1 ou Ang1) qui stimule l'autophosphorylation du récepteur et la signalisation cellulaire [S. Davis et al (1996) Cell 87, 1161-1169] et l'antagoniste (angiopoïetine 2 ou Ang2) [P.C. Maisonpierre et al. (1997) Science 277, 55-60]. L'angiopoïetine 1 peut synergiser avec le VEGF dans les derniers stades de la néo-angio-génèse [AsaharaT. Circ. Res.(1998) 233-240]. Les expériences de knock-out et les manipulations transgéniques de l'expression de Tie2 ou de Ang1 conduisent à des animaux qui présentent des défauts de vascularisation [D.J. Dumont et al (1994) Genes Dev. 8, 1897-1909 *et* C. Suri (1996) Cell 87, 1171-1180]. La liaison d'Ang1 à son récepteur conduit à l'autophosphorylation du domaine kinase de Tie2 qui est essentielle pour la néovascularisation ainsi que pour le recrutement et l'interaction des vaisseaux avec les péricytes et les cellules musculaires lisses; ces phénomènes contribuant à la maturation et la stabilité des vaisseaux nouvellement formés [P. C. Maisonpierre et al (1997) Science 277, 55-60]. Lin et al (1997) J. Clin. Invest. 100, 8: 2072-2078 *et* Lin P. (1998) PNAS 95, 8829-8834, ont montré une inhibition de la croissance et de la vascularisation tumorale, ainsi qu'une diminution des métastases de poumon, lors d'infections adénovirales ou d'injections du domaine extracellulaire de Tie-2 (Tek) dans des modèles de xénogreffes de tumeur du sein et de mélanome.
Les inhibiteurs de Tie2 peuvent être utilisés dans les situations où une néovascularisation se fait de façon inappropriée (c'est-à-dire dans la rétinopathie diabétique, l'inflammation chronique, le psoriasis, le sarcome de Kaposi, la néovascularisation chronique due à la dégénération maculaire, l'arthrite rhumatoïde, l'hémoangiome infantile et les cancers).

La progression du cycle cellulaire est souvent gérée par des kinases dépendantes de cyclines **(CDK)** qui sont activées par une balance dans la famille des cyclines, activation qui se termine par la phosphorylation de substrats et finalement par la division cellulaire. En plus les inhibiteurs endogènes des CDK qui sont activés (famille des INK4 et des KIP/CIP) régulent de façon négative l'activité des CDK. La croissance des cellules normales est due à une balance entre les activateurs des CDK (les cyclines) et les inhibiteurs endogènes des CDK. Dans plusieurs types de cancers, l'expression aberrante ou l'activité de plusieurs composants du cycle cellulaire a été décrite.

La cycline E active la kinase Cdk2 qui agit ensuite pour phosphoryler pRb résultant en un engagement dans la division cellulaire irréversible et une transition vers la phase S (PL Toogood, Medicinal Research Reviews (2001), 21(6); 487-498), il est également possible selon ces auteurs que les kinases CDK2 et CDK3 soient nécessaires pour la progression dans la phase G1 et l'entrée en phase S. Lors de la formation de complexe avec la cycline E, elles maintiennent l'hyperphosphorylation de pRb pour aider la progression de la phase G1 en phase S. Dans les complexes avec la Cycline A, CDK2 joue un rôle dans l'inactivation de E2F et est nécessaire pour la réalisation de la phase S (TD. Davies et al. (2001) Structure 9, 389-3).

Le complexe CDK1/cycline B régule la progression du cycle cellulaire entre la phase G2 et la phase M. La régulation négative du complexe CDK/Cycline B empêche les cellules normales d'entrer en phase S avant que la phase G2 ait été correctement et complètement réalisée. (K.K. Roy and E.A. Sausville Current Pharmaceutical Design, 2001, 7, 1669-1687.

Un niveau de régulation de l'activité des CDK existe. Les activateurs de cycline dépendantes kinases (CAK) ont une action positive de régulation des CDK. CAK phosphoryle les CDK sur le résidu thréonine pour rendre l'enzyme cible totalement active.

La présence de défauts dans les molécules intervenant sur le cycle cellulaire entraîne l'activation des CDK et la progression du cycle, il est normal de vouloir inhiber l'activité des enzymes CDK pour bloquer la croissance cellulaire des cellules cancéreuses.

La présente invention concerne de nouveaux dérivés organophosphorés d'indazoles.

Elle concerne également l'utilisation desdits dérivés pour la préparation d'un médicament destiné au traitement de l'homme.

Parmi l'art antérieur connu à ce jour décrivant des 5-phospho-indazoles, on peut citer la demande de brevet publiée sous le numéro WO93/18008 qui décrit des dérivés de formule générale suivante : 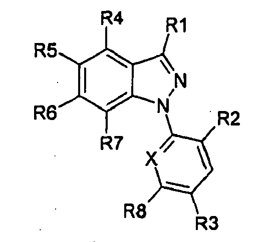 dans laquelle : X = N, CR₁₄ (R₁₄ = H, alkyl...); R₁ = H ou halogène; R₂ = H, NO₂, halogène, alkyle...; R₃ = H, halogène, haloalkyle, haloalkoxy, CN, NH₂: R₄-R₆ = H, NO₂, halo, alkyle, ..., alkylsulfonamido,..., P(=L)(Q)(M); L = O, S; M, Q = alkoxy, alkyl, (alkyl)ₙamino, OH, H, akenyloxy, (alkenyl)ₙamino, alkynyloxy, (alkynyl)ₙamino; R₇ = H, halo, alkyl, NO₂, et R₈ = H, halogène.

Ces composés sont utilisés comme herbicides.

La demande internationale WO 03/078402 décrit des composés anticancéreux de formule :

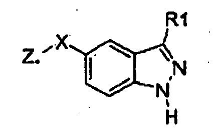

La demande internationale WO 03/064397 décrit des composés anticancéreux de formule

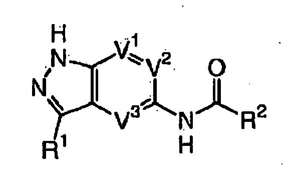

La demande européenne EP 1256574 décrit des composés de formule Het-X-Z inhibiteurs de l'enzyme Rho kinase pour lesquels Het peut représenter un cycle indazole.

Dans aucune de ces demandes, le cycle indazole est porteur en position 5 du motif-P(=O)RR2 tel qu'envisagé dans la présente demande.

Les composés revendiqués dans cette demande ont une application en agronomie alors que les composés de la présente invention ont une application phamaceutique.

Selon un premier aspect, l'invention concerne des produits de formule générale (I) suivante: 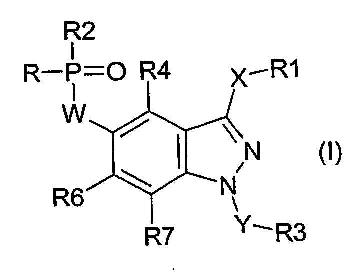 dans Laquelle:
- W représente un groupe choisi parmi une liaison covalente ou O;
- X représente une liaison covalente, un groupe -C=O-NRₐ-, NRₐ-C=O,
- (CH₂)ₙ-, -CH=CH-, -C≡C-, -NRₐ-, S, O, -SO₂-, -SO, -CO, -COO dans lequel Rₐ représente H ouun groupe (C₁-C₄)alkyle qui peut éventuellement former un cycle avec R₁, et dans lequel n = 0, 1, 2, 3, 4, 5, 6, 7, 8, 9, 10, 11, ou 12 ;
- R₁ représente H (sauf quand X = -SO₂-, -SO-), alkyle, cycloalkyle, aryle, hétéroaryle ;
- R et R₂, identiques ou différents représentent H ou un groupe choisi parmi les radicaux alkyle, cycloalkyle, hétérocycloalkyle, aryle, hétéroaryle, hydroxy, alkoxy et aryloxy ;
- Y représente une liaison covalente ou un radical choisi parmi: -C=O-NRₐ-,
- C=O-O-, -C=O-, -(CH₂)ₙ-, -SO₂-, dans lequel Rₐ est sélectionné dans le groupe constitué par H, (C₁-C₄)alkyle, et (C₁-C₄)alkyle lié à R₃ pour former un cycle;
- R₃ est sélectionné dans le groupe constitué par H (sauf quand Y est -C=O-O-, ou -SO₂-), alkyle, cycloalkyle, aryle, et hétéroaryle ;
- R₄, R₆ et R₇, identiques ou différents, peuvent être indépendamment choisis parmi : H, halogène, (C₁-C₄)alkyle, (C₁-C₄)alkoxy, cyano, -N(R_{b})R_{c}, -C=ON(R_{b})R_{c}, -N(R_{b})-CO-R_{c}, dans lequel R_{b} et R_{c} sont indépendamment choisis parmi H, (C₁-C₄)alkyle, et (C₃-C₆)cycloalkyle ;
   à l'exception des produits suivants : 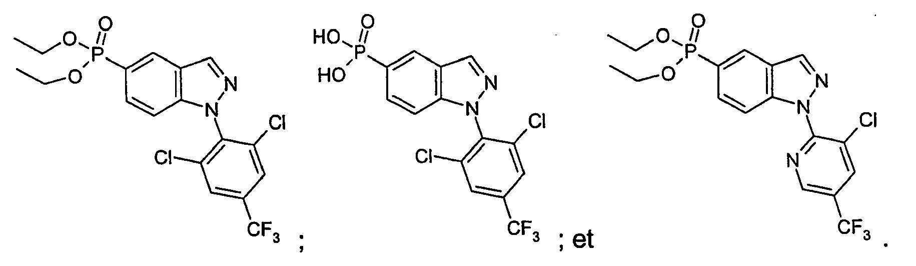 W est de préférence O.

Des radicaux aryle et hétéroaryle préférés sont indépendamment choisis parmi :
(i) des radicaux monocycliques comportant de zéro à quatre hétéroatomes choisis parmi O, N et S, et
(ii) des radicaux bicycliques condensés comprenant :
   (a) un radical monocyclique comportant 5, 6, 7 ou 8 chaînons et comportant de zéro à quatre hétéroatomes choisis parmi O, N et S, condensé à
   (b) un autre cycle comportant 5 ou 6 chaînons, et comportant de zéro à trois hétéroatomes choisis parmi O, N et S.

Plus préférentiellement, les radicaux aryle ou hétéroaryle sont indépendamment sélectionnés dans le groupe constitué par : phényle, pyridyle, pyrimidyle, triazinyle, pyrrolyle, imidazolyle, thiazolyle, furyle, thiényle, indolyle, indazolyle, azaindazolyle, isobenzofuranyle, isobenzo-thiényle, benzimidazolyle, benzoxazolyle, benzothiazolyle, arylvinylène, arylamido, arylcarboxamide, aralkylamine, quinoleyle, isoquinoleyle, cinnolyle, quinazolyle, naphtyridyle, triazolyle ou tétrazolyle.

Très préférentiellement, les radicaux aryle ou hétéroaryle sont indépendamment sélectionnés dans le groupe constitué par : phényle, pyrrolyle, indolyle et arylvinylène.

L'invention est particulièrement avantageusement mise en oeuvre lorsque X représente une liaison covalente et R₁ représente un radical hétérocyclique notamment indolyle pour la définition des produits de formule générale (I).

Un substituant R₂ préféré est un radical (C₁-C₄)alkyle.

De préférence, Y est avantageusement une liaison, et R₃ est H.

Selon un second aspect, l'invention concerne un produit de formule générale (I) suivante : 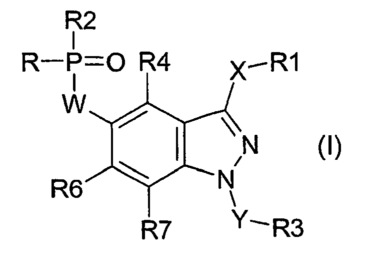 dans laquelle :
- W représente un groupe choisi parmi une liaison covalente ou O;
- X représente une liaison covalente, un groupe -C=O-NRₐ-, NRₐ-C=O,
- (CH₂)ₙ-, -CH=CH-, -C≡C-, -NRₐ-, S, O, -SO₂-, -SO, -CO, -COO dans lequel Rₐ représente H or un groupe alkyle (C₁-C₄) qui peut éventuellement former un cycle avec R1, et dans lequel n est choisi dans l'intervalle [0 à 12], ces deux bornes incluses;
- R₁ représente H (sauf quand X = -SO₂-, -SO-), alkyle, cycloalkyle, aryle, hétéroaryle ;
- R et R₂, identiques ou différents représentent H ou un groupe choisi parmi les radicaux alkyle, cycloalkyle, aryle, hétéroaryle, hydroxy, alkoxy et aryloxy ;
- Y représente une liaison covalente ou un radical choisi parmi: -C=O-NRₐ-,
- C=O-O-, -C=O-, -(CH₂)ₙ-, -SO₂-, dans lequel Rₐ est sélectionné dans le groupe constitué par H, (C₁-C₄)alkyle, et (C₁-C₄)alkyle lié à R₃ pour former un cycle 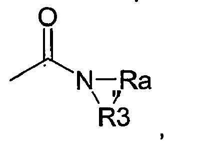
- R₃ est sélectionné dans le groupe constitué par H (sauf quand Y=C=O-O, SO₂), alkyle, cycloalkyle, aryle, et hétéroaryle ;
- R₄, R₆ et R₇, identiques ou différents, peuvent être indépendamment choisis parmi : H, halogène, (C₁-C₄)alkyle, (C₁-C₄)alkoxy, cyano, -N(R_{b})R_{c}, -C=ON(R_{b})R_{c}, -N(R_{b})-CO-R_{c}, dans lequel R_{b} et R_{c} sont indépendamment choisis parmi H, (C₁-C₄)alkyle, et (C₃-C₆)cycloalkyle ;
   en tant que médicament.

On peut citer parmi les composés répondant à la formule (I) les composés suivants :
1) Methyl-phosphonic acid methyl ester 3-[5-(2-morpholin-4-yl-ethoxy)-1H-indol-2-yl]-1 H-indazol-5-yl ester
2) Methyl-phosphonic acid methyl ester 3-{5-[2-(4-methyl-piperazin-1-yl)-ethoxy]-1H-indol-2-yl}-1H-indazol-5-yl ester
3) Phenyl-phosphonic acid methyl ester 3-thiophen-2-yl-1 H-indazol-5-yl ester
4) (2-Methanesulfonyl-phenyl)-phosphonic acid 3-(1H-indol-2-yl)-1H-indazol-5-yl ester methyl ester
5) Propyl-phosphonic acid 3-(1 H-indol-2-yl)-1 H-indazol-5-yl ester methyl ester
6) tert-Butyl-phosphonic acid 3-(1H-indol-2-yl)-1H-indazol-5-yl ester methyl ester
7) Cyclohexyl-phosphonic acid 3-(1H-indol-2-yl)-1H-indazol-5-yl ester methyl ester
8) (2-Methoxy-phenyl)-phosphonic acid 3-(1H-indol-2-yl)-1H-indazol-5-yl ester methyl ester
9) (2-Methylsulfanyl-phenyl)-phosphonic acid 3-(1H-indol-2-yl)-1H-indazol-5-yl ester methyl ester
10) (2,6-Dimethyl-phenyl)-phosphonic acid 3-(1H-indol-2-yl)-1H-indazol-5-yl ester methyl ester
11) (2-Trifluoromethoxy-phenyl)-phosphonic acid 3-(1 H-indol-2-yl)-1H-indazol-5-yl ester methyl ester
12) Thiophen-2-yl-phosphonic acid 3-(1H-indol-2-yl)-1H-indazol-5-yl ester methyl ester
13) Furan-2-yl-phosphonic acid 3-(1 H-indol-2-yl)-1 H-indazol-5-yl ester methyl ester
14) Methyl-phosphonic acid methyl ester 3-((E)-styryl)-1H-indazol-5-yl ester
15) Phenyl-phosphonic acid methyl ester 3-((E)-styryl)-1H-indazol-5-yl ester
16) Phenyl-phosphonic acid methyl ester 3-thiophen-2-yl-1H-indazol-5-yl ester
17) Methyl-phosphonic acid methyl ester 3-thiophen-2-yl-1 H-indazol-5-yl ester
18) Methyl-phosphonic acid methyl ester 3-(1H-pyrrol-2-yl)-1H-indazol-5-yl ester
19) Methyl-phosphonic acid 3-benzo[b]thiophen-2-yl-1H-indazol-5-yl ester methyl ester
20) Phenyl-phosphonic acid 3-benzo[b]thiophen-2-yl-1H-indazol-5-yl ester methyl ester
21) Phenyl-phosphonic acid 3-(5-methoxy-1H-pyrrolo[3,2-b]pyridin-2-yl)-1H-indazol-5-yl ester methyl ester
22) Methyl-phosphonic acid 3-(5-methoxy-1H-pyrrolo[3,2-b]pyridin-2-yl)-1H-indazol-5-yl ester methyl ester

| Produit | Structure |
|---|---|
| 1 | 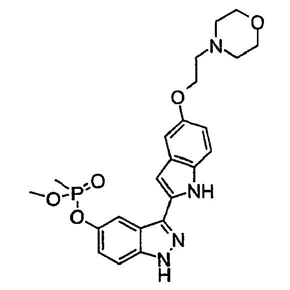 |
| 2 | 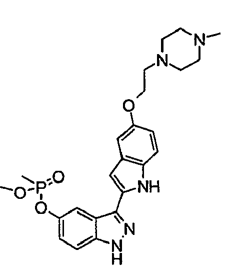 |
| 3 | 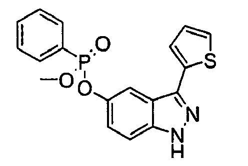 |
| 4 | 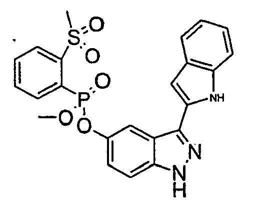 |
| 5 | 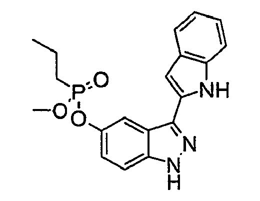 |
| 6 | 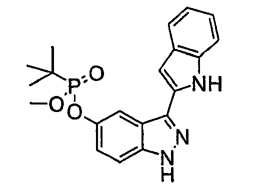 |
| 7 | 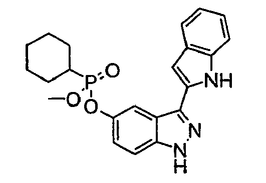 |
| 8 | 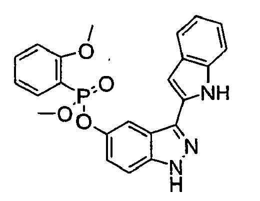 |
| 9 | 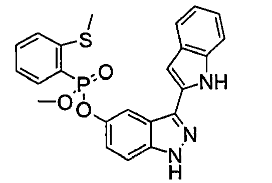 |
| 10 | 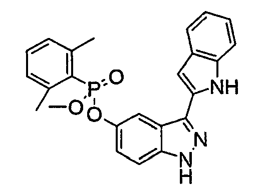 |
| 11 | 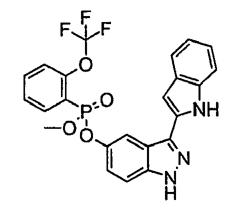 |
| 12 | 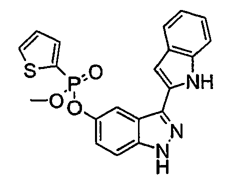 |
| 13 | 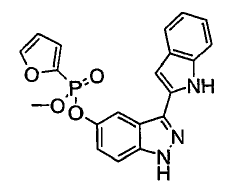 |
| 14 | 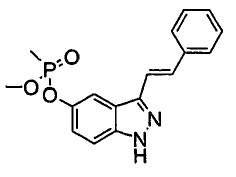 |
| 15 | 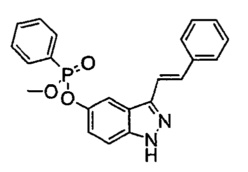 |
| 16 | 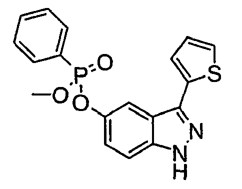 |
| 17 | 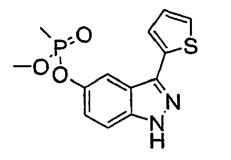 |
| 18 | 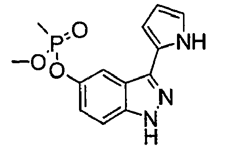 |
| 19 | 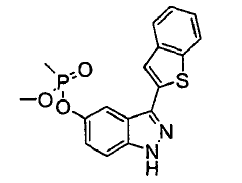 |
| 20 | 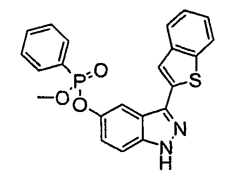 |
| 21 | 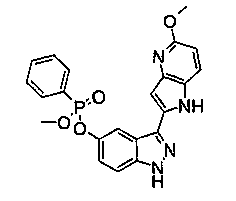 |
| 22 | 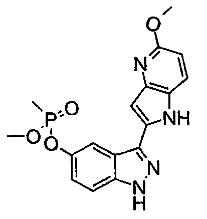 |

Un des procédés de préparation des composés selon l'invention peut être schématisé de la façon suivante :

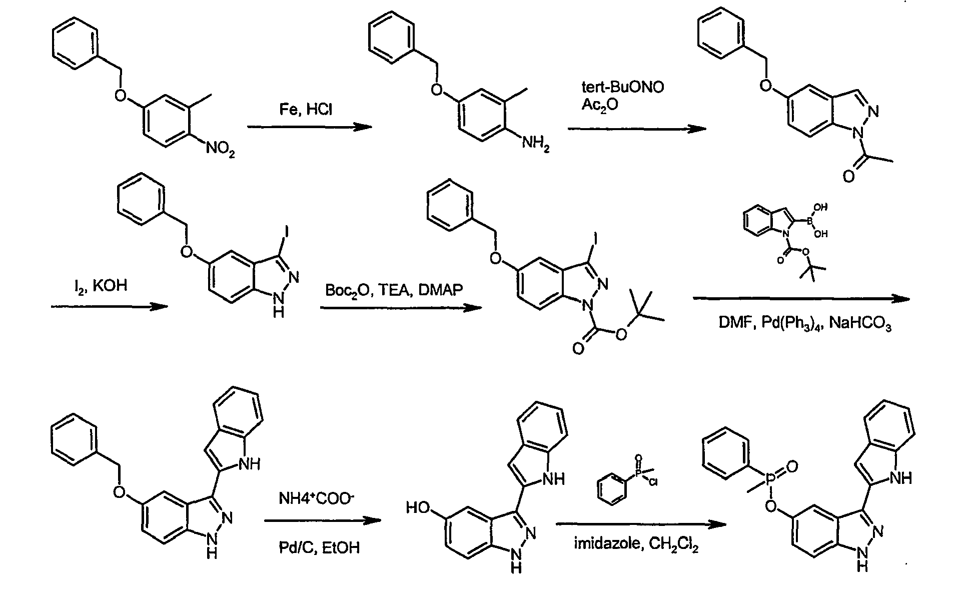

Les composés selon l'invention sont utilisables en thérapie humaine et plus particulièrement dans le traitement du cancer, plus particulièrement des cancers sensibles aux inhibiteurs d'Aurora-2 et à Tie2.

La présente invention sera plus complètement décrite à l'aide des exemples suivants qui ne doivent pas être considérés comme limitatifs de l'invention.

### Analyses LC/MS

Les analyses LC/MS ont été réalisées sur un appareil Micromass modèle LCT relié à un appareil HP 1100. L'abondance des produits a été mesurée à l'aide d'un détecteur à barrette de diodes HP G1315A sur une gamme d'onde de 200-600 nm et un détecteur à dispersion de lumière Sedex 65. L'acquisition des spectres de masses a été réalisée sur une gamme de 180 à 800. Les données ont été analysées en utilisant le logiciel Micromass MassLynx. La séparation a été effectuée sur une colonne Hypersil BDS C18, 3 µm (50 x 4.6 mm), en éluant par un gradient linéaire de 5 à 90 % d'acétonitrile contenant 0,05 % (v/v) d'acide trifluoroacétique (TFA) dans l'eau contenant 0,05 % (v/v) TFA en 3,5 minutes à un débit de 1 mL/mn. Le temps total d'analyse, incluant la période de rééquilibration de la colonne, est de 7 minutes.

### Purification par LC/MS préparative

Les produits ont été purifiés par LC/MS en utilisant un système Waters FractionsLynx composé d'une pompe à gradient Waters modèle 600, d'une pompe de régénération Waters modèle 515, d'une pompe de dilution Waters Reagent Manager, d'un auto-injecteur Waters modèle 2700, de deux vannes Rheodyne modèle LabPro, d'un détecteur à barrette de diodes Waters modèle 996, d'un spectromètre de masse Waters modèle ZMD et d'un collecteur de fractions Gilson modèle 204. Le système est contrôlé par du logiciel Waters FractionLynx. La séparation a été effectuée alternativement sur deux colonnes Waters Symmetry (C₁₈, 5 µM, 19x50 mm, référence catalogue 186000210), une colonne étant en cours de régénération par un mélange eau/acétonitrile 95/5 (v/v) contenant 0,07 % (v/v) d'acide trifluoroacétique, pendant que l'autre colonne était en cours de séparation. L'élution des colonnes a été effectuée en utilisant un gradient linéaire de 5 à 95 % d'acétonitrile contenant 0,07 % (v/v) d'acide trifluoroacétique dans l'eau contenant 0,07 % (v/v) d'acide trifluoroacétique, à un débit de 10 mL/mn. A la sortie de la colonne de séparation, un millième de l'effluent est séparé par un LC Packing Accurate, dilué à l'alcool méthylique à un débit de 0,5 mL/mn et envoyé vers les détecteurs, à raison de 75 % vers le détecteur à barrette de diodes, et les 25 % restants vers le spectromètre de masse. Le reste de l'effluent (999/1000) est envoyé vers le collecteur de fractions où le flux est éliminé tant que la masse du produit attendu n'est pas détectée par le logiciel FractionLynx. Les formules moléculaires des produits attendus sont fournies au logiciel FractionLynx qui déclenche la collecte du produit quand le signal de masse détecté correspond à l'ion [M+H]⁺ et/ou au [M+Na]⁺. Dans certains cas, dépendant des résultats de LC/MS analytique, quand un ion intense correspondant à [M+2H]⁺⁺ a été détecté, la valeur correspondant à la moitié de la masse moléculaire calculée (MW/2) est aussi fournie au logiciel FractionLynx. Dans ces conditions, la collecte est aussi déclenchée quand le signal de masse de l'ion [M+2H]⁺⁺ et/ou [M+Na+H]⁺⁺ sont détectés. Les produits ont été collectés en tube de verre tarés. Après collecte, les solvants ont été évaporés, dans un évaporateur centrifuge Savant AES 2000 ou Genevac HT8 et les masses de produits ont été déterminées par pesée des tubes après évaporation des solvants.

Purification par chromatographie flash: les produits bruts sont purifiés par chromatographie flash sur silice de granulométrie 15-35 µm sous une pression d'argon de 0.5 bar. Les fractions correspondant au produit attendu sont rassemblées et concentrées sous pression réduite à l'évaporateur rotatif.

L'intermédiaire A 5-Benzyloxy-3-iodo-indazole-1-carboxylic acid tert-butyl ester a été préparé en 4 étapes, selon le schéma 1.

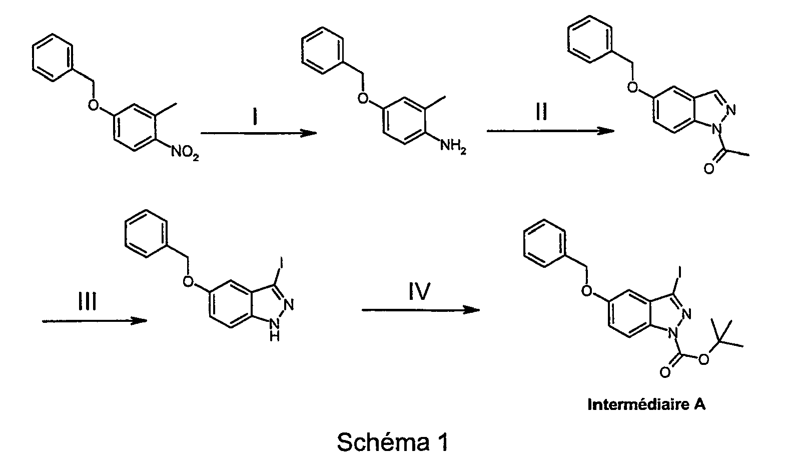

### Etape I : préparation de 4-benzyloxy-2-methyl-phenylamine

Une solution de 190 ml d'acide chlorhydrique concentré dans 300 ml d'éthanol est additionnée goutte à goutte sur un mélange de 50 g de 4-benzyloxy-2-methyl-1-nitro-benzene et de 46 g de zinc. La solution est refroidie aux alentours de 45°C par un bain de glace tout au long de la coulée. Le milieu est agité pendant 3 heures à température ambiante. Le pH de la solution est ajusté autour de pH 8 par ajout de 500 ml d'une solution saturée de carbonate de potassium. Le précipité est filtré et lavé par 5 x 500 ml d'acétate d'éthyle. Les phases organiques sont réunies et lavées par 2 x 1 litre d'eau distillée, puis par 1 litre d'une solution saturée de chlorure de sodium. Après séchage sur sulfate de magnésium, le solvant est évaporé sous pression réduite à l'évaporateur rotatif. Le brut réactionnel est purifié par chromatographie flash (silice 35-70 µm), éluant : acétate d'éthyle/cyclohexane 80 : 20 ; 75 : 25 ; 70 :30. 30.81 g de 4-benzyloxy-2-methyl-phenylamine sont isolés.

Spectre de R.M.N. ¹H (300 MHz, (CD₃)₂SO d6, δ en ppm) : 2,04 (s : 3H) ; 4,40 (s large : 2H) ; 4,95 (s : 2H) ; 6,55 (d, J = 8,5 Hz : 1 H) ; 6,61 (dd, J = 8,5 et 2,5 Hz : 1 H) ; 6,68 (d, J = 2,5 Hz : 1 H) ; de 7,25 à 7,55 (mt : 5H).

### Etape II : préparation de 1-(5-benzyloxy-indazol-1-yl)-ethanone

10.5 ml d'anhydride acétique sont coulés sur une solution de 7.14 g 4-benzyloxy-2-methyl-phenylamine dans 26 ml de toluène. Le milieu est chauffé autour de 90°C et 9.28 ml de tert-butyl nitrite sont coulés goutte à goutte sur la solution. Le milieu réactionnel est chauffé aux alentours de 90°C pendant deux heures. Le brut réactionnel est concentré à sec à l'évaporateur rotatif. Le solide est repris dans l'acétate d'éthyle puis filtré, il est rincé à l'éther isopropylique. 3.41 g de 1-(5-benzyloxy-indazol-1-yl)-ethanone sont recueillis.

Spectre de R.M.N. ¹H (300 MHz, (CD₃)₂SO d6, δ en ppm) : 2,72 (s : 3H) ; 5,21 (s large : 2H) ; 7,34 (dd, J = 9 et 2,5 Hz : 1 H) ; de 7,35 à 7,50 (mt : 3H) ; 7,47 (d, J = 2,5 Hz : 1 H) ; 7,51 (dd large, J = 7,5 et 1,5 Hz : 2H) ; 8,23 (d, J = 9 Hz : 1 H) ; 8,39 (d, J = 1 Hz : 1 H).

### Etape III : procédure A - préparation de 5-benzyloxy-3-iodo-1 H-indazole

A une solution de 28.24 g de 1-(5-benzyloxy-indazol-1-yl)-ethanone dans 620 ml de diméthylformamide sont ajoutés 68.84 g d'iode puis 23 g d'hydroxyde de potassium. Le milieu réactionnel est agité à température ambiante pendant environ 3 heures. 23 g d'hydroxyde de potassium sont ajoutés et le milieu est agité à température ambiante pendant 48 heures. Le milieu est traité par 600 ml d'une solution de thiosulfate de sodium (100 g de thiosulfate de sodium dans 250 ml d'eau distillée), 600 ml d'eau distillée et 1 litre d'acétate d'éthyle. Le milieu est agité quelques minutes puis décanté. La phase aqueuse est extraite par 4 x 600 ml d'acétate d'éthyle. Les phases organiques rassemblées sont lavées par 1 litre d'une solution saturée de chlorure de sodium, puis séchées sur sulfate de magnésium. Le solvant est évaporé sous pression réduite à l'évaporateur rotatif. Le brut réactionnel est repris dans du dichlorométhane, le solide est filtré, rincé au dichlorométhane et à l'éther éthylique. 20.8 g de 5-benzyloxy-3-iodo-1H-indazole sont recueillis.

Spectre de R.M.N. ¹H (300 MHz, (CD₃)₂SO d6, δ en ppm) : 5,19 (s large : 2H) ; 6,90 (d, J = 2 Hz : 1H); 7,18 (dd, J = 9 et 2 Hz : 1 H) ; 7,35 (t large, J = 7,5 Hz: 1 H) ; 7,43 (t large, J = 7,5 Hz : 2H) ; 7,50 (d, J = 9 Hz : 1 H) ; 7,52 (d large, J = 7,5 Hz : 2H) ; de 13,00 à 13,70 (mf très étalé : 1 H).

LC/MS : [M+H]⁺=351.10 ; temps de rétention : 3.97 minutes.

### Etape IV: procédure B - Préparation de 5-benzyloxy-3-iodo-indazole-1-carboxylic acid tert-butyl ester

A une solution de 19.54 g 5-benzyloxy-3-iodo-1 H-indazole, 36.50 g de di-tert-butyldicarbonate, 23.30 ml de triéthylamine dans 550 ml de dichlorométhane sont ajoutés 1.70 g de 4-diméthylaminopyridine. Un fort dégagement gazeux est observé. La solution est agitée une nuit à température ambiante. La phase organique est lavée par 2 x 500 ml d'eau distillée, séchée sur sulfate de magnésium, filtrée et concentrée sous pression réduite à l'évaporateur rotatif. Le solide brut est repris dans l'acétonitrile. Le solide est filtré, rincé à l'acétonitrile et à l'éther éthylique. 19.43 g de 5-benzyloxy-3-iodo-indazole-1-carboxylic acid tert-butyl ester sont recueillis. Le filtrat est purifié par chromatographie flash (silice 70-200 µm), éluant: acétate d'éthyle/cyclohexane 3/97. 3.05 g de 5-Benzyloxy-3-iodo-indazole-1-carboxylic acid tert-butyl ester sont recueillis.

LC/MS : : [M+H]+=451.08 ; temps de rétention : 4.91 minutes.

L'intermédiaire B 5-benzyloxy-3-(1H-indol-2-yl)-1H-indazole a été préparé en deux étapes à partir de l'intermédiaire A selon le schéma 2 :

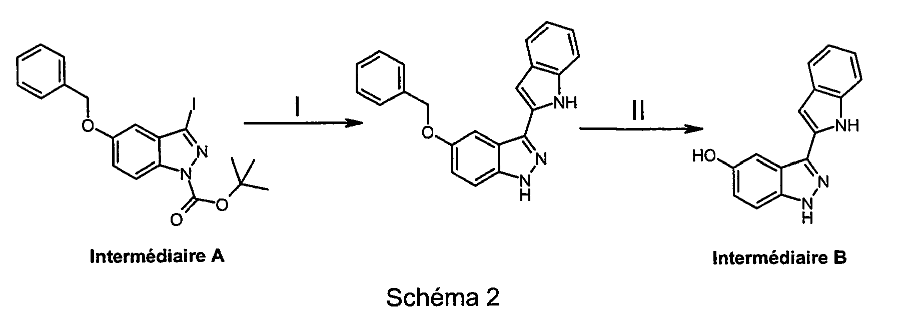

### Procédure C

Etape la : Préparation de 2-[4-(1-tert-butoxycarbonyl-2,3-dihydro-1H-indol-2-yl)-1,3,2,4-dioxadiboretan-2-yl]-indole-1-carboxylic acid tert-butyl ester selon la procédure décrite dans l'article de E. Vasquez, J. Org. Chem., 67, 7551-7552 (2002).

A une solution de 13 g de N-Boc indole dans 50 ml de THF anhydre sont additionnés goutte à goutte 21 ml de triisopropylborate. Le milieu réactionnel est refroidi aux alentours de 5°C. 50 ml d'une solution de LDA1.5M dans le THF sont additionnés goutte à goutte de façon à maintenir la température du milieu autour de 5°C. La solution est agitée pendant 90 minutes à cette température puis le milieu est traité par 40 ml d'une solution aqueuse d'acide chlorhydrique 2N. La suspension est filtrée et le solide est lavé par 2 x 40 ml de THF. Le filtrat est décanté. La phase aqueuse est extraite par 80 ml d'acétate d'éthyle puis les phases organiques rassemblées sont séchées sur sulfate de magnésium et filtrées. Le solvant est évaporé sous pression réduite à l'évaporateur rotatif. 19 g de 2-[4-(1-tert-Butoxycarbonyl-2,3-dihydro-1 H-indol-2-yl)-1,3,2,4-dioxadiboretan-2-yl]-indole-1-carboxylic acid tert-butyl ester sont obtenus sous forme d'huile orange.

LC/MS : [M+H]+=487.19; temps de rétention : 3.30 minutes.

Etape Ib : Une suspension de 3.23 g de 5-benzyloxy-3-iodo-indazole-1-carboxylic acid tert-butyl ester, 7.31 g de 2-[4-(1-tert-butoxycarbonyl-2,3-dihydro-1H-indol-2-yl)-1,3,2,4-dioxadiboretan-2-yl]-indole-1-carboxylic acid tert-butyl ester, 2.07 g de palladium tetrakistriphenylphosphine et 11 ml d'une solution aqueuse saturée de bicarbonate de sodium est chauffée au reflux pendant environ deux heures puis une nuit à température ambiante. Le milieu réactionnel est filtré sur papier, puis le filtrat et dilué avec 150 ml d'acétate d'éthyle. La phase organique est lavée par 200 ml d'eau distillée. La phase aqueuse est extraite par 2 x 150 ml d'acétate d'éthyle. Les phases organiques sont réunies et lavées par une solution aqueuse saturée de chlorure de sodium, séchées sur sulfate de magnésium et filtrées. Le solvant est évaporé sous vide à l'évaporateur rotatif. Le brut réactionnel est purifié par chromatographie flash sur silice 70-200 µm, éluant: gradient de cyclohexane/acétate d'éthyle 95 : 5 à 70 : 30. 1.97 g de 5-benzyloxy-3-(1H-indol-2-yl)-1 H-indazole.

Spectre de R.M.N. ¹H (300 MHz, (CD₃)₂SO d6, δ en ppm) : 5,28 (s large : 2H) ; 7,03 (t dédoublé, J = 7,5 et 1,5 Hz : 1 H) ; 7,12 (t dédoublé, J = 7,5 et 1,5 Hz : 1H); 7,12 (s large: 1H); 7,19 (dd, J = 9 et 2,5 Hz : 1 H) ; 7,36 (t large, J = 7,5 Hz : 1 H) ; 7,44 (t large, J = 7,5 Hz : 2H) ; 7,47 (d large, J = 7,5 Hz : 1 H) ; 7,54 (d, J = 9 Hz : 1H); 7,58 (d large, J = 7,5 Hz : 2H) ; 7,62 (d large, J = 7,5 Hz : 1 H) ; 7,65 (d, J = 2,5 Hz : 1 H) ; 11,50 (mf : 1 H) ; de 12,90 à 13,40 (mf très étalé: 1H).

LC/MS : [M+H]+=340.24; temps de rétention : 4.23 minutes.

Etape II : procédure D préparation du 3-(1H-indol-2-yl)-1H-indazol-5-ol

Une solution de 2.54 g de 3-(1 H-indol-2-yl)-5-phenoxy-1 H-indazole, de 2.83 g de formiate d'ammonium et 2.54 g de palladium à 10% sur charbon dans 150 ml d'éthanol est chauffée au reflux pendant une heure. Le catalyseur est filtré sur papier et rincé à l'éthanol. Le filtrat est concentré à sec. 1.74 g de 3-(1H-indol-2-yl)-1H-indazol-5-ol sont obtenus.

Spectre de R.M.N. ¹H (300 MHz, (CD₃)₂SO d6, δ en ppm) : 6,90 (d, J = 1,5 Hz : 1 H) ; de 6,95 à 7,05 (mt : 1 H) ; 7,01 (dd, J = 9 et 2,5 Hz : 1 H) ; 7,10 (t dédoublé, J = 7,5 et 1,5 Hz : 1H) ; 7,39 (d, J = 2,5 Hz: 1H); de 7,40 à 7,50 (mt : 2H) ; 7,60 (d large, J = 7,5 Hz : 1H) ; 9,26 (s large: 1H); 11,48 (s large: 1 H); 13,05 (s large: 1 H).

LC/MS analytique : : [M+H]⁺=250.22 ; temps de rétention : 3.16 minutes.

Le composé 5-benzyloxy-3((E)-styryl)-1H-indazole est préparé selon la procédure C étape Ib à partir de 300 mg de 5-benzyloxy-3-iodo-indazole-1-carboxylic acid tert-butyl ester, de 197 mg d'acide boronique E-phenylethenyle, 192 mg de palladium tetrakistriphenylphosphine en suspension dans 12 ml de dimethylformamide et 0.61 ml d'une solution saturée de bicarbonate de sodium. Le brut réactionnel est traité comme décrit précédemment et 150 mg de 5-benzyloxy-3((E)-styryl)-1H-indazole sont recueillis.

Spectre de R.M.N. ¹H (300 MHz, (CD₃)₂SO d6, δ en ppm) : 5,24 (s large : 2H) ; 7,14 (dd, J = 9 et 2 Hz : 1 H) ; 7,30 (t large, J = 7,5 Hz : 1H); de 7,30 à 7,50 (mt : 5H) ; 7,38 (d, J = 16,5 Hz: 1 H) ; 7,49 (d, J = 9 Hz : 1 H) ; 7,56 (d, J = 16,5 Hz: 1H); 7,56 (d large, J = 7,5 Hz : 2H); 7,68 (d, J = 2 Hz: 1H); 7,73 (d large, J = 7,5 Hz : 2H) ; de 12,50 à 13,50 (mf très étalé : 1 H).

IR (KBr) : 3178 ; 3153 ; 2924; 1587 ; 1497 ; 1228; 1075 ; 957 ; 947 ; 812 ; 787 ; 758 et 691 cm⁻¹

LC/MS analytique : : [M+H]⁺=327.24 ; temps de rétention : 4.74 minutes.

Préparation du 3-styryl-1H-indazol-5-ol: une solution de 652 mg de 5-benzyloxy-3((E)-styryl)-1H-indazole dans 60 ml d'acétonitrile est agitée sous argon. 1.13 ml de iodotrimethylsilane sont additionnés goutte à goutte sous atmosphère inerte. La suspension est agitée à environ 50°C pendant 3 heures puis à température ambiante pendant la nuit. Le milieu est chauffé autour de 50°C puis 1.2 ml de iodotrimethylsilane sont ajoutés. Après 3 heures d'agitation 0.8 ml de iodotrimethylsilane sont ajoutés. Après environ 4 heures d'agitation, le milieu est traité par 10 ml de méthanol et agité à température ambiante pendant une quinzaine de minutes. La suspension est filtrée sur filtre papier et le filtrat est concentré sous vide à l'évaporateur rotatif. Le brut réactionnel est repris dans 50 ml d'acétate d'éthyle et la phase organique est lavée par 2 x 50 ml d'une solution de thiosulfate de sodium, 2 x 50 ml d'une solution saturée de bicarbonate de sodium, 50 ml d'une solution saturée de chlorure de sodium. La phase organique est séchée sur sulfate de magnésium puis concentrée sous vide à l'évaporateur rotatif. Le brut réactionnel est purifié par chromatographie flash sur silice (cartouche Varian 20 g) éluant : gradient acétate d'éthyle/cyclohexane 5 : 95 à 35 : 65. 265.4 mg de 3-styryl-1H-indazol-5-ol.

Spectre de R.M.N. ¹H (300 MHz, (CD₃)₂SO d6, δ en ppm) : 7,08 (dd, J = 9 et 2 Hz : 1 H) ; de 7,30 à 7,50 (mt : 4H) ; 7,31 (t large, J = 7,5 Hz : 1 H) ; 7,38 (d, J = 17 Hz: 1H); 7,49 (d, J = 17 Hz: 1H); 7, 69 (d large, J = 7,5 Hz : 2H); 9,20 (s: 1 H) ; 12,41 (mf:1H).

IR (KBr) : 3397 ; 3261 ; 3058 ; 2923 ; 1629; 1490; 1222; 1071 ; 952 ; 846 ; 804 ; 787 ; 760 ; 742 ; 691 ; 564 cm⁻¹

LC/MS analytique : [M+H]⁺=237.27 ; temps de rétention : 3.16 minutes.

### Procédure E : Préparation des esters de p-nitrophénols selon DS Tawfik, Synthesis, 968-972 (1993) ; S Gobec, Tetrahedron lett., 43, 167-170 (2002).

Le methyl-phenyl-phosphinic acid 4-nitro-phenyl ester est préparé de la manière suivante : une suspension de 173 mg de NaH à 50 % dans l'huile dans 2 ml de tétrahydrofurane anhydre est agitée à température ambiante sous argon. Une solution de 500 mg de 4-nitrophénol dans 2 ml de tétrahydrofurane anhydre est additionnée goutte à goutte à température ambiante. Après environ 1 heure d'agitation à température ambiante, une solution de 630 mg de chlorure méthylphenylphosphinique dans 2 ml de tétrahydrofurane anhydre est additionnée goutte à goutte en une dizaine de minutes. Après 2 heures environ d'agitation le milieu réactionnel est dilué avec 20 ml d'eau distillée et la phase aqueuse est extraite par 2 x 20 ml de dichlorométhane. Les phases organiques réunies sont séchées sur sulfate de magnésium, filtrées et concentrés sous pression réduite à l'évaporateur rotatif. 995 mg de Methyl-phenyl-phosphinic acid 4-nitro-phenyl ester sont obtenus avec une pureté de 50 %.

LC/MS analytique : [M+H]⁺=278.1; temps de rétention : 3.16 minutes.

### Exemple 1 : Préparation de methyl-phenyl-phosphinic acid 3-((E)-styryl)-1H-indazol-5-yl ester

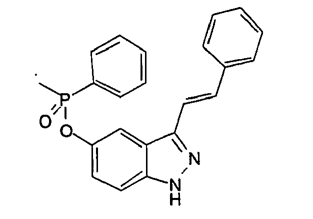

Une solution de 20 mg de 3-styryl-1H-indazol-5-ol, 5.76 mg d'imidazole, de 20.7 mg de chlorure méthylphenylphosphinique dans 5 ml de dichlorométhane est agitée à température ambiante. Après environ une heure d'agitation 5.76 mg d'imidazole et 20.7 mg de chlorure methylphenylphosphinique sont ajoutés. La suspension est agitée à température ambiante pendant 18 heures environ. 10 mg d'imidazole et 70 mg de chlorure methylphenylphosphinique sont ajoutés. Après 30 heures à température ambiante, le milieu réactionnel est traité par 10 ml d'eau distillée. La phase aqueuse est extraite par 2 x 10 ml de dichlorométhane. La phase organique est séchée sur sulfate de magnésium et concentrée sous pression réduite à l'évaporateur rotatif. Le brut réactionnel est purifié par LC/MS préparative. 9.1 mg de methyl-phenyl-phosphonic acid 3-((E)-styryl)-1 H-indazol-5-yl ester sont recueillis.

Spectre de R.M.N. ¹H (300 MHz, (CD₃)₂SO d6, δ en ppm) : 1,93 (d, J = 14,5 Hz : 3H) ; 7,23 (ddd, J = 9 - 2 et 1 Hz : 1 H) ; 7,31 (d, J = 17 Hz : 1 H) ; 7,32 (t large, J = 7,5 Hz : 1H) ; 7,43 (t large, J = 7,5 Hz : 2H) ; 7,50 (d, J = 17 Hz: 1 H) ; 7,50 (d, J = 9 Hz : 1 H) ; de 7,50 à 7,65 (mt : 3H) ; 7, 70 (d large, J = 7,5 Hz : 2H) ; 7,82 (mt : 1H); 7,95 (ddd, J = 12 - 7,5 et 1,5 Hz : 2H) ; 13,21 (mf : 1 H).

IR (KBr) : 3427 ; 3056 ; 2921 ; 1487 ; 143 ; 1211 ; 1183 ; 1124 ; 1070 ; 959 ; 911 ; 806 ; 783 ; 761 ; 745 ; 693 cm⁻¹

LC/MS analytique : : [M+H]⁺=375.22 ; temps de rétention : 3.45 minutes.

### Exemple 2 : Préparation de diphenyl-phosphinic acid 3-((E)-styryl)-1H-indazol-5-yl ester

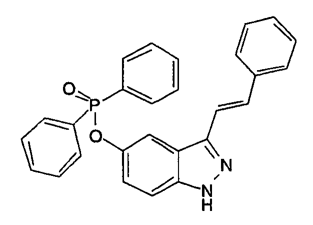

Une solution de 70 mg de 3-styryl-1 H-indazol-5-ol, de 300 mg d'imidazole, de 273 µl de chlorure de diphenylphosphinyle dans 15 ml de dichlorométhane est agitée à température ambiante. Après environ 90 minutes d'agitation, le milieu est traité par 30 ml d'eau distillée. La phase aqueuse est extraite par 2 x 30 ml de dichlorométhane puis les phases organiques sont séchées sur sulfate de magnésium, filtrées et concentrées sous pression réduite à l'évaporateur rotatif. Le brut est repris par de l'acétate d'éthyle. Le solide est filtré sur verre fritté, et rincé à l'éther éthylique. Le filtrat est concentré sous pression réduite à l'évaporateur rotatif, et purifié par chromatographie flash sur silice 35-70 µm. Eluant : cyclohexane/acétate d'éthyle gradient de 80 : 20 à 70 : 30. 143 mg d'un solide jaune sont recueillis. Ce composé est purifié par LC/MS préparative. 47.3 mg de diphenyl-phosphinic acid 3-((E)-styryl)-1H-indazol-5-yl ester sont recueillis.

Spectre de R.M.N. ¹H (300 MHz, (CD₃)₂SO d6, δ en ppm) : 7,31 (d, J = 17 Hz : 1 H); 7,32 (t large, J = 7,5 Hz : 1 H) ; 7,23 (dd large, J = 9 et 2 Hz : 1 H); 7,44 (t large, J = 7,5 Hz : 2H) ; 7,50 (d, J = 17 Hz : 1 H) ; 7,50 (d, J = 9 Hz : 1 H) ; de 7,50 à 7,70 (mt : 6H) ; 7,70 (d large, J = 7,5 Hz : 2H) ; 7,97 (mt : 1 H) ; 8,00 (ddd, J = 12 - 8 et 2 Hz : 4H) ; 13,24 (mf étalé: 1 H).

IR (KBr) : 3433 ; 3174 ; 3057 ; 1484 ; 1439 ; 1226 ; 1130 ; 1072 ; 962 ; 755 ; 734 ; 692 et 565 cm⁻¹

LC/MS analytique : [M+H]⁺= 437.16 ; temps de rétention : 3.89 minutes.

### Exemple 3 : Préparation de methyl-phenylphosphinic acid 3-(1H-indol-2-yl)-1 H-indazol-5-yl ester

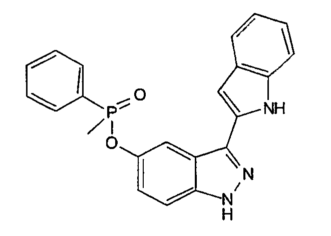

### Procédure F :

Une solution de 497 mg de methyl-phenyl-phosphinic acid 4-nitro-phenyl ester pur à 50 % dans 8 ml de dichlorométhane est agitée à température ambiante. Une suspension de 403 mg 3-(1H-indol-2-yl)-1H-indazol-5-ol et 268 µl de 1,8-diazabicyclo[5.4.0]undec-7-ene dans 12 ml de dichlorométhane est additionnée goutte à goutte. Le milieu réactionnel est agité à température ambiante pendant une nuit puis évaporé à sec sous pression réduite à l'évaporateur rotatif. Le brut réactionnel est purifié par chromatographie flash sur cartouche de 50 g de silice 15-35 µm. 300 mg de methyl-phenylphoshinic acid 3-(1H-indol-2-yl)-1H-indazol-5-yl ester sont recueillis et recristallisés de l'acétate d'éthyle. 220 mg de methyl-phenylphoshinic acid 3-(1H-indol-2-yl)-1 H-indazol-5-yl ester sont isolés.

Spectre de R.M.N. ¹H (300 MHz, (CD₃)₂SO d6, δ en ppm) : 1,95 (d, J = 14,5 Hz : 3H) ; 6,87 (s large : 1 H) ; 7,04 (t dédoublé, J = 7,5 et 1 Hz : 1 H) ; 7,13 (t dédoublé, J = 7,5 et 1 Hz : 1 H) ; 7,26 (dd large, J = 8,5 et 2 Hz : 1H) ; 7,46 (d large, J = 8,5 Hz : 1 H) ; de 7,50 à 7,70 (mt : 5H) ; 7,81 (mt : 1 H) ; 7,97 (ddd, J = 12 - 8 et 2 Hz : 2H); 11,57 (mf : 1 H) ; de 13,00 à 13,70 (mf étalé : 1H).

### Exemple 4 : Préparation de diphenyl-phosphinic acid 3-(1H-indol-2-yl)-1H-indazol-5-yl ester

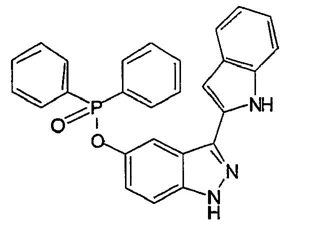

Le 3-(1-tert-butoxycarbonyl-2,3-dihydro-1H-indol-2-yl)-5-hydroxy-indazole-1-carboxylic acid tert-butyl ester est préparé selon la procédure D à partir de 800 mg de 5-benzyloxy-3-(1-tert-butoxycarbonyl-1H-indol-2-yl)-indazole-1-carboxylic acid tert-butyl ester, 560 mg de formiate d'ammonium, 800 mg de palladium 10 % sur charbon dans 30 ml d'éthanol absolu. 700 mg de 3-(1-tert-butoxycarbonyl-2,3-dihydro-1H-indol-2-yl)-5-hydroxy-indazole-1-carboxylic acid tert-butyl ester sont obtenus.

Spectre de R.M.N. ¹H (300 MHz, (CD₃)₂SO d6, δ en ppm) : 1,20 (mf : 9H) ; 1,63 (s : 9H) ; 3,09 (dd large, J = 16,5 et 5 Hz : 1 H) ; 3,79 (dd, J = 16,5 et 11 Hz: 1H) ; 5,79 (dd, J = 11 et 5 Hz: 1H) ; 6,62 (d, J = 2 Hz : 1 H) ; de 6,95 à 7,10 (mt : 2H) ; de 7,20 à 7,35 (mt : 2H) ; 7,81 (mf : 1 H) ; 7,91 (d, J = 9 Hz : 1H) ; 9,57 (mf : 1 H).

### Préparation de 3-(1-tert-butoxycarbonyl-2,3-dihydro-1H-indol-2-yl)-5-(diphenyl-phosphinoyloxy)-indazole-1-carboxylic acid tert-butyl ester

A une solution de 140 mg de 3-(1-tert-butoxycarbonyl-2,3-dihydro-1 H-indol-2-yl)-5-hydroxy-indazole-1-carboxylic acid tert-butyl ester dans 15 ml de dichlorométhane, sont ajoutés 90 mg d'imidazole et 250 µl de chlorure de diphénylphosphinyle. Le milieu réactionnel est agité à température ambiante pendant une nuit. Après dilution avec 10 ml de dichlorométhane et 10 ml d'eau distillée, le milieu est décanté. La phase organique est séchée sur sulfate de magnésium, filtrée et concentrée sous pression réduite à l'évaporateur rotatif. Le brut est repris dans un mélange dichlorométhaneméthanol, le solide est filtré. Après concentration sous pression réduite, le filtrat est purifié par chromatographie flash, éluant : cyclohexane/acétate d'éthyle 8 : 2. 200 mg de 3-(1-tert-butoxycarbonyl-2,3-dihydro-1H-indol-2-yl)-5-(diphenyl-phosphinoyloxy)-indazole-1-carboxylic acid tert-butyl ester sont obtenus.

LC/MS analytique : [M+H]⁺= 652.14 ; temps de rétention : 4.74 minutes

### Préparation de diphenyl-phosphinic acid 3-(2,3-dihydro-1H-indol-2-yl)-1H-indazol-5-yl ester

Une solution de 200 mg de 3-(1-tert-butoxycarbonyl-2,3-dihydro-1H-indol-2-yl)-5-(diphenyl-phosphinoyloxy)-indazole-1-carboxylic acid tert-butyl ester dans 4 ml de dioxane et 1 ml d'une solution d'acide chlorhydrique 4M dans le dioxane est agitée à température ambiante. Après 1 heure, on rajoute 1 ml d'une solution d'acide chlorhydrique 4M dans le dioxane. Le milieu réactionnel est agité une nuit à température ambiante. La suspension est filtrée sur verre fritté. Le solide est rincé à l'éther éthylique. Le solide est repris dans 200 ml de dichlorométhane et 8 ml d'une solution aqueuse d'hydroxyde de sodium 2N. La solution est agitée quelques minutes puis décantée. La phase organique est séchée sur sulfate de magnésium, filtrée et concentrée sous pression réduite. 114 mg de diphenyl-phosphinic acid 3-(2,3-dihydro-1H-indol-2-yl)-1H-indazol-5-yl ester

LC/MS analytique : [M+H]⁺= 452.20 ; temps de rétention : 3.35 minutes

### Préparation de diphenyl-phosphinic acid 3-(1H-indol-2-yl)-1H-indazol-5-yl ester

170 mg de diphenyl-phosphinic acid 3-(2,3-dihydro-1H-indol-2-yl)-1 H-indazol-5-yl ester sont agités pendant 10 heures à 100°C dans le dimethylsulfoxyde. Le solvant est évaporé sous pression réduite à 30°C. Le brut réactionnel est purifié par chromatographie flash sur silice (cartouche interchim de 8 g) éluant : cyclohexane/acétate d'éthyle 4 : 6, 2 : 8 acétate d'éthyle/méthanol 9 : 1. 23 mg de diphenyl-phosphinic acid 3-(1H-indol-2-yl)-1H-indazol-5-yl ester sont obtenus.

Spectre de R.M.N. ¹H (300 MHz, (CD₃)₂SO d6, δ en ppm) : 6,90 (s large: 1 H) ; 7,04 (t large, J = 7,5 Hz : 1 H) ; 7,13 (t large, J = 7,5 Hz : 1H); 7,45 (mt : 2H) ; de 7,50 à 7,70 (mt : 8H) ; 7,99 (mt : 1H) ; 8,02 (dd large, J = 12 et 7,5 Hz : 4H) ; 11,57 (mf : 1 H) ; de 13,00 à 13,70 (mf étalé: 1 H).

LC/MS analytique : [M+H]⁺ = 450.19 ; temps de rétention : 4.06 min

L'intermédiaire C 3-[1-tert-butoxycarbonyl-5-(2-morpholin-4-yl-ethoxy)-1H-indol-2-yl]-5-ydroxy-indazole-1-carboxylic acid tert-butyl ester peut être préparé en 5 étapes, selon le schéma 3.

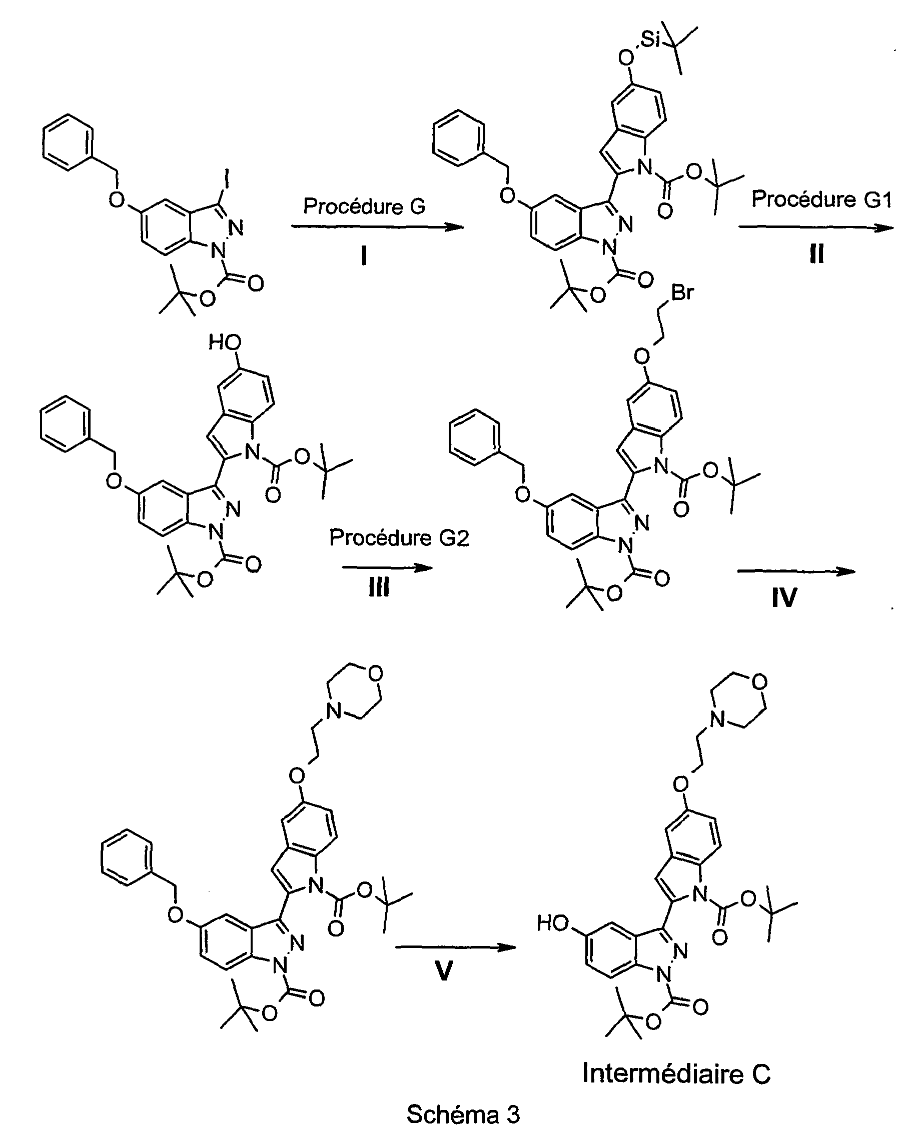

### Etape I : Procédure G

### Préparation de 5-benzyloxy-3-(1-tert-butoxycarbonyl-5-tert-butylsilanyloxy-1 H-indol-2-yl)-indazole-1-carboxylic acid tert-butyl ester.

A une solution de 10 g de 5-benzyloxy-3-iodo-indazole-1-carboxylic acid tert-butyl ester dans 156 ml de dioxanne sont ajoutés 13 g d'acide 1-(*tert-*butyldiméthylsilyloxy)-5-indole-2-boronique, 28.9 g de carbonate de césium, 906.4 mg de dichlorure de palladium (II) [1,1'-bis(diphenylphosphino) ferrocene] en complexe avec du dichloromethane et 51 ml d'eau. Le milieu est agité et chauffé à 88°C pendant 45 minutes puis refroidi à température ambiante. Le mélange réactionnel est ensuite décanté, la phase organique est évaporée sous pression réduite à l'évaporateur rotatif. Le brut réactionnel est solubilisé par 250 ml de dichlorométhane, la solution obtenue est ensuite lavée par 3 fois 50 ml d'eau. Le pH des lavages aqueux passe de 11 à 7.

Après séchage de la phase organique, sur sulfate de magnésium, le solvant est évaporé sous pression réduite à l'évaporateur rotatif. Le brut réactionnel est purifié par chromatographie flash (silice 40-63 µm), éluant: cyclohexane/dichlorométhane 40 : 60, on isole 13.4 g de 5-benzyloxy-3-(1-tert-butoxycarbonyl-5-tert-butylsilanyloxy-1 H-indol-2-yl)-indazole-1-carboxylic acid tert-butyl ester.

Spectre de R.M.N. ¹H (300 MHz, (CD₃)₂SO d6, δ en ppm) : 0,26 (s : 6H) ; 1,02 (s : 9H) ; 1,17 (s : 9H) ; 1,67 (s : 9H) ; 5,16 (s large : 2H) ; 7,00 (dd, J = 9 et 2,5 Hz : 1H) ; 7,06 (s : 1H) ; 7,17 (d, J = 2,5 Hz : 1H) ; de 7,30 à 7,50 (mt : 3H) ; 7,32 (d, J = 2,5 Hz : 1 H) ; 7,38 (dd, J = 9 et 2,5 Hz : 1 H) ; 7,47 (d large, J = 7,5 Hz : 2H) ; 8,08 (d, J = 9 Hz : 2H).

### Etape II : Procédure G1 - préparation de 5-benzyloxy-3-(1-tert-butoxy-carbonyl-5-hydroxy-1 H-indol-2-yl)-indazole-1-carboxylic acid tert-butyl ester

6.15 g de fluorure de tétrabutylammonium sont ajoutés à une solution de 13.4 g de 5-benzyloxy-3-(1-tert-butoxycarbonyl-5-tert-butylsilanyloxy-1H-indol-2-yl)-indazole-1-carboxylic acid tert-butyl ester dans 140 ml de tétrahydrofuranne. Le milieu est agité 30 minutes à température ambiante. Le brut réactionnel est lavé par 3 fois 25 ml d'eau, la phase organique est ensuite séchée sur sulfate de magnésium puis filtrée et le solvant est évaporé sous pression réduite à l'évaporateur rotatif. On obtient 14.4 g de produit brut qui sont purifiés par chromatographie flash (silice 40-63 µm), éluant : dichlorométhane/méthanol 98 : 2.

On isole 8.54 g de 5-benzyloxy-3-(1-tert-butoxycarbonyl-5-hydroxy-1H-indol-2-yl)-indazole-1-carboxylic acid tert-butyl ester.

LC/MS : [M+H]⁺ = 556.35 ; temps de rétention : 4.76 minutes.

### Etape III : procédure G2 - préparation de 5-benzyloxy-3-[5-(2-bromo-ethoxy)-1-tert-butoxycarbonyl-1H-indol-2-yl]-indazole-1-carboxylic acid tert-butyl ester.

Une suspension de 2.22 g de 5-benzyloxy-3-(1-tert-butoxycarbonyl-5-hydroxy-1H-indol-2-yl)-indazole-1-carboxylic acid tert-butyl ester, de 3.90 g de carbonate de césium dans 22 ml de 1,2-dibromoéthane est chauffée à 80°C pendant 40 heures puis est refroidie à température ambiante et évaporée sous pression réduite à l'évaporateur rotatif. Le brut réactionnel est purifié par chromatographie flash (silice 40-63 µm), éluant : dichlorométhane, on isole 2 g de 5-benzyloxy-3-[5-(2-bromo-ethoxy)-1-tert-butoxycarbonyl-1H-indol-2-yl]-indazole-1-carboxylic acid tert-butyl ester.

Spectre de R.M.N. ¹H (300 MHz, (CD₃)₂SO d6, δ en ppm) : 1,17 (s : 9H) ; 1,67 (s : 9H) ; 3,85 (t large, J = 5,5 Hz : 2H) ; 4,41 (t large, J = 5,5 Hz : 2H) ; 5,16 (s : 2H) ; 7,07 (s : 1H) ; 7,11 (dd, J = 9 et 2,5 Hz : 1 H) ; 7,29 et 7,30 (2d, J = 2,5 Hz : 2H en totalité) ; de 7,30 à 7,50 (mt : 6H) ; 8.08 et 8,11 (2d, J = 9 Hz : 2H en totalité).

LC/MS : [M+H]⁺ = 664.22 ; temps de rétention : 5.67 minutes.

### Etape IV : Préparation de 5-benzyloxy-3-[1-tert-butoxycarbonyl-5-(2-morpholin-4-yl-ethoxy)-1 H-indol-2-yl]-indazole-1-carboxylic acid tert-butyl ester.

Une supension de 2.0 g de 5-benzyloxy-3-[5-(2-bromo-ethoxy)-1-tert-butoxycarbonyl-1 H-indol-2-yl]-indazole-1-carboxylic acid tert-butyl ester et de 498 mg de iodure de potassium dans 90 ml d'acétonitrile est chauffée à 80°C pendant 7 heures. On ajoute ensuite 394 µl de morpholine, 150 mg de iodure de potassium et 1.24 g de carbonate de potassium et chauffée à 80°C pendant 15 heures. Le mélange réactionnel est refroidi à température ambiante puis filtré. Le filtrat est évaporé sous pression réduite à l'évaporateur rotatif, on obtient 1.90 g de 5-benzyloxy-3-[1-tert-butoxycarbonyl-5-(2-morpholin-4-yl-ethoxy)-1 H-indol-2-yl]-indazole-1-carboxylic acid tert-butyl ester.

LC/MS : [M+H]⁺ = 669.43 ; temps de rétention : 3.99 minutes.

### Etape V : Préparation de 3-[1-tert-butoxycarbonyl-5-(2-morpholin-4-yl-ethoxy)-1H-indol-2-yl]-5-hydroxy-indazole-1-carboxylic acid tert-butyl ester.

Une solution de 740 mg de 5-benzyloxy-3-[1-tert-butoxycarbonyl-5-(2-morpholin-4-yl-ethoxy)-1H-indol-2-yl]-indazole-1-carboxylic acid tert-butyl ester, de 328 mg de formiate d'ammonium et de 234 mg de palladium à 10 % sur charbon dans 56 ml d'éthanol est chauffé à 70°C pendant 35 minutes. Le mélange réactionnel est ensuite refroidi à température ambiante. Le catalyseur est filtré sur papier et lavé abondamment par de l'éthanol. On obtient 533 mg du composé brut : 3-[1-tert-butoxycarbonyl-5-(2-morpholin-4-yl-ethoxy)-1H-indol-2-yl]-5-hydroxy-indazole-1-carboxylic acid tert-butyl ester.

LC/MS : [M+H]⁺ = 579.32 ; temps de rétention : 3.92 minutes.

### Préparation de 3-[1-tert-butoxycarbonyl-5-(2-morpholin-4-yl-ethoxy)-1H-indol-2-yl]-5-(methyl-phenyl-phosphinoyloxy)-indazole-1-carboxylic acid tert-butyl ester.

A une solution de 27 mg de 3-[1-tert-butoxycarbonyl-5-(2-morpholin-4-yl-ethoxy)-1H-indol-2-yl]-5-hydroxy-indazole-1-carboxylic acid tert-butyl ester dans 2 ml de dichlorométhane est ajouté 16 mg d'imidazole et 40.5 mg de chlorure méthylphenylphosphinique. La solution obtenue est agitée à température ambiante. Après 15 heures d'agitation ajoute à nouveau 16 mg d'imidazole et 40.5 mg de chlorure méthylphenylphosphinique et agite à température ambiante encore 3.5 heures pour que la réaction soit complète. Le mélange réactionnel est ensuite filtré et évaporé sous pression réduite à l'évaporateur rotatif. Le résidu obtenu est purifié par LC/MS. On isole 10.5 mg de 3-[1-tert-butoxycarbonyl-5-(2-morpholin-4-yl-ethoxy)-1H-indol-2-yl]-5-(methyl-phenyl-phosphinoyloxy)-indazole-1-carboxylic acid tert-butyl ester.

LC/MS : [M+H]⁺ = 717.41 ; temps de rétention : 3.61 minutes.

### Exemple 5 : Préparation de methyl-phenyl-phosphinic acid 3-[5-(2-morpholin-4-yl-ethoxy)-1H-indol-2-yl]-1H-indazol-5-yl ester

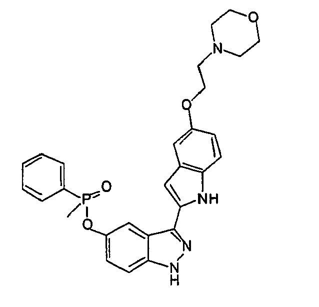

Une solution de 10.5 mg de 3-[1-tert-butoxycarbonyl-5-(2-morpholin-4-yl-ethoxy)-1H-indol-2-yl]-5-(methyl-phenyl-phosphinoyloxy)-indazole-1-carboxylic acid tert-butyl ester dans un mélange de 500 µl de dichlorométhane et 500 µl d'acide trifluoroacétique est agitée à température ambiante pendant 4 heures. Le mélange réactionnel est évaporé sous flux d'azote puis remis en solution dans 1.4 ml de DMSO. La solution obtenue est agitée à 60°C pendant 3 jours puis purifiée par LC/MS. On isole 5.1 mg de methyl-phenyl-phosphinic acid 3-[5-(2-morpholin-4-yl-ethoxy)-1H-indol-2-yl]-1 H-indazol-5-yl ester

LC/MS : [M+H]⁺ = 517.35 ; temps de rétention : 2.64 minutes.

### Exemple 6 : Préparation de phenyl-phosphonic acid 3-(1H-indol-2-yl)-1H-indazol-5-yl ester methyl ester

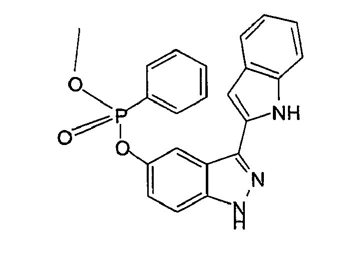

Etape I : Préparation de phenyl-phosphonic acid bis-(4-nitro-phenyl) ester selon la procédure E à partir de 0.345 g d'hydrure de sodium dans 3 ml de tetrahydrofurane anhydre et une solution de 1g de 4-nitrophenol dans 5 ml de tetrahydrofurane et de 0.701 g de dichlorure phénylphosphinique. 1.46 g de phenyl-phosphonic acid bis-(4-nitro-phenyl) ester sont obtenus.

Etape II : le phenyl-phosphonic acid 3-(1H-indol-2-yl)-1H-indazol-5-yl ester methyl ester est préparé selon la procédure F à partir de 230 mg de phenyl-phosphonic acid bis-(4-nitro-phenyl) ester en solution dans 4 ml de dichlorométhane auxquels est ajoutée une solution de 120 mg de 3-(1H-indol-2-yl)-1H-indazol-5-ol et 72 µl de 1,8-diazabicyclo[5.4.0]undec-7-ene dans 4 ml de dichlorométhane. Après 2 heures d'agitation à température ambiante, le milieu est traité avec par 4 fois 50 ml d'une solution saturée de bicarbonate de sodium, puis par 2 x 50 ml d'une solution saturée de chlorure de sodium. La phase organique est séchée sur sulfate de magnésium, puis concentrée à sec. 200 mg de phenyl-phosphonic acid 3-(1H-indol-2-yl)-1H-indazol-5-yl ester methyl ester brut sont obtenus.

Etape III : le phenyl-phosphonic acid 3-(1H-indol-2-yl)-1H-indazol-5-yl ester methyl ester est préparé selon la procédure F à partir de 200 mg de phenyl-phosphonic acid 3-(1H-indol-2-yl)-1H-indazol-5-yl ester methyl ester brut dans 4 ml de dichlorométhane, de 95 µl de méthanol, 72 µl de 1,8-diazabicyclo[5.4.0]undec-7-ene et de 4 ml de dichlorométhane. Après concentration sous pression réduite à l'évaporateur rotatif, le brut est purifié sur cartouche flash interchim de 8 g de silice d'une granulométrie de 15-35 µm. Le produit est élué par un gradient de 15 à 50 % d'acétate d'éthyle dans le cyclohexane. 70 mg d'un produit non pur sont obtenus et repurifiés par LCMS préparative. Le produit obtenu en solution est concentré à sec dans un évaporateur Jouan RC1010. 34 mg phenyl-phosphonic acid 3-(1 H-indol-2-yl)-1H-indazol-5-yl ester methyl ester sont obtenus sous forme d'un solide jaune.

Spectre de R.M.N. ¹H (300 MHz, (CD₃)₂SO d6, δ en ppm) : 3,86 (d, J = 11 Hz : 3H) ; 6,93 (s large : 1 H) ; 7,04 (t large, J = 7,5 Hz : 1 H) ; 7,13 (t large, J = 7,5 Hz : 1 H) ; 7,31 (d large, J = 9 Hz : 1 H) ; 7,46 (d large, J = 7,5 Hz : 1 H) ; de 7,55 à 7,65 (mt : 4H) ; 7,73 (t très large, J = 7,5 Hz : 1 H) ; 7,86 (s large : 1 H) ; 7,93 (dd large, J = 13,5 et 7,5 Hz : 2H) ; 11,59 (mf: 1H) ; 13,41 (mf : 1 H).

LC/MS analytique : [M+H]⁺ = 404.19 ; temps de rétention : 3.62 minutes.

### Exemple 7

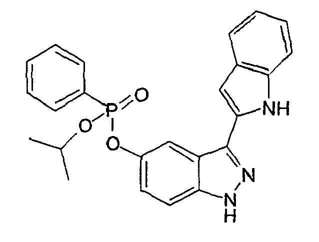

Le phenyl-phosphonic acid 3-(1H-indol-2-yl)-1H-indazol-5-yl ester isopropyl ester peut être préparé selon la procédure F à partir de 100 mg de phenyl-phosphonic acid bis-(4-nitro-phenyl) ester brut en solution dans 5 ml de dichlorométhane, de 150 µl d'isopropanol, de 30 µl de 1,8-diazabicyclo[5.4.0]undec-7-ene. Après concentration du milieu réactionnel, le brut est purifié par LC/MS préparative. 21.5 mg de composé sont recueillis.

Spectre de R.M.N. ¹H (300 MHz, (CD₃)₂SO d6, δ en ppm) : 1,29 (d, J = 6 Hz : 3H) ; 1,33 (d, J = 6 Hz : 3H) ; 4,84 (mt : 1H) ; 6,90 (d, J = 1,5 Hz : 1H) ; 7,04 (t large, J = 7,5 Hz : 1 H) ; 7,14 (t large, J = 7,5 Hz : 1 H) ; 7,30 (dd large, J = 8,5 et 1,5 Hz : 1 H) ; 7,46 (d, J = 8,5 Hz : 1 H) ; de 7,50 à 7,65 (mt : 4H) ; 7,70 (mt : 1H) ; 7,86 (s large : 1 H) ; 7,91 (mt : 2H) ; 11,58 (s large : 1 H) ; 13,39 (s large : 1 H).

### Exemple 8

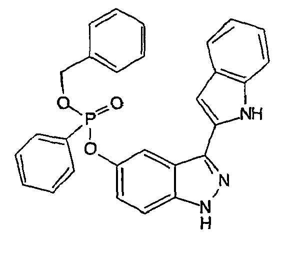

Le phenyl-phosphonic acid benzyl ester 3-(1H-indol-2-y1)-1H-indazol-5-yl ester peut être préparé selon la procédure F à partir de 150 mg de phenyl-phosphonic acid 3-(1H-indol-2-yl)-1H-indazol-5-yl ester 4-nitro-phenyl ester (préparé selon la procédure F) en solution dans 2 ml de dichlorométhane stabilisé sur amylène, 310 µl d'alcool benzylique et 44 µl de 1,8-diazabicyclo[5.4.0]undec-7-ene. Le brut réactionnel est purifié par LC/MS préparative, 16.9 mg de phenyl-phosphonic acid benzyl ester 3-(1H-indol-2-yl)-1H-indazol-5-yl ester sont recueillis.

Spectre de R.M.N. ¹H (300 MHz, (CD₃)₂SO d6, δ en ppm) : 5,28 (d, J = 8 Hz : 2H) ; 6,87 (d, J = 1,5 Hz : 1 H) ; 7,04 (t large, J = 7,5 Hz : 1 H) ; 7,14 (t large, J = 7,5 Hz : 1 H) ; de 7,20 à 7,75 (mt : 12H) ; 7,86 (s large : 1 H) ; 7,94 (mt : 2H) ; 11,58 (s large : 1 H) ; 13,40 (s large : 1 H).

### Exemple 9 : Préparation de methyl-phosphonic acid 3-(1H-indol-2-yl)-1H-indazol-5-yl ester methyl ester

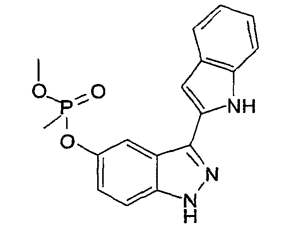

Etape I : le methylphosphonic acid bis-(4-nitrophenyl)ester est préparé selon la procédure E à partir de 1 g de 4-nitrophénol, 12 ml de THF, 345 mg d'hydrure de sodium à 50 % dans l'huile et 478 mg de dichlorure methylphosphonique. 1.07 g de methylphosphonic acid bis-(4-nitrophenyl)ester brut sont obtenus.

Etape II : le methyl-phosphonic acid 3-(1H-indol-2-yl)-1H-indazol-5-yl ester methyl ester est préparé selon la procédure F à partir de 150 mg de methylphosphonic acid bis-(4-nitrophenyl)ester, 4 ml de dichlorométhane, 100 mg de 3-(1H-indol-2-yl)-1H-indazole-5-ol, 67 µl de 1,8-diazabicyclo[5.4.0] undec-7-ene et 4 ml dichlorométhane. Après 3 heures d'agitation est ajoutée goutte-à-goutte une solution de 100 µl de méthanol dans 2 ml de dichlorométhane. Le milieu réactionnel est mis sous agitation pendant environ 16 heures, puis concentré à sec sous pression réduite. L'huile obtenue est purifiée sur cartouche flash Interchim de 5 g de silice d'une granulométrie de 15-35 µ. Eluant : 30 % d'acétate d'éthyle dans le cyclohexane, puis par 80 % d'acétate d'éthyle dans le méthanol. Les solvants sont évaporés sous pression réduite et le produit obtenu est cristallisé de l'acétate d'éthyle. 36 mg de methyl-phosphonic acid 3-(1H-indol-2-yl)-1H-indazol-5-yl ester methyl ester sont obtenus sous forme de cristaux blancs.

Spectre de R.M.N. ¹H (300 MHz, (CD₃)₂SO d6, δ en ppm) : 1,72 (d, J = 17,5 Hz : 3H) ; 3,80 (d, J = 11 Hz : 3H) ; 7,04 (t large, J = 7,5 Hz : 1 H) ; 7,07 (s large : 1 H) ; 7,13 (t large, J = 7,5 Hz : 1 H) ; 7,35 (d large, J = 9 Hz : 1 H) ; 7,47 (d large, J = 7,5 Hz : 1 H) ; 7,63 (d large, J = 7,5 Hz : 1 H) ; 7,65 (d, J = 9 Hz : 1 H) ; 7,95 (s large : 1 H) ; 11,59 (mf : 1 H).

### Exemple 10 : Préparation de 3-iodo-5-(methoxy-phenyl-phosphinoyloxy)-indazole-1-carboxylic acid tert-butyl ester, intermédiaire D, en 4 étapes :

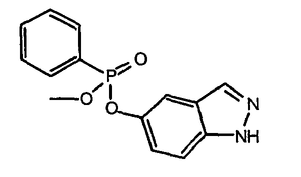

Etape I : Préparation de 1-(5-hydroxy-indazol-1-yl)-ethanone. Une suspension de 939 mg de 1-(5-benzyloxy-indazol-1-yl)-ethanone, 1.33 g de formiate d'ammonium et 939 mg de palladium sur charbon à 10 % dans 100 ml d'éthanol absolu est chauffée aux environs de 50°C pendant une trentaine de minutes. Lorsque le dégagement gazeux est terminé, le catalyseur est filtré sur filtre papier et rincé à l'éthanol absolu. Le filtrat est concentré sous pression réduite à l'évaporateur rotatif. 571.1 mg de 1-(5-hydroxy-indazol-1-yl)-ethanone sont recueillis.

Spectre de R.M.N. ¹H (300 MHz, (CD₃)₂SO d6, δ en ppm) : 2,69 (s : 3H) ; 7,11 (dd, J = 9 et 2 Hz : 1H) ; 7,14 (d, J = 2 Hz : 1H) ; 8,15 (d, J = 9 Hz : 1H) ; 8,31 (d, J = 1 Hz : 1 H) ; de 9,40 à 9,90 (mf étalé : 1H).

Etape II : Préparation de phenyl-phosphonic acid 1-acetyl-1H-indazol-5-yl ester 4-nitro-phenyl ester selon la procédure F à partir de 200 mg de phenyl-phosphonic acid bis-(4-nitro-phenyl) ester, 88 mg de 1-(5-hydroxy-indazol-1-yl)-ethanone, 74.7 µl de 1,8-diazabicyclo[5.4.0]undec-7-ene et 8 ml de dichlorométhane. Après lavage de la phase organique par une solution de bicarbonate de sodium 0.1 M, séchage sur sulfate de magnésium, filtration et concentration sous pression réduite à l'évaporateur rotatif, 219 mg de phenyl-phosphonic acid 1-acetyl-1H-indazol-5-yl ester 4-nitro-phenyl ester bruts sont recueillis et utilisés sans purification dans l'étape suivante.

Etape III : Préparation de phenyl-phosphonic acid 1H-indazol-5-yl ester methyl ester **(Exemple 10)** selon la procédure F à partir de 219 mg de phenyl-phosphonic acid 1-acetyl-1H-indazol-5-yl ester 4-nitro-phenyl ester, 5 ml de dichlorométhane, 74.8 µl de 1,8-diazabicyclo[5.4.0]undec-7-ene et 202 µl de méthanol. Le brut est purifié par chromatographie flash sur silice de granulométrie 35-70 µm. Eluant : cyclohexane puis cyclohexane/acétate d'éthyle 80 : 20. 15 mg de phenyl-phosphonic acid 1H-indazol-5-yl ester methyl ester sous forme d'huile incolore sont recueillis.

Spectre de R.M.N. ¹H (300 MHz, (CD₃)₂SO d6, δ en ppm) : 3,82 (d, J = 11 Hz : 3H) ; 7,19 (dd large, J = 9 et 2,5 Hz : 1 H) ; de 7,50 à 7,75 (mt : 4H) ; 7,53 (d large, J = 9 Hz : 1 H) ; 7,86 (dd large, J = 13,5 et 8 Hz : 2H) ; 8,05 (s large : 1 H) ; 13,13 (mf : 1H).

IR (CCl₄) : 3232 ; 3064 ; 2953 ; 1500 ; 1440 ; 1247 ; 1133 ; 1044 ; 964 ; 943 ; 907 ; 693 et 559 cm⁻¹

LC/MS analytique : [M+H]⁺ = 289.18 ; temps de rétention : 2.84 minutes

Etape IV : Préparation de phenyl-phosphonic acid 3-iodo-1H-indazol-5-yl ester methyl ester selon la procédure A à partir de 76 mg de phenyl-phosphonic acid 1H-indazol-5-yl ester methyl ester, 134 mg d'iode, 30.6 mg d'hydroxyde de potassium préalablement broyé dans 3 ml de diméthylformamide. Le brut est purifié par chromatographie flash sur silice de granulométrie 15-35 µm. Eluant : acétate d'éthyle/cyclohexane 1 : 1. 77.6 mg de phenyl-phosphonic acid 3-iodo-1H-indazol-5-yl ester methyl ester sont recueillis.

Spectre de R.M.N. ¹H (300 MHz, (CD₃)₂SO d6, δ en ppm) : 3,83 (d, J = 11 Hz : 3H) ; 7,19 (t, J = 2 Hz : 1 H) ; 7,27 (ddd, J = 9 - 2 et 1 Hz : 1 H) ; de 7,50 à 7,65 (mt : 2H) ; 7,56 (d, J = 9 Hz : 1H); 7,72 (tq, J = 7,5 et 2 Hz : 1 H) ; 7,88 (ddd, J = 13,5 - 7,5 et 1,5 Hz : 2H) ; de 13,20 à 13,90 (mf étalé : 1 H).

IR (CH₂Cl₂) : 3444 ; 3184 ; 2853 ; 1494 ; 1440 ; 1165 ; 1133 ; 1045 ; 958 ; 906 ; 817 et 559 cm⁻¹

LC/MS analytique : [M+H]⁺= 415.04 ; temps de rétention : 3.24 minutes

Etape V : Préparation de 3-iodo-5-(methoxy-phenyl-phosphinoyloxy)-indazole-1-carboxylic acid tert-butyl ester intermédiaire D selon la procédure B à partir de 77.6 mg de phenyl-phosphonic acid 3-iodo-1H-indazol-5-yl ester methyl ester, 122.7 mg de di-tert-butyl-dicarbonate, 78.1 µl de triéthylamine, 5.7 mg de 4-dimethylaminopyridine en solution dans 2.4 ml de dichlorométhane. Le brut réactionnel est purifié par chromatographie flash sur silice de granulométrie 15-35 µm. Eluant : cyclohexane/acétate d'éthyle 80 : 20 puis 50: 50. 66.3 mg de 3-iodo-5-(methoxy-phenyl-phosphinoyloxy)-indazole-1-carboxylic acid tert-butyl ester sont recueillis.

LC/MS analytique : [M+H]⁺= 515.02 ; temps de rétention : 4.13 minutes.

Préparation de 3-(1-tert-butoxycarbonyl-1H-pyrrol-2-yl)-5-(methoxy-phenyl-phosphinoyloxy)-indazole-1-carboxylic acid tert-butyl ester selon la procédure G à partir de 66.34 mg de 3-iodo-5-(methoxy-phenyl-phosphinoyloxy)-indazole-1-carboxylic acid tert-butyl esterl, 54.44 mg d'acide boronique 2-pyrrole-1-(tert-butoxycarbonyl), 5.28 mg de dichlorure de 1,1'-bis (diphenylphosphino)ferrocene-palladium (II), 168 mg de carbonate de césium, 328 µl d'eau et 1 ml de dioxanne. Le brut est purifié par chromatographie flash sur silice de granulométrie 15-35 µm. Eluant : acétate d'éthyle/cyclohexane 20: 80 puis 30: 70. 35.3 mg de 3-(1-tert-Butoxycarbonyl-1H-pyrrol-2-yl)-5-(methoxy-phenyl-phosphinoyloxy)-indazole-1-carboxylic acid tert-butyl ester sont recueillis.

LC/MS analytique : [M+H]⁺= 554.18 ; temps de rétention : 4.42 minutes.

### Exemple 11 : Préparation de phenyl-phosphonic acid methyl ester 3-(1H-pyrrol-2-yl)-1H-indazol-5-yl ester

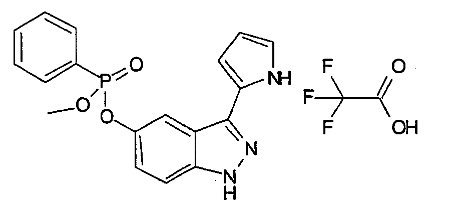

39 mg de 3-(1-tert-butoxycarbonyl-1H-pyrrol-2-yl)-5-(methoxy-phenyl-phosphinoyloxy)-indazole-1-carboxylic acid tert-butyl ester sont mis en solution dans 0.5 ml de dichlorométhane puis 0.5 ml d'acide trifluoroacétique sont ajoutés. La solution est agitée à température ambiante pendant environ deux heures. Le solvant est évaporé sous pression réduite à l'évaporateur rotatif. Le brut réactionnel est purifié par LC/MS préparative. 19 mg de phenyl-phosphonic acid methyl ester 3-(1H-pyrrol-2-yl)-1H-indazol-5-yl ester sont obtenus sous forme de trifluoroacétate.

Spectre de R.M.N. ¹H (300 MHz, (CD₃)₂SO d6, δ en ppm) : 3,84 (d, J = 11,5 Hz : 3H) ; 6,20 (q, J = 3 Hz : 1 H) ; 6,52 (mt : 1 H) ; 6,83 (mt : 1 H) ; 7,24 (ddd, J = 9 - 2,5 et 1,5 Hz: 1H) ; 7,51 (d, J = 9 Hz : 1 H) ; de 7,50 à 7,65 (mt : 2H) ; de 7,65 à 7,75 (mt : 2H) ; 7,89 (ddd, J = 13,5 - 8 et 1,5 Hz : 2H) ; 11,33 (s large : 1H) ; 13,02 (s large : 1 H).

LC/MS analytique : [M+H]⁺= 354.19 ; temps de rétention : 3.23 minutes

Le trifluoro-methanesulfonic acid 3-((E)-styryl)-1H-indazol-5-yl ester est préparé en une étape, selon le schéma 4 :

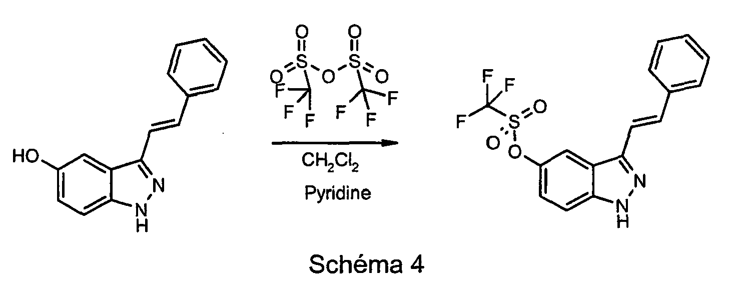

A une solution de 200 mg de 3-((E)-styryl)-1H-indazol-5-ol dans 20 mL de dichlorométhane sur amylène, préalablement refroidit à 0°C, sont ajoutés sous atmosphère d'argon 171 µL d'anhydride trifluorométhanesulfonique goutte-à-goutte puis 512 µL de pyridine goutte-à-goutte. Le milieu est agité et maintenu à 0°C pendant 4 heures, puis laissé sous agitation à température ambiante pendant 48 heures. Le mélange réactionnel est ensuite lavé par 2 fois 20 mL d'eau. La phase aqueuse est extraite avec 3 fois 30 mL de dichlorométhane. La phase organique est séchée sur sulfate de magnésium puis filtrée et le solvant est évaporé sous pression réduite à l'évaporateur rotatif avec un ajout de 10 mL de toluène. 343.8 mg de produit brut sont obtenus.

LC/MS : [M+H]⁺= 369.13 ; temps de rétention=4.98 minutes.

### Exemple 12 : Préparation de [3-((E)-styryl)-1H-indazol-5-yl]-phosphonic acid dimethyl ester

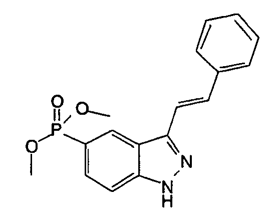

### Procédure H selon le schéma 5 :

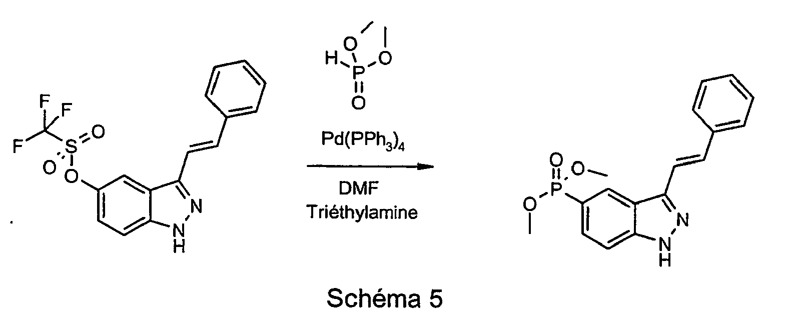

40.7 µL (1.09eq ; 0.444 mmole) de diméthylphosphite, 61.9 µL (1.09eq ; 0.444 mmole) de triéthylamine et 18.8 mg (0.04eq ; 0.016 mmole) de tétrakis(triphenylphosphine)palladium(0) sont mis sous agitation et sous atmosphère d'argon pendant 15 minutes. 150 mg (1eq ; 0.407 mmole) de trifluoro-methanesulfonic acid 3-((E)-styryl)-1H-indazol-5-yl ester en solution dans 5 mL de diméthyl formamide sont ajoutés. Le milieu est chauffé à 85°C toute la nuit. 41 µL de diméthylphosphite, 62 µL de triéthylamine et 20 mg de tétrakis(triphenylphosphine)palladium(0) sont ajoutés au milieu, chauffé deux heures supplémentaires. 100 µL de diméthylphosphite, 100 µL de triéthylamine et 30 mg de tétrakis(triphenylphosphine)palladium(0) sont ensuite additionnés au milieu, chauffé toute la nuit à 105°C. Après filtration du catalyseur sur papier filtre, le solvant est évaporé sous pression réduite à l'évaporateur rotatif. Le brut réactionnel est purifié par LC/MS. 8.6 mg de [3-((E)-styryl)-1H-indazol-5-yl]-phosphonic acid diméthyl ester sont isolés ainsi que 27.2 mg de [3-((E)-styryl)-1H-indazol-5-yl]-phosphonic acid monométhyl ester

Description de [3-((E)-styryl)-1H-indazol-5-yl]-phosphonic acid diméthyl ester

LC/MS : [M+H]⁺=329.20 ; temps de rétention : 3.20 minutes.

Spectre de R.M.N. ¹H (300 MHz, (CD₃)₂SO d6, δ en ppm) : 3,71 (d, J = 11,5 Hz : 6H) ; 7,32 (t large, J = 7,5 Hz : 1 H) ; 7,43 (t large, J = 7,5 Hz : 2H) ; de 7,45 à 7,70 (mt : 4H) ; 7,78 (d large, J = 7,5 Hz : 2H) ; 8,57 (d, J = 14 Hz : 1 H) ; 13,52 (s large : 1 H).

Description de [3-((E)-Styryl)-1H-indazol-5-yl]-phosphonic acid monométhyl ester

LC/MS : [M+H]⁺=315.18; temps de rétention : 2.66 minutes.

### Exemple 13 : Préparation de [3-((E)-styryl)-1H-indazol-5-yl]-phosphonic acid monomethyl ester

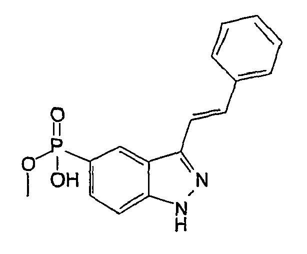

18.5 mg de [3-((E)-styryl)-1H-indazol-5-yl]-phosphonic acid dimethyl ester sont mis en solution dans 500 µL de solution de soude 1 M dans le méthanol. Le milieu est agité à température ambiante pendant 4 heures. 100 µL d'acide chlorhydrique 2N sont ajoutés au mélange agité 15 minutes à température ambiante. La phase organique est extraite par 3 mL d'acétate d'éthyle. Le solvant est évaporé sous vide à l'évaporateur centrifuge. 18 mg de produit sont obtenus.

Spectre de R.M.N. ¹H (300 MHz, (CD₃)₂SO d6, δ en ppm) : 3,20 (d, J = 10,5 Hz : 3H) ; 7,31 (t large, J = 7,5 Hz : 1H); de 7,35 à 7,50 (mt : 3H) ; 7,45 (d, J = 17 Hz : 1 H) ; 7,58 (d, J = 17 Hz : 1 H) ; 7,62 (d, J = 9 Hz : 1 H) ; 7,72 (d large, J = 7,5 Hz : 2H) ; 8,33 (d, J = 13 Hz : 1 H) ; 13,10 (mf : 1H).

LC/MS : [M+H]⁺=315.18 ; temps de rétention : 2.73 minutes.

### Exemple 14 : Préparation de [3-((E)-styryl)-1H-indazol-5-yl]-phosphonic acid diethyl ester

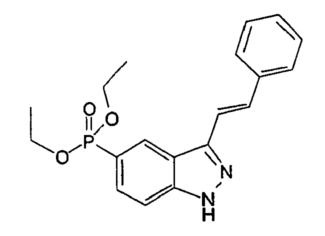

La procédure H est suivie en introduisant 70 mg (1eq ; 0.190 mmole) de trifluoro-methanesulfonic acid 3-((E)-styryl)-1H-indazol-5-yl ester en solution dans 3 mL de diméthylformamide, 26.7 µL (1.09eq ; 0.207 mmole) de diéthylphosphite, 8.9 mg (0.04eq-0.0076 mmole) de tétrakis(triphényl-phosphine)palladium(0) et 28.8 µL (1.09eq ; 0.207 mmole) de triéthylamine. Les ajouts effectués sont successivement de 27 µL de diéthylphosphite, 29 µL de triéthylamine, 10 mg de tétrakis(triphénylphosphine)palladium(0), puis de 50 µL de diéthylphosphite, 50 µL de triéthylamine, 20 mg de tétrakis(triphénylphosphine)palladium(0) et enfin de 27 µL de diéthylphosphite, 29 µL de triéthylamine, 9 mg de tétrakis(triphénylphosphine) palladium(0). 22 mg de [3-((E)-styryl)-1H-indazol-5-yl]-phosphonic acid diethyl ester sont isolés ainsi que 8.7 mg de [3-((E)-styryl)-1H-indazol-5-yl]-phosphonic acid monoethyl ester.

### Description du [3-((E)-styryl)-1H-indazol-5-yl]-phosphonic acid diethyl ester

LC/MS : [M+H]⁺=357.19 ; temps de rétention : 3.63 minutes.

Spectre de R.M.N. ¹H (300 MHz, (CD₃)₂SO d6, δ en ppm) : 1,28 (t, J = 7 Hz : 6H) ; 4,07 (mt : 4H) ; 7,33 (t large, J = 7,5 Hz : 1 H) ; 7,43 (t large, J = 7,5 Hz : 2H); de 7,45 à 7,75 (mt: 2H); 7,53 (d, J = 16,5 Hz : 1H); 7,72 (d, J = 16,5 Hz : 1 H) ; 7,78 (d large, J = 7,5 Hz : 2H) ; 8,56 (d, J = 14 Hz : 1 H) ; 13,50 (mf : 1 H).

### Description du [3-((E)-Styryl)-1H-indazol-5-yl]-phosphonic acid monoethyl ester.

LC/MS: [M+H]⁺=329.17; temps de rétention : 3.00 minutes.

L'intermédiaire réactionnel E 5-(dimethoxy-phosphoryl)-3-iodo-indazole-1-carboxylic acid tert-butyl ester est préparé en quatre étapes, selon le schéma 6.

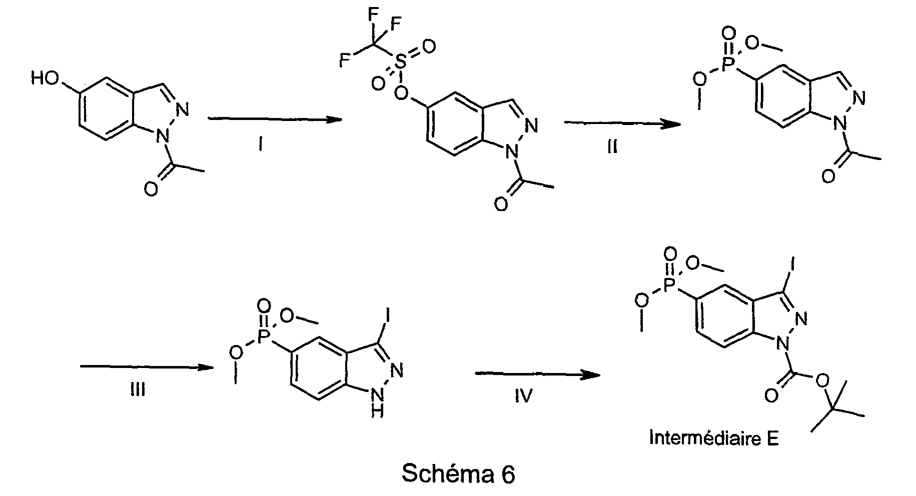

### Etape 1 : procédure I - Préparation de trifluoro-methanesulfonic acid 1-acetyl-1 H-indazol-5-yl ester

A une solution de 98 mg (1eq ;0.556 mmole) de 1-(5-hydroxy-indazol-1-yl)-ethanone dans 10 mL de dichlorométhane sur amylène, préalablement refroidit à 0°C, sont ajoutés goutte-à-goutte 112 µL (1.2eq ; 0.667 mmole) d'anhydride trifluorométhane sulfonique et 337 µL (17.5eq ; 49.557 mmoles) de pyridine sous atmosphère d'argon. Le milieu est agité et maintenu à 0°C toute le nuit sous atmosphère d'argon. 112 µL d'anhydride trifluorométhane sulfonique et 337 µL de pyridine sont ajoutés au milieu qui est agité pendant 2 heures à 0°C. Puis ajout de 112 µL d'anhydride trifluorométhane sulfonique et 337 µL de pyridine suivi d'une agitation du milieu pendant 3 heures à 0°C. Le mélange réactionnel est lavé avec 10 mL d'eau. La phase aqueuse est traitée avec 2 fois 10 mL de dichlorométhane. La phase organique est séchée sur sulfate de magnésium puis filtrée et le solvant est évaporé sous pression réduite à l'évaporateur rotatif. 168.9 mg de trifluoro-methanesulfonic acid 1-acetyl-1H-indazol-5-yl ester sont obtenus.

Spectre de R.M.N. ¹H (300 MHz, (CD₃)₂SO d6, δ en ppm) : 2,76 (s : 3H) ; 7,77 (dd, J = 9 et 2,5 Hz : 1 H) ; 8,17 (d, J = 2,5 Hz : 1 H) ; 8,47(d, J = 9 Hz : 1 H) ; 8,60 (s : 1 H).

### Etape II : préparation de (1-acetyl-1H-indazol-5-yl)-phosphonic acid dimethyl ester

291 µL (1.09eq ; 3.172 mmoles) de diméthylphosphite, 442 µL (1.09eq ; 3.172 mmoles) de triéthylamine et 134 mg (0.04eq ; 0.116 mmole) de tétrakis(triphénylphosphine)palladium(0) sont mis sous agitation et sous atmosphère d'argon pendant 15 minutes. 897 mg (1eq ; 2.91 mmoles) de trifluoro-methanesulfonic acid 1-acetyl-1 H-indazol-5-yl ester en solution dans 60 mL de diméthyl formamide. Le milieu est chauffé à 105°C durant 4 heures sous atmosphère d'argon. Le solvant est évaporé sous pression réduite à l'évaporateur rotatif avec ajout de 40 mL de toluène. Le brut réactionnel est repris dans 40 mL d'acétate d'éthyle puis lavé par 50 mL d'eau. La phase organique est séchée sur sulfate de magnésium puis filtrée et le solvant est évaporé sous pression réduite à l'évaporateur rotatif. 973.6 mg de produit brut sont obtenus. Le brut réactionnel est purifié par chromatographie flash (silice 35-70 µm), éluant : acétate d'éthyle/cyclohexane 80 :20. 430.5 mg de (1-acetyl-1 H-indazol-5-yl)-phosphonic acid dimethyl ester sont isolés.

LC/MS : [M+H]⁺=269.18, temps de rétention : 2.79 minutes.

### Etape III : Préparation de (3-iodo-1H-indazol-5-yl)-phosphonic acid dimethyl ester

A une solution de 430 mg (1eq-1.603 mmoles) de (1-acetyl-1H-indazol-5-yl)-phosphonic acid dimethyl ester dans 20 mL de diméthyl formamide sont ajoutés 814 mg (2eq-3.206 mmoles) d'iode et 180 mg (2eq-3.206 mmoles) d'hydroxyde de potassium broyé. Le milieu est agité à température ambiante pendant 48 heures. 814 mg d'iode et 180 mg d'hydroxyde de potassium sont ajoutés au milieu qui est agité 3 heures à température ambiante. 20 mL d'une solution saturée de thiosulfate de sodium sont ajoutés, le milieu est agité durant 10 minutes. Le mélange réactionnel est lavé par 40 mL d'eau. La phase aqueuse est traitée avec 4 fois 50 mL d'acétate d'éthyle. La phase organique est séchée sur sulfate de magnésium puis filtrée et le solvant est évaporé sous pression réduite à l'évaporateur rotatif. 458 mg de (3-Iodo-1H-indazol-5-yl)-phosphonic acid dimethyl ester sont obtenus.

LC/MS : [M+H]⁺=353.06, temps de rétention : 2.65 minutes.

### Etape IV : Préparation de 5-(dimethoxy-phosphoryl)-3-iodo-indazole-1-carboxylic acid tert-butyl ester

A une solution de 458 mg (1eq ;1.301 mmoles) de (3-iodo-1H-indazol-5-yl)-phosphonic acid dimethyl ester en solution dans 10 mL de dichlorométhane sont ajoutés 851.7 mg (3eq ; 3.903 mmoles) de di-tert-butyl dicarbonate, 39.7 mg (0.25eq ; 0.325 mmole) de 4-diméthylaminopyridine et 544 µL (3eq ; 3.903 mmoles) de triéthylamine. Le milieu est agité à température ambiante toute la nuit. Le mélange réactionnel est lavé par 20 mL d'eau puis 10 mL d'une solution saturée de chlorure de sodium. La phase aqueuse est traitée avec 4 fois 20 mL d'acétate d'éthyle. La phase organique est séchée sur sulfate de magnésium puis filtrée et le solvant est évaporé sous pression réduite à l'évaporateur rotatif. 415 mg de produit brut sont obtenus.

LC/MS : [M+H]⁺=452.99, temps de rétention : 3.61 minutes.

### Exemple 15 : Préparation de [3-(1H-indol-2-yl)-1H-indazol-5-yl]-phosphonic acid monomethyl ester

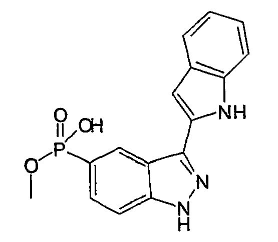

A 130 mg (1eq ; 0.287 mmole) de 5-(dimethoxy-phosphoryl)-3-iodo-indazole-1-carboxylic acid tert-butyl ester sont ajoutés 82.9 mg (0.25eq ; 0.072 mmole) de tétrakis(triphénylphosphine)palladium(0), 150.1 mg (2eq ; 0.575 mmole) de 1-boc-indole-2-acide boronique, 5 mL de diméthylformamide et 250 µL de solution saturée de bicarbonate de sodium. Le milieu est agité à 130°C pendant 5 heures. Le milieu réactionnel est filtré sur papier et le solvant est évaporé sous pression réduite à l'évaporateur rotatif. Le brut réactionnel est purifié par LC/MS. 41 mg de [3-(1H-indol-2-yl)-1H-indazol-5-yl]-phosphonic acid monomethyl ester sont isolés.

Spectre de R.M.N. ¹H (300 MHz, (CD₃)₂SO d6, δ en ppm) : 3,58 (d, J = 11 Hz : 3H) ; 7,05 (t dédoublé, J = 7,5 et 1 Hz : 1 H) ; 7,08 (s large : 1 H) ; 7,16 (t dédoublé, J = 7,5 et 1 Hz : 1H) ; 7,49 (d large, J = 7,5 Hz : 1H) ; de 7,60 à 7,75 (mt : 2H) ; 7,67 (d large, J = 7,5 Hz : 1 H) ; 8,51 (d, J = 14 Hz : 1H) ; 11,69 (s large : 1 H) ; 13,62 (mf : 1 H).

LC/MS : [M+H]+=328.17 ; temps de rétention : 2.57 minutes.

### Exemple 16 : Préparation de (3-thiophen-2-yl-1H-indazol-5-yl)-phosphonic acid monomethyl ester

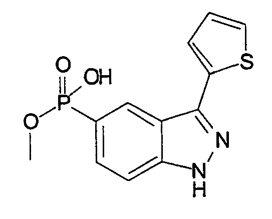

A 70 mg (1eq ; 0.155 mmole) de 5-(dimethoxy-phosphoryl)-3-iodo-indazole-1-carboxylic acid tert-butyl ester sont ajoutés 44.8 mg (0.25eq ; 0.039 mmole) de tétrakis(triphénylphosphine)palladium(0), 39.6 mg (2eq ; 0.310 mmole) de 2-thiophène acide boronique, 3 mL de diméthylformamide et 150 µL de solution saturée de bicarbonate de sodium. Le milieu est agité à 130°C pendant 5 heures. Le milieu réactionnel est filtré sur papier et le solvant est évaporé sous pression réduite à l'évaporateur rotatif. Le brut réactionnel est purifié par LC/MS. 2 mg de (3-thiophen-2-yl-1 H-indazol-5-yl)-phosphonic acid dimethyl ester (pureté de 40 % en RMN) sont obtenus, ainsi que 12.9 mg de (3-thiophen-2-yl-1H-indazol-5-yl)-phosphonic acid monomethyl ester

Spectre de R.M.N. ¹H (300 MHz, (CD₃)₂SO d6, δ en ppm) : 3,53 (d, J = 11 Hz : 3H) ; 7,26 (dd, J = 5,5 et 3 Hz : 1 H) ; 7,65 (dd, J = 5,5 et 1 Hz : 1 H) ; de 7,65 à 7,75 (mt : 2H) ; 7,68 (d large, J = 3 Hz : 1 H) ; 8,41 (d, J = 14 Hz : 1H) ; 13,49 (mf :1 H).

### Exemple 17 : Préparation de [3-(1H-pyrrol-2-yl)-1H-indazol-5-yl]-phosphonic acid monomethyl ester

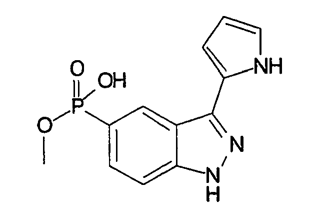

A 70 mg (1eq ; 0.155 mmole) de 5-(dimethoxy-phosphoryl)-3-iodo-indazole-1-carboxylic acid tert-butyl ester sont ajoutés 44.8 mg (0.25eq ; 0.039 mmole) de tétrakis(triphénylphosphine)palladium(0), 65.3 mg (2eq ; 0.310 mmole) de 1-boc-pyrrole-2-acide boronique, 3 mL de diméthylformamide et 150 µL de solution saturée de bicarbonate de sodium. Le milieu est agité à 130°C pendant 5 heures. Le milieu réactionnel est filtré sur papier et le solvant est évaporé sous pression réduite à l'évaporateur rotatif. Le brut réactionnel est purifié par LC/MS. 4.10 mg de ([3-(1H-pyrrol-2-yl)-1H-indazol-5-yl]-phosphonic acid monomethyl ester sont obtenus.

Spectre de R.M.N. ¹H (300 MHz, (CD₃)₂SO d6, δ en ppm) : 3,55 (d, J = 11 Hz : 3H) ; 6,24 (q, J = 3 Hz : 1 H) ; 6,66 (mt : 1 H) ; 6,91 (mt : 1 H) ; 7,65 (mt : 2H) ; 8,36 (d, J = 14,5 Hz : 1 H) ; 11,46 (mf : 1 H) ; 13,24 (mf : 1H).

### Exemple 18 : Préparation de dimethyl-phosphinic acid 3-(1H-indol-2-yl)-1H-indazol-5-yl ester

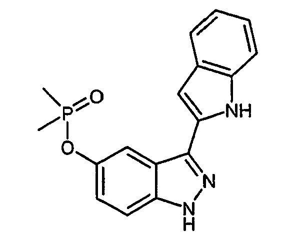

Le réactif dimethylphosphinic acid 4-nitrophenyl ester est préparé selon la procédure E à partir de 500 mg de dimethylphosphinic chloride, 214 mg d'hydrure de sodium (50 % dans l'huile), 618 mg de p-nitrophénol en solution dans 10 ml de tétrahydrofurane. On recueille 700 mg d'attendu.

Rendement = 26 %

Le composé dimethyl-phosphinic acid 3-(1H-indol-2-yl)-1h-indazol-5-yl ester est préparé selon la procédure F à partir 230 mg de 3-(1H-indol-2-yl)-H-indazole-5-ol (intermédiaire B) en solution avec 245 mg de dimethylphosphinic acid 4-nitrophenyl ester dans 5 ml de dichlorométhane (stabilisé sur amylène) à laquelle on ajoute 170 µl de 1,8-diazabicyclo[5.4.0]undec-7-ene (DBU) en solution dans 2 ml de dichlorométhane. Après agitation 24 heures à température ambiante, le solvant est évaporé et le brut est purifié par LC/MS préparative. Les cristaux obtenus après concentration des fractions sont lavés à l'acétate d'éthyle puis à l'éther isopropylique. On recueille 129 mg de produit attendu.

Rendement = 43 %

Spectre RMN déplacements chimiques (δ en ppm) - dans le solvant diméthylsulfoxide - d6 (DMSO-d6) référencé à 2,50 ppm :

1,65 (d, J = 15,0 Hz, 6H) ; de 7,00 à 7,06 (m, 2H) ; 7,13 (t large, J = 8,0 Hz, 1 H) ; 7,30 (d large, J = 8,0 Hz, 1 H) ; 7,46 (d large, J = 8,0 Hz, 1 H) ; de 7,58 à 7,65 (m, 2H) ; 7,91 (m, 1 H) ; 11,6 (m large, 1 H) ; 13,4 (s large, 1 H).

### Exemple 19 : préparation de l'ester méthylique de l'acide 3-[5-(2-piperidin-1-yl-ethoxy)-1H-indol-2-yl]-1H-indazol-5-yl phosphonique

Le composé peut être préparé en 6 étapes selon le schéma suivant :

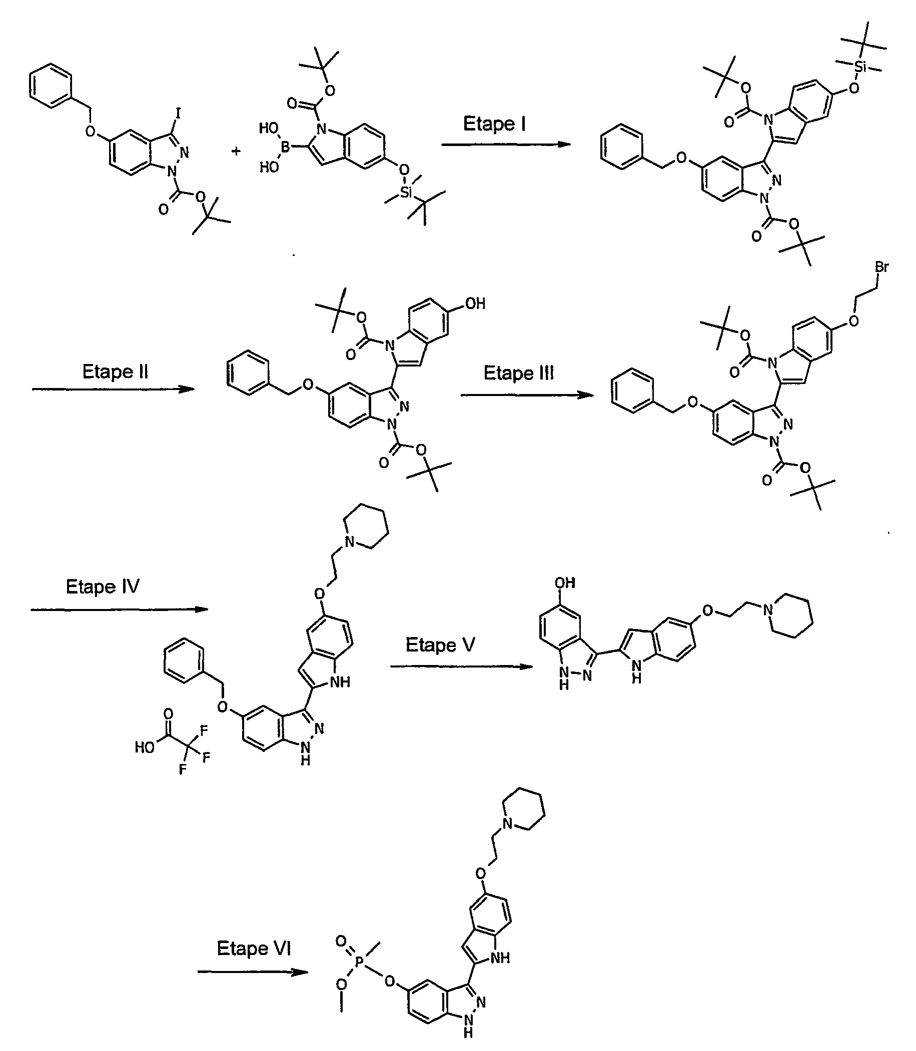

Etape I : Le tertiobutylate de l'acide 5-benzyloxy-3-[1-tert-butoxycarbonyl-5-(tert-butyl-dimethyl-silanyloxy)-1H-indol-2-yl]-indazole-1-carboxylique est préparé selon la Procédure G, à partir de 5-benzyloxy-3-iodo-indazole-1-carboxylic acid tert-butyl ester et de [5-(tert-butyl-dimethyl-silanyloxy)-indole-1-carboxylic acid tert-butyl este]-2 boronic acid lui même obtenu comme décrit dans WO 2003020699 A2.

Etape II : Le tertio butylate de l'acide 5-benzyloxy-3-(1-tert-butoxycarbonyl-5-hydroxy-1H-indol-2-yl)-indazole-1-carboxylique est préparé selon la procédure G1.

Etape III : Le tertio butylate de 5-benzyloxy-3-[5-(2-bromo-ethoxy)-1-tert-butoxycarbonyl-1H-indol-2-yl]-indazole-1-carboxylique est préparé selon la ns procédure G2.

Etape IV : Le trifluoro acétate de 5-benzyloxy-3-[5-(2-piperidin-1-yl-ethoxy)-1H-indol-2-yl]-1H-indazole est obtenu de la façon suivante :

Une suspension de 600 mg de tertio butylate de 5-benzyloxy-3-[5-(2-bromo-ethoxy)-1-tert-butoxycarbonyl-1H-indol-2-yl]-indazole-1-carboxylique, de 442 mg de carbonate de potassium, de 272 mg de pipéridine dans 30 ml d'acétonitrile est agitée à 85°C pendant 5 heures. Après retour à 20°C, le mélange réactionnel est évaporé sous pression réduite puis le résidu est repris dans un mélange d'acétate d'éthyle (100 ml) et d'eau (100 ml). Après décantation et extraction avec l'acétate d'éthyle (1 x 50 ml), les extraits organiques sont réunis, séchés sur sulfate de magnésium puis évaporés sous pression réduite. Le composé issu de l'évaporation est repris dans un mélange de 15 ml de dichlorométhane et 5 ml d'acide trifluoroacétique puis agité à 20°C pendant une heure. Le mélange réactionnel est évaporé sous pression réduite le résidu est repris dans un mélange d'acétate d'éthyle (50 ml) et de bicarbonate de sodium à 10 % (50 ml). Après décantation et extraction avec l'acétate d'éthyle (2 x 50 ml), les extraits organiques sont réunis, séchés sur sulfate de magnésium puis évaporés sous pression réduite. Le composé brut ainsi obtenu est purifié par chromatographie sur silice (colonne Interchrom, DC0210, 20g silice ; éluant dichlorométhane : méthanol 9 :1 volumes, 15 ml/min). Les fractions contenant le composé attendu sont réunies et évaporées sous pression réduite. On isole 360 mg de trifluoro acétate de 5-benzyloxy-3-[5-(2-piperidin-1-yl-ethoxy)-1H-indol-2-yl]-1 H-indazole.

Etape V : Le 3-[5-(2-piperidin-1-yl-ethoxy)-1H-indol-2-yl]-1H-indazol-5-ol est obtenu de la façon suivante :

Une suspension de 360 mg de 5-benzyloxy-3-[5-(2-piperidin-1-yl-ethoxy)-1 H-indol-2-yl]-1 H-indazole, de 916 mg de formiate d'ammonium et de 120 mg de palladium dans 10 ml d'éthanol est placé dans un réacteur que l'on irradie dans un champ de micro-ondes (Synthwave 402) à une température de 90°C pendant 30 minutes puis le mélange réactionnel est filtré sur un lit de célite. Le filtrat est évaporé sous pression réduite puis le résidu est repris dans un mélange d'acétate d'éthyle (150 ml) d'eau (50 ml). On ajoute une solution d'hydroxyde d'ammonium 30 % de façon à amener le pH à 10. Après décantation et extraction avec l'acétate d'éthyle (1 x 50 ml), les extraits organiques sont réunis, séchés sur sulfate de magnésium puis évaporés sous pression réduite. On isole et caractérise 193 mg de 3-[5-(2-piperidin-1-yl-ethoxy)-1H-indol-2-yl]-1H-indazol-5-ol.

Etape VI : préparation de l'ester méthylique de l'acide 3-[5-(2-piperidin-1-yl-ethoxy)-1H-indol-2-yl]-1 H-indazol-5-yl phosphonique :

Dans une solution de 193 mg de 3-[5-(2-piperidin-1-yl-ethoxy)-1H-indol-2-yl]-1H-indazol-5-ol et de 173.5 mg de methylphosphonic acid bis-(4-nitrophenyl)ester dans 10 ml de dichlorométhane, on ajoute 77µl de 1,8-diazabicyclo[5.4.0]Undec-7-Ene puis on laisse réagir à 20°C. Après trois heures de réaction, on ajoute 300 µl de méthanol puis 77µl de 1,8-diazabicyclo[5.4.0]Undec-7-ene et on poursuit la réaction à 20°C pendant 16 heures. Le milieu réactionnel est alors repris dans un mélange de dichlorométhane (150 ml) et une solution saturée de bicarbonate de sodium (100 ml). Après décantation et extraction avec du dichlorométhane (50 ml), les extraits organiques sont réunis, séchés sur sulfate de magnésium puis évaporés sous pression réduite. Le composé brut ainsi obtenu est purifié par chromatographie sur silice (Colonne Interchrom, référence DC0210, 20 g Silice, éluant dichlorométhane méthanol 9 / 1 v/v, 15 ml/min). Les fractions contenant le composé attendu sont réunies et évaporées sous pression réduite. On isole et caractérise 89 mg de l'ester méthylique de l'acide3-[5-(2-piperidin-1-yl-ethoxy)-1 H-indol-2-yl]-1 H-indazol-5-yl phosphonique :

LC/MS analytique : [M+H]⁺ = 469.25 ; Temps de rétention = 3.02 minutes.

### Exemple 20 : Préparation du methyl-phosphonic acid mono-{3-[5-(2-piperazin-1-yl-ethoxy)-1H-indol-2-yl]-1H-indazol-5-yl} ester

Le composé peut être préparé en 6 étapes selon le schéma suivant :

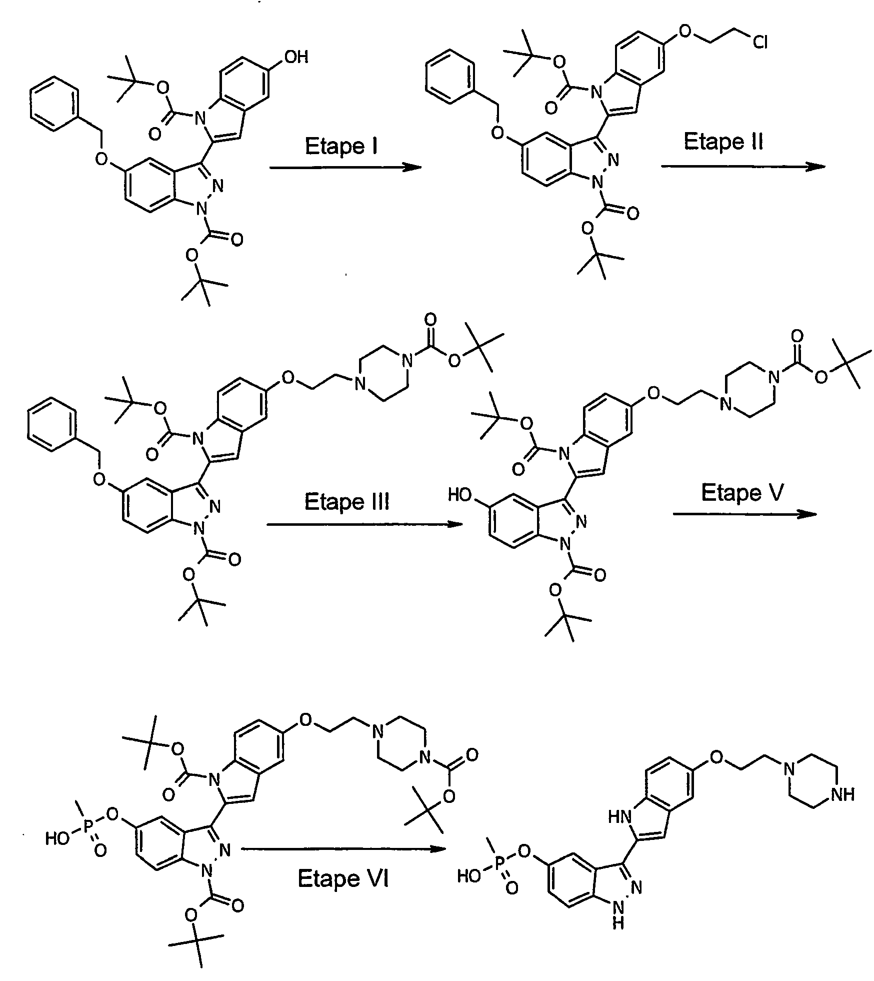

### Etape I : procédure J - Le 5-benzyloxy-3-[1-tert-butoxycarbonyl-5-(2-chloro-ethoxy)-1H-indol-2-yl]-indazole-1-carboxylic acid tert-butyl ester est préparé de la façon suivante :

Une solution de 100 mg de 5-benzyloxy-3-(1-tert-butoxycarbonyl-5-hydroxy-1H-indol-2-yl)-indazole-1-carboxylic acid tert-butyl ester et de 132 µl de 1-bromo-2-chloroéthane dans 5 ml de dichlorométhane est mélangée à une solution aqueuse de 72 mg de bromure de tétrabutyl ammonium et de 270 µl d'hydroxyde de sodium 2N dans 5 ml d'eau distillée. Le mélange réactionnel est placé sous vive agitation à 20°C pendant 6 jours. Après décantation et lavage avec de l'eau (4 x 5 ml), les extraits organiques sont réunis, séchés sur sulfate de magnésium puis évaporés sous pression réduite. Le composé brut obtenu est purifié par LCMS préparative. Les fractions contenant le composé attendu sont réunies et évaporées sous pression réduite. On isole et caractérise 18.1 mg de 5-benzyloxy-3-[1-tert-butoxycarbonyl-5-(2-chloro-ethoxy)-1H-indol-2-yl]-indazole-1-carboxylic acid tert-butyl ester :

LC/MS analytique : [M+H]⁺ = 618.15 ; Temps de rétention = 5.56 minutes

### Etape II : le 5-benzyloxy-3-{1-tert-butoxycarbonyl-5-[2-(4-tert-butoxycarbonyl-piperazin-1-yl)-ethoxy]-1H-indol-2-yl}-indazole-1-carboxylic acid tert-butyl ester est préparé de la façon suivante :

Une suspension de 330 mg de 5-benzyloxy-3-[1-tert-butoxycarbonyl-5-(2-chloro-ethoxy)-1H-indol-2-yl]-indazole-1-carboxylic acid tert-butyl ester, de 442 mg de carbonate de potassium, de 133 mg d'iodure de potassium et de 596 mg de N-1-Boc-pipérazine dans 15 ml d'acétonitrile est agitée à 85°C pendant 48 heures. Après retour à 20°C, le mélange réactionnel est évaporé sous pression réduite puis le résidu est repris dans un mélange d'acétate d'éthyle (15 ml) et d'eau (15 ml). Après décantation et extraction avec l'acétate d'éthyle (1 x 15 ml), les extraits organiques sont réunis, séchés sur sulfate de magnésium puis évaporés sous pression réduite. Le composé brut ainsi obtenu est purifié par chromatographie sur silice (colonne AIT, BPSUP 20-40 µm, 25 g silice ; éluant cyclohexane : acétate d'éthyle 1 :1 -volumes). Les fractions contenant le composé attendu sont réunies et évaporées sous pression réduite. Le composé ainsi obtenu est dissous dans 10 ml de dichlorométhane puis traité par 350 mg de di-tertio-butyle dicarbonate et de 10 mg de diméthylamino pyridine à 20°C pendant 3 heures. le mélange réactionnel est évaporé sous pression réduite, on isole et caractérise 220 mg de 5-benzyloxy-3-{1-tert-butoxycarbonyl-5-[2-(4-tert-butoxycarbonyl-piperazin-1-yl)-ethoxy]-1H-indol-2-yl}-indazole-1-carboxylic acid tert-butyl ester, utilisé sans purification.

LC/MS analytique : [M+H]⁺ = 768.39 ; Temps de rétention = 4.00 minutes

### Etape III : Le 3-{1-tert-butoxycarbonyl-5-[2-(4-tert-butoxycarbonyl-piperazin-1-yl)-ethoxy]-1H-indol-2-yl}-5-hydroxy-indazole-1-carboxylic acid tert-butyl ester est préparé de la façon suivante :

Une suspension de 220 mg de 5-benzyloxy-3-{1-tert-butoxycarbonyl-5-[2-(4-tert-butoxycarbonyl-piperazin-1-yl)-ethoxy]-1 H-indol-2-yl}-indazole-1-carboxylic acid tert-butyl ester de 108 mg de formiate d'ammonium et de 29.8 mg de palladium dans 30 ml d'éthanol est placé dans un réacteur que l'on agite vigoureusement tout en le portant à une température de 80°C. Après 4 heures, le mélange réactionnel est filtré sur un lit de célite. Le filtrat est remis en réaction avec 108 mg de formiate d'ammonium et de 29.8 mg de palladium pendant 4 heures supplémentaires. Le mélange réactionnel est alors filtré sur un lit de célite, évaporé sous pression réduite puis le résidu est repris dans un mélange d'acétate d'éthyle (20 ml) et d'une solution saturée de bicarbonate de sodium (20 ml). Après décantation et extraction avec l'acétate d'éthyle (2 x 20 ml), les extraits organiques sont réunis, séchés sur sulfate de magnésium puis évaporés sous pression réduite.

Le composé obtenu (60 mg) est soumis à un troisième cycle de réaction en présence de 38 mg de formiate d'ammonium et de 12 mg de palladium dans 30 ml d'éthanol en irradiant dans un four à micro-ondes (température 90°C) pendant 30 minutes. Le mélange réactionnel est filtré sur un lit de célite, évaporé sous pression réduite. On isole 34 mg d'un composé brut contenant le 3-{1-tert-butoxycarbonyl-5-[2-(4-tert-butoxycarbonyl-piperazin-1-yl)-ethoxy]-1H-indol-2-yl}-5-hydroxy-indazole-1-carboxylic acid tert-butyl ester que l'on utilise dans l'étape suivante sans purification.

LC/MS analytique : [M+H]⁺= 678.74 ; Temps de rétention = 3.31 minutes

### Etape IV : 3-{1-tert-butoxycarbonyl-5-[2-(4-tert-butoxycarbonyl-piperazin-1-yl)-ethoxy]-1H-indol-2-yl}-5-(hydroxy-methyl-phosphinoyloxy)-indazole-1-carboxylic acid tert-butyl ester

Une solution de 34 mg de 3-{1-tert-butoxycarbonyl-5-[2-(4-tert-butoxycarbonyl-piperazin-1-yl)-ethoxy]-1H-indol-2-yl}-5-hydroxy-indazole-1-carboxylic acid tert-butyl ester dans 1.5 ml de dichlorométhane est refroidie das un bain de glace. On ajoute 16.9 mg de methylphosphonic acid bis-(4-nitrophenyl)ester préparé selon la procédure E et 7.6 mg de 1,8-diazabicyclo[5.4.0]undec-7-ene dans 500 µl de dichlorométhane et on poursuit la réaction à 20°C pendant 3 heures. Le milieu réactionnel est lavé par 2 fois 3 ml d'une solution saturée de bicarbonate de sodium puis par 3 ml d'eau distillée. Après décantation les extraits organiques sont séchés sur sulfate de magnésium puis évaporés sous pression réduite. On isole 39 mg d'un mélange contenant maoritairement le 3-{1-tert-butoxycarbonyl-5-[2-(4-tert-butoxycarbonyl-piperazin-1-yl)-ethoxy]-1H-indol-2-yl}-5-(hydroxy-methyl-phosphinoyloxy)-indazole-1-carboxylic acid tert-butyl ester que l'on utilise dans l'étape suivante sans purification.

LC/MS analytique : [M+H]⁺ = 756.76 ; Temps de rétention = 3.45 minutes

### Etape V : methyl-phosphonic acid mono-{3-[5-(2-piperazin-1-yl-ethoxy)-1H-indol-2-yl]-1H-indazol-5-yl} ester :

Une solution de 39 mg du mélange précédent contenant majoritairement le 3-{1-tert-butoxycarbonyl-5-[2-(4-tert-butoxycarbonyl-piperazin-1-yl)-ethoxy]-1H-indol-2-yl}-5-(hydroxy-methyl-phosphinoyloxy)-indazole-1-carboxylic acid tert-butyl ester dans 1 ml de dichlorométhane et 200 µl d'acide trifluoroacétique est agitée à 20°C pendant 4 heures puis le mélange réactionnel est évaporé sous pression réduite. Le composé brut obtenu est purifié par LCMS préparative, les fractions contenant le composé de masse molaire 455 sont réunies et évaporées sous pression réduite. On isole et caractérise 4 mg de methyl-phosphonic acid mono-{3-[5-(2-piperazin-1-yl-ethoxy)-1H-indol-2-yl]-1 H-indazol-5-yl} ester.

LC/MS analytique :[M+H]⁺ = 456.33 ; Temps de rétention = 1.85 minutes

### Exemples 21 et 22 : les composés methyl-phosphonic acid mono-{3-[5-(2-diethylamino-ethoxy)-1 H-indol-2-yl]-1H-indazol-5-yl} ester et methyl-phosphonic acid 3-[5-(2-diethylamino-ethoxy)-1H-indol-2-yl]-1H-indazol-5-yl ester methyl ester peuvent être préparés en 4 étapes selon le schéma suivant :

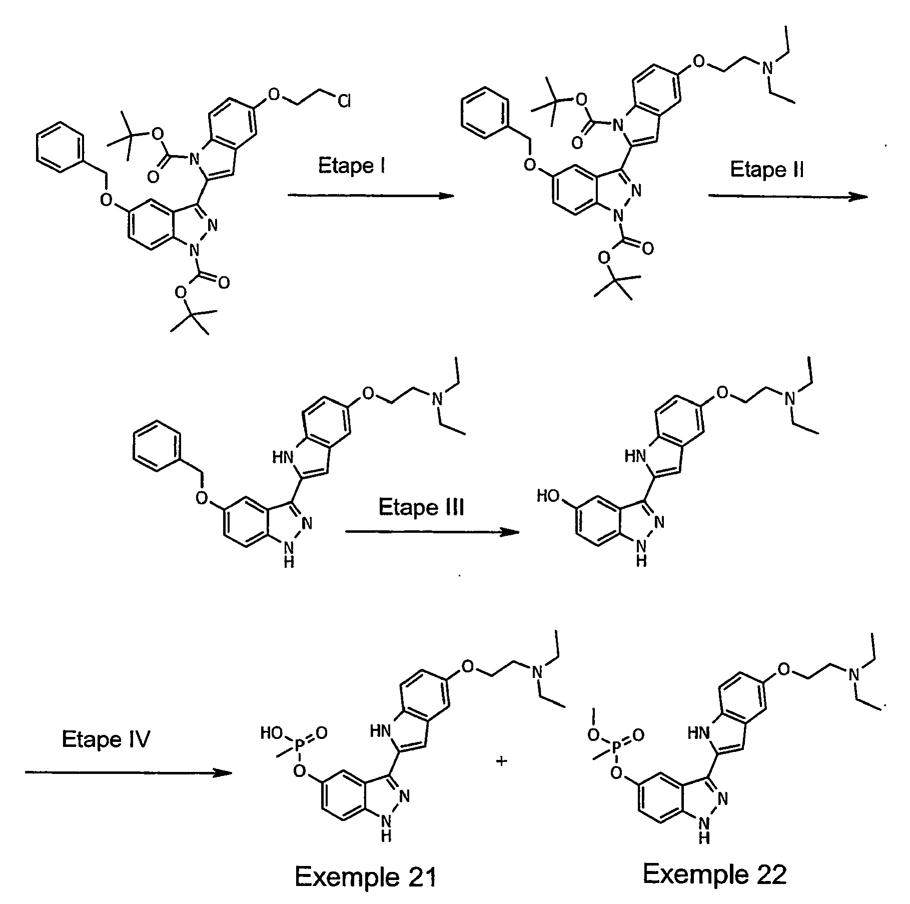

### Etape I: préparation de 5-benzyloxy-3-[1-tert-butoxycarbonyl-5-(2-diethyl-amino-ethoxy)-1H-indol-2-yl]-indazole-1-carboxylic acid tert-butyl ester

Une solution contenant 1.5 g de 5-benzyloxy-3-[1-tert-butoxycarbonyl-5-(2-chloro-ethoxy)-1H-indol-2-yl]-indazole-1-carboxylic acid tert-butyl ester préparé selon la procédure J, 1 g de carbonate de potassium, 600 mg d'iodure de potassium et 761 µl d'éthyle amine dans 75 ml d'acétonitrile est chauffée à 80°C pendant 18 heures. Après cette période, on ajoute 1.5 ml d'éthyle amine et on poursuit la réaction pendant 18 heures supplémentaires à 80°C. Cette opération est renouvelée après encore 18 heures puis le mélange réactionnel est évaporé sous pression réduite. L'huile brune obtenue est reprise dans un mélange d'acétate d'éthyle (80 ml) et d'eau (80 ml). Après décantation et extraction avec l'acétate d'éthyle (80 ml), les extraits organiques sont réunis, lavés à l'eau (100 ml) et à la saumure (100 ml), séchés sur sulfate de magnésium puis évaporés sous pression réduite. On isole 1.15 g de 5-benzyloxy-3-[1-tert-butoxycarbonyl-5-(2-diethylamino-ethoxy)-1H-indol-2-yl]-indazole-1-carboxylic acid tert butyl ester que l'on utilise tel quel dans l'étape suivante.

### Etape II: préparation de {2-[2-(5-benzyloxy-1H-indazol-3-yl)-1H-indol-5-yloxy]-ethyl}-diethyl-amine

Une solution contenant 1.15 g de 5-benzyloxy-3-[1-tert-butoxycarbonyl-5-(2-diethylamino-ethoxy)-1H-indol-2-yl]-indazole-1-carboxylic acid tert butyl ester et 4 ml d'acide trifluoroacétique dans 5 ml de dichlorométhane est agitée à 20°C pendant 2 heures. Le mélange réactionnel est évaporé sous pression réduite, le composé brut ainsi obtenu est purifié par chromatographie (colonne Nucléodur C18, 100-10, 250 mm x 40 mm, référence N°762020, n°série 3051181, n° lot 2023 ; éluant A : eau/acide trifluoroacétique 0.07 %, éluant B : acétonitrile/acide trifluoroacétique 0.07 %, Gradient de composition A/B de 95 % / 5 % à 5 % / 95 % sur 52 minutes à 75 ml/min, détection 300 nm). Les fractions contenant le composé attendu sont réunies et évaporées sous pression réduite. Le composé est repris dans l'acétate d'éthyle (20 ml), séché sur sulfate de magnésium puis évaporé sous pression réduite. On isole et caractérise 320 mg de {2-[2-(5-benzyloxy-1H-indazol-3-yl)-1H-indol-5-yloxy]-ethyl}-diethyl-amine.

### Etape III: préparation de 3-[5-(2-diethylamino-ethoxy)-1H-indol-2-yl]-1H-indazol-5-ol

Une suspension de 320 mg de {2-[2-(5-benzyloxy-1H-indazol-3-yl)-1H-indol-5-yloxy]-ethyl}-diethyl-amine, de 32 mg de palladium sur charbon à 10 %, de 180 mg de formiate d'ammonium est irradiée dans un four à micro-ondes à pression atmosphérique Synthwave 402 pendant 35 minutes à 75°C à 5% puissance puis pendant 10 minutes à 20 % de puissance. Le catalyseur est filtré sur un lit de célite, le filtrat est évaporé sous pression réduite. Le composé obtenu est repris dans l'acétate d'éthyle (20 ml) et une solution saturée de bicarbonate de sodium (20 ml), décanté, séché sur sulfate de magnésium puis évaporé sous pression réduite. On isole et caractérise 115 mg de 3-[5-(2-diethylamino-ethoxy)-1H-indol-2-yl]-1 H-indazol-5-ol.

LC/MS analytique : [M+H]⁺= 365.29 ;.Temps de rétention = 2.33 minutes

### Etape IV: préparation de methyl-phosphonic acid mono-{3-[5-(2-diethyl-amino-ethoxy)-1H-indol-2-yl]-1H-indazol-5-yl} ester et methyl-phosphonic acid 3-[5-(2-diethylamino-ethoxy)-1H-indol-2-yl]-1 H-indazol-5-yl ester methyl ester

Une suspension de 115 mg de 3-[5-(2-diethylamino-ethoxy)-1H-indol-2-yl]-1H-indazol-5-ol dans 3 ml dichlorométhane est agitée à 20°C pendant l'addition de 107 mg de methylphosphonic acid bis-(4-nitrophenyl)ester (préparé selon la procédure E) et de 48 µl de 1,8-diazabicyclo [5.4.0]undec-7-ene. Après 18 heures à cette température, on ajoute 128 µl de méthanol et de 50 µl de 1,8-diazabicyclo [5.4.0]undec-7-ene dans 1 ml de tétrahydrofurane et on poursuit la réaction pendant 4 heures. Le mélange réactionnel est évaporé sous pression réduite. On isole 300 mg d'un composé que l'on purifie par chromatographie (colonne Nucléodur C18, 100-10, 250 mm x 40 mm, référence N°762020, n°série 3051181, n°lot 2023; éluant A : eau/acide trifluoroacétique 0.07 %, éluant B : acétonitrile/acide trifluoroacétique 0.07 %, Gradient de composition A/B de 95 % / 5% à 5 % / 95 % sur 52 minutes à 75 ml/min, détection 300 nm).

Les fractions contenant le methyl-phosphonic acid mono-{3-[5-(2-diethylamino-ethoxy)-1H-indol-2-yl]-1H-indazol-5-yl} ester (MM 442) sont réunies et évaporées sous pression réduite.

Les fractions contenant le Methyl-phosphonic acid 3-[5-(2-diethylamino-ethoxy)-1H-indol-2-yl]-1H-indazol-5-yl ester methyl ester (MM 456 ) sont réunies et évaporées sous pression réduite.

Chaque composé isolé ci-dessus est repris dans 500 µl de méthanol puis déposé sur une cartouche de type SCX (Varian, 500 mg). Après le dépôt, la cartouche est d'abord rincée au méthanol, puis éluée avec un mélange de méthanol et d'ammoniac 2M. Les éluats sont évaporés sous pression réduite. Les composés obtenus sont purifiés par LCMS préparative.

Les fractions contenant le composé de masse molaire 442 sont réunies et évaporées sous pression réduite. On isole 22.9 mg de methyl-phosphonic acid mono-{3-[5-(2-diethylamino-ethoxy)-1H-indol-2-yl]-1 H-indazol-5-yl} ester **(exemple 21),** que l'on caractérise par LC/MS analytique.

LC/MS analytique : [M+H]⁺ = 442.18 ; temps de rétention = 2.43 minutes

Les fractions contenant le composé de masse molaire 456 sont réunies et évaporées sous pression réduite. On isole 12.8 mg de methyl-phosphonic acid 3-[5-(2-diethylamino-ethoxy)-1H-indol-2-yl]-1H-indazol-5-yl ester methyl ester **(exemple 22)** que l'on caractérise par LC/MS.

LC/MS analytique : [M+H]⁺ = 456.19 ; temps de rétention = 2.76 minutes

### Exemple 23: préparation de phenyl-phosphonic acid ethyl ester 3-(5-methoxy-1H-pyrrolo[3,2-b]pyridin-2-yl)-1H-indazol-5-yl ester

Le composé est préparé en 6 étapes selon le schéma suivant :

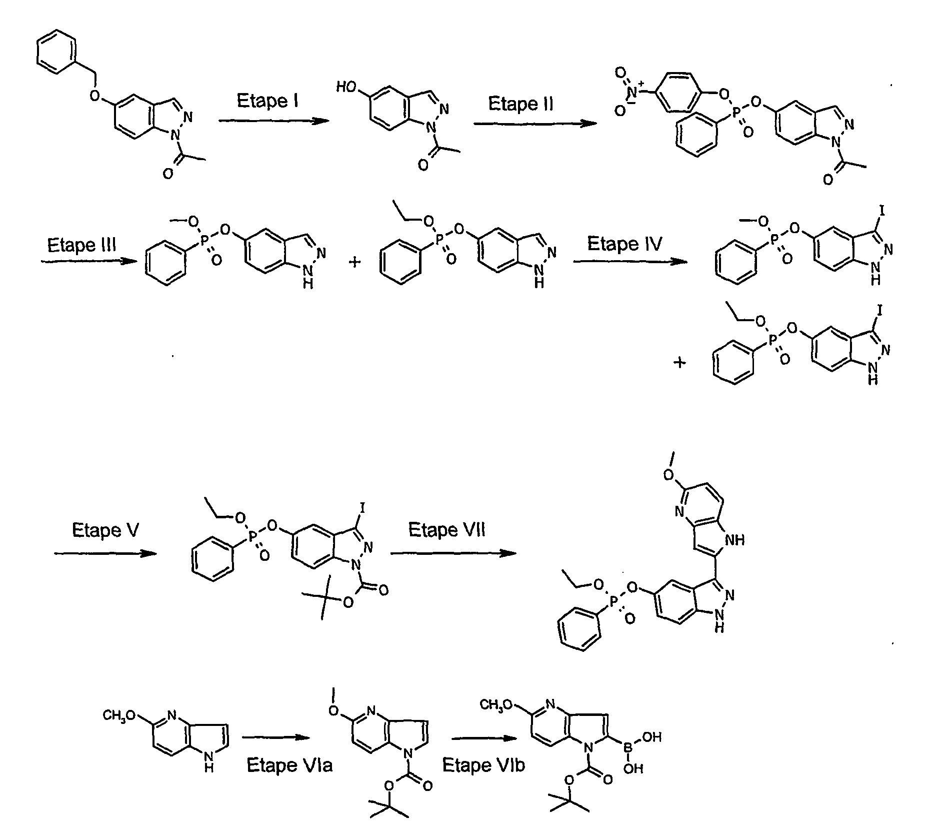

La procédure K correspond aux étapes I à V.

### Etape I : préparation du phenyl-phosphonic acid ethyl ester 3-(5-methoxy-1H-pyrrolo[3,2-b]pyridin-2-yl)-1 H-indazol-5-yl ester

Le 1-(5-benzyloxy-indazol-1-yl)-ethanone est préparé selon la méthode décrite lors de l'étape 2 de synthèse de l'intermédiaire A.

Une suspension de 1 g de 1-(5-benzyloxy-indazol-1-yl)-ethanone, de 1.42 g de formiate d'ammonium et de 0.59 g de palladium à 10 % sur charbon dans l'éthanol est chauffée au reflux pendant 16 heures. Après retour à 20°C, le mélange réactionnel est filtré sur un lit de célite et le filtrat est évaporé sous pression réduite. Le composé brut ainsi obtenu est trituré dans 10 ml d'éther diisopropylique, puis filtré et séché sous pression réduite. On isole et caractérise 400 mg de 1-(5-hydroxy-indazol-1-yl)-éthanone.

### Etape II : préparation de phenyl-phosphonic acid 1-acetyl-1H-indazol-5-yl ester 4-nitro-phenyl ester

Une solution de 2.36 g de phenylphosphonic acid bis-(4-nitrophenyl)ester (préparé selon la procédure E) dans 60 ml de dichlorométhane est refroidie dans un bain de glace. On y ajoute 1.041 g 1-(5-hydroxy-indazol-1-yl)-éthanone et 900 mg de 1,8-diazabicyclo[5.4.0]undec-7-ene dans 40 ml de dichlorométhane et on poursuit la réaction à 20°C pendant 2 heures. Le milieu réactionnel est lavé par une solution saturée de bicarbonate de sodium jusqu'à décoloration de la phase organique. Après décantation les extraits organiques sont séchés sur sulfate de magnésium puis évaporés sous pression réduite. On isole 2.4 g de phenyl-phosphonic acid 1-acetyl-1H-indazol-5-yl ester 4-nitro-phenyl ester que l'on utilise sans purification dans l'étape suivante.

### Etape III: préparation de phenyl-phosphonic acid 1H-indazol-5-yl ester methyl ester

Une solution de 2.4 g de phenyl-phosphonic acid 1-acetyl-1H-indazol-5-yl ester 4-nitro-phenyl ester dans 30 ml de dichlorométhane stabilisé sur éthanol est agitée à 20°C. On y ajoute une solution de 3.78 ml de méthanol et de 836 mg de 1,8-diazabicyclo[5.4.0]undec-7-ene dans 30 ml de dichlorométhane. La réaction est poursuivue à cette température pendant une nuit. Le mélange réactionnel est évaporé sous pression réduite, puis le composé brut ainsi obtenu est purifié par chromatographie sur silice ( AIT BP-SUP 20-40 µm, éluant dichlorométhane/méthanol 90/10). Les fractions contenant le composé attendu sont réunies et évaporées sous pression réduite. On isole et caractérise 980 mg de phenyl-phosphonic acid 1H-indazol-5-yl ester methyl ester.

LC/MS analytique : [M+H ]⁺= 289.13 ; Temps de rétention = 2.83 minutes

Ce composé est contaminé par le phenyl-phosphonic acid 1H-indazol-5-yl ester ethyl ester, que l'on n'isole pas.

LC/MS analytique : M+H ]⁺= 303.0 ; Temps de rétention = 3.01 minutes

### Etape IV: préparation de phenyl-phosphonic acid 3-iodo-1H-indazol-5-yl ester methyl ester

Attention, au cours de cette réaction, l'utilisation de la matirère première décrite à l'étape III, contenant 25 % de l'isomère éthyle ester, permet d'isoler également le dérivé iodé correspondant.

Une solution de 980 mg de phenyl-phosphonic acid 1H-indazol-5-yl ester methyl ester contaminé par le phenyl-phosphonic acid 1 H-indazol-5-yl ester ethyl ester, dans le diméthylformamide est placée sous vive agitation à 20°C. On y ajoute 1.72 g d'iode et 381.5 mg d'hydroxyde de potassium puis on laisse réagir pendant 16 heures. Le milieu réactionnel est dilué dans un mélange d'acétate d'éthyle (60 ml) et d'une solution saturée de thiosulfate de sodium (40 ml). Après 10 minutes d'agitation à 20°C, le milieu réactionnel est décanté, lavé à l'eau distillée (40 ml) ; les extraits organiques sont réunis, séchés sur sulfate de magnésium puis évaporés sous pression réduite. On isole ainsi 1.08 g de composé brut que l'on purifié par chromatographie (colonne Nucléodur C18, 100-10, 250 mm x 40 mm, référence N°762020, n°série 3051181, n°lot 2023 ; éluant A : eau/acide trifluoroacétique 0.07 %, éluant B : acétonitrile/acide trifluoroacétique 0.07 %, Gradient de composition A/B de 95 % / 5 % à 5 % / 95 % sur 52 minutes à 75 ml/min).

Les fractions contenant le composé de masse molaire 414 sont réunies et évaporées sous pression réduite. On isole et caractérise 510 mg de phenyl-phosphonic acid 3-iodo-1H-indazol-5-yl ester methyl ester.

Les fractions contenant le composé de masse molaire 428 sont réunies et évaporées sous pression réduite. On isole et caractérise 80 mg de phenyl-phosphonic acid 3-iodo-1 H-indazol-5-yl ester éthyle ester.

### Etape V : préparation de 5-(ethoxy-phenyl-phosphinoyloxy)-3-iodo-indazole-1-carboxylic acid tert-butyl ester

Une solution de 80 mg de phenyl-phosphonic acid 3-iodo-1H-indazol-5-yl ester éthyle ester dans 2 ml dichlorométhane est agitée à 20°C. On y ajoute 40 mg de di-tertio-butyle dicarbonate et de 22 mg de diméthylamino pyridine et on poursuit la réaction à 20°C pendant 16 heures. Le mélange réactionnel est évaporé sous pression réduite, on isole et caractérise 60 mg de 5-(ethoxy-phenyl-phosphinoyloxy)-3-iodo-indazole-1-carboxylic acid tert-butyl ester utilisé tel quel.

LC/MS analytique : [M+H]⁺ = 529.06 ; Temps de rétention = 4.29 minutes

### Etape VI: préparation de 5-methoxy-pyrrolo[3,2-b]pyridine-2-boronic acid 1-carboxylic acid tert-butyl ester

Ce composé est préparé en deux étapes à partir de 5-methoxy-pyrrolo[3,2-b]pyridine, commé décrit ci-dessous.

### Etape VIa: préparation de 5-methoxy-pyrrolo[3,2-b]pyridine-1-carboxylic acid tert-butyl ester

Sous agitation magnétique et à 20°C, 102 mg de 4-diméthyl-aminopyridine sont ajoutés à une solution de 4.50 g de 5-methoxy-pyrrolo[3,2-b]pyridine (préparé comme décrit dans Liebigs Ann. Chem. 1988, 203-208) et de 10.7 g de di-tert-butyl dicarbonate dans 10 ml de dichlorométhane anhydre. La solution obtenue est agitée à température ambiante pendant la nuit puis le milieu réactionnel est lavé avec 75 ml d'eau puis 75 ml de saumure. L'extrait organique est séché sur sulfate de magnésium puis évaporé sous pression réduite. Le composé brut ainsi obtenu est purifié par chromatographie sur silice en éluant au dichlorométhane puis avec un mélange de dichlorométhane et d'acétate d'éthyle 90/10 pour donner 7.06 g de 5-methoxy-pyrrolo[3,2-b]pyridine-1-carboxylic acid tert-butyl ester sous forme d'une huile ambrée que l'on caractérise par RMN.

RMN : 1 H NMR [300 Mhz, (CD3)2SO]: δ 8.21 (d, J = 9 Hz, 1 H), 7.85 (d, J = 4 Hz, 1 H), 6.76 (d, J = 9 Hz, 1 H), 6.70 (d, J = 4 Hz, 1 H), 3.89 (s, 3H), 1.63 (s, 9H).

### Etape VIb : introduction de l'acide boronique

Une solution de 15 ml de ter-butyl lithium 1.5M dans le pentane est ajoutée par portion à une solution de 4.66 g de 5-methoxy-pyrrolo[3,2-b]pyridine-1-carboxylic acid tert-butyl ester (préparé ci-dessus) dans 85 ml de tétrahydrofurane anhydre maintenue sous un courant d'azote sec. Le milieu réactionnel ainsi obtenu est agité à -78°C pendant 40 minutes puis 8 ml d'une solution de tri-isopropyl borate (37.7 mmol) est ajoutée sur une période de 2 minutes, puis le milieu réactionnel est agité et maintenu à-78°C pendant 20 minutes. Le milieu réactionnel est chauffé à 0°C pendant 2,5 heures, puis on ajoute 50 ml d'eau. Après une heure d'agitation à 20°C, le tétrahydrofurane est évaporé sous pression réduite. La phase aqueuse obtenue est basifiée par addition d'hydroxyde d'ammonium 5N puis lavée deux fois à l'acétate d'éthyle (30 ml). L'extrait aqueux est refroidit à 0°C puis traité par une solution aqueuse de sulfate acide de potassium jusqu'à pH4. Le milieu est agité à 0°C pendant 15 minutes. Le solide foré est isolé par filtration, séché pour fournir 2.48 g de 5-methoxy-pyrrolo[3,2-b]pyridine-2-boronic acid 1-carboxylic acid tert-butyl ester sous forme d'une poudre blanche.

RMN [300 Mhz, (CD₃)₂SO]: δ 8.28 (s, 2H), 8.23 (d, J = 9 Hz, 1 H), 6.70 (d, J = 9 Hz, 1 H), 6.58 (s, 1 H), 3.87 (s, 3H), 1.60 (s, 9H).

### Etape VII : préparation de phenyl-phosphonic acid ethyl ester 3-(5-methoxy-1H-pyrrolo[3,2-b]pyridin-2-yl)-1H-indazol-5-yl ester

Une suspension de 50 mg 5-(ethoxy-phenyl-phosphinoyloxy)-3-iodo-indazole-1-carboxylic acid tert-butyl ester, de 55.3 mg de 5-methoxy-pyrrolo[3,2-b]pyridine-2-boronic acid 1-carboxylic acid tert-butyl ester, de 3.78 mg de 1,1'-bis(diphenylphosphino)ferrocene-palladium(ii)dichloride et de 123.4 mg de carbonate de césium dans un mélange de 800 µl de dioxane eau et 250 µl d'eau est chauffée à 100°C pendant 45 minutes. Après retour à 20°C, le milieu réactionnel est dilué par 3 ml d'acétate d'éthyle puis lavé par 2 fois 1.5 ml d'eau distillée. Après décantation l'extrait organique est séché sur sulfate de magnésium puis évaporé sous pression réduite. Le composé brut obtenu est purifié par LCMS préparative. Les fractions contenant le composé attendu sont réunies et évaporées sous pression réduite. On isole et caractérise 16.7 mg de phenyl-phosphonic acid ethyl ester 3-(5-méthoxy-1H-pyrrolo[3,2-b]pyridin-2-yl)-1H-indazol-5-yl ester.

LC/MS analytique : [M+H]⁺ = 449.17 ; Temps de rétention = 3.05 minutes

### Exemple 24: procédure L - préparation de phenyl-phosphonic acid 3-(5-methoxy-1H-pyrrolo[3,2-b]pyridin-2-yl)-1H-indazol-5-yl ester methyl ester

Dans un réacteur, on place une suspension composée de 50 mg de 3-iodo-5-(methoxy-phenyl-phosphinoyloxy)-indazole-1-carboxylic acid tert-butyl ester obtenu selon la procédure K, 3.9 mg de 1,1'-bis(diphenylphosphino) ferrocene-palladium(ii)dichloride, 56.81 mg de 5-methoxy-pyrrolo[3,2-b] pyridine-1-carboxylic acid tert-butyl ester 2-boronic acid, 126 mg de carbonate de césium; 260 µl de dioxane et 813 µl d'eau distillée puis on chauffe le mélange réactionnel à 100°C pendant 45 minutes. Après retour à 20°C, le milieu réactionnel est dilué par 4 ml d'acétate d'éthyle puis lavé par 2 fois 3 ml d'eau distillée. Après décantation la phase organique est séchée sur sulfate de magnésium puis évaporée sous pression réduite. Le composé brut obtenu est purifié par LCMS préparative. Les fractions contenant le composé attendu sont réunies et évaporées sous pression réduite.

Le composé intermédiaire ainsi obtenu est placé en solution dans 300 µl de dioxane chlorhydrique 1 M et agité pendant 2 heures à 20°C, puis le mélange réactionnel est évaporé sous pression réduite. Le composé brut obtenu est purifié par LCMS préparative. Les fractions contenant le composé attendu sont réunies et évaporées sous pression réduite.

On isole et caractérise 3 mg de phenyl-phosphonic acid 3-(5-methoxy-1H-pyrrolo[3,2-b]pyridin-2-yl)-1H-indazol-5-yl ester methyl ester.

LC/MS analytique : [M+H]⁺ = 435 ; Temps de rétention = 2.95 minutes

### Exemple 25: préparation de phenyl-phosphonic acid methyl ester 3-styryl-1 H-indazol-5-yl ester

Le composé est préparé selon la procédure L avec 28.78 mg de trans-beta-styreneboronic acid. On isole et caractérise 3 mg de phenyl-phosphonic acid methyl ester 3-styryl-1 H-indazol-5-yl ester.

LC/MS analytique : [M+H]⁺ = 391 ; Temps de rétention = 3.66 minutes

### Exemple 26: préparation de phenyl-phosphonic acid 3-benzo[b]thiophen-2-yl-1 H-indazol-5-yl ester methyl ester

Le composé est préparé selon la procédure L avec 24.89 mg de thiophene-2-boronic acid. On isole et caractérise 3 mg de phenyl-phosphonic acid 3-benzo [b]thiophen-2-yl-1 H-indazol-5-yl ester methyl ester.

LC/MS analytique : [M+H]⁺ = 371 ; Temps de rétention = 3.41 minutes

### Exemple 27: Phenyl-phosphonic acid 3-benzo[b]thiophen-2-yl-1H-indazol-5-yl ester methyl ester

Le composé est préparé selon la procédure L avec 30 mg de 3-iodo-5-(methoxy-phenyl-phosphinoyloxy)-indazole-1-carboxylic acid tert-butyl ester, 21.2 mg de benzo[b]thiophène-2-boronic acid, 2.23 mg de 1,1'-bis(diphenyl-phosphino)ferrocene-palladium(ii)dichloride), 71 mg de carbonate de césium, dans 500 µl de diméthylformamide. On isole 0.7 mg de phenyl-phosphonic acid 3-benzo[b]thiophen-2-yl-1 H-indazol-5-yl ester methyl ester.

LC/MS analytique : [M+H]⁺ = 421.18 ; Temps de rétention = 3.86 minutes

### Exemple 28: Préparation de methyl-phosphonic acid methyl ester 3-[5-(2-morpholin-4-yl-ethoxy)-1H-indol-2-yl]-1H-indazol-5-yl ester

Le produit est préparé en 7 étapes à partir de l'intermédiaire A.

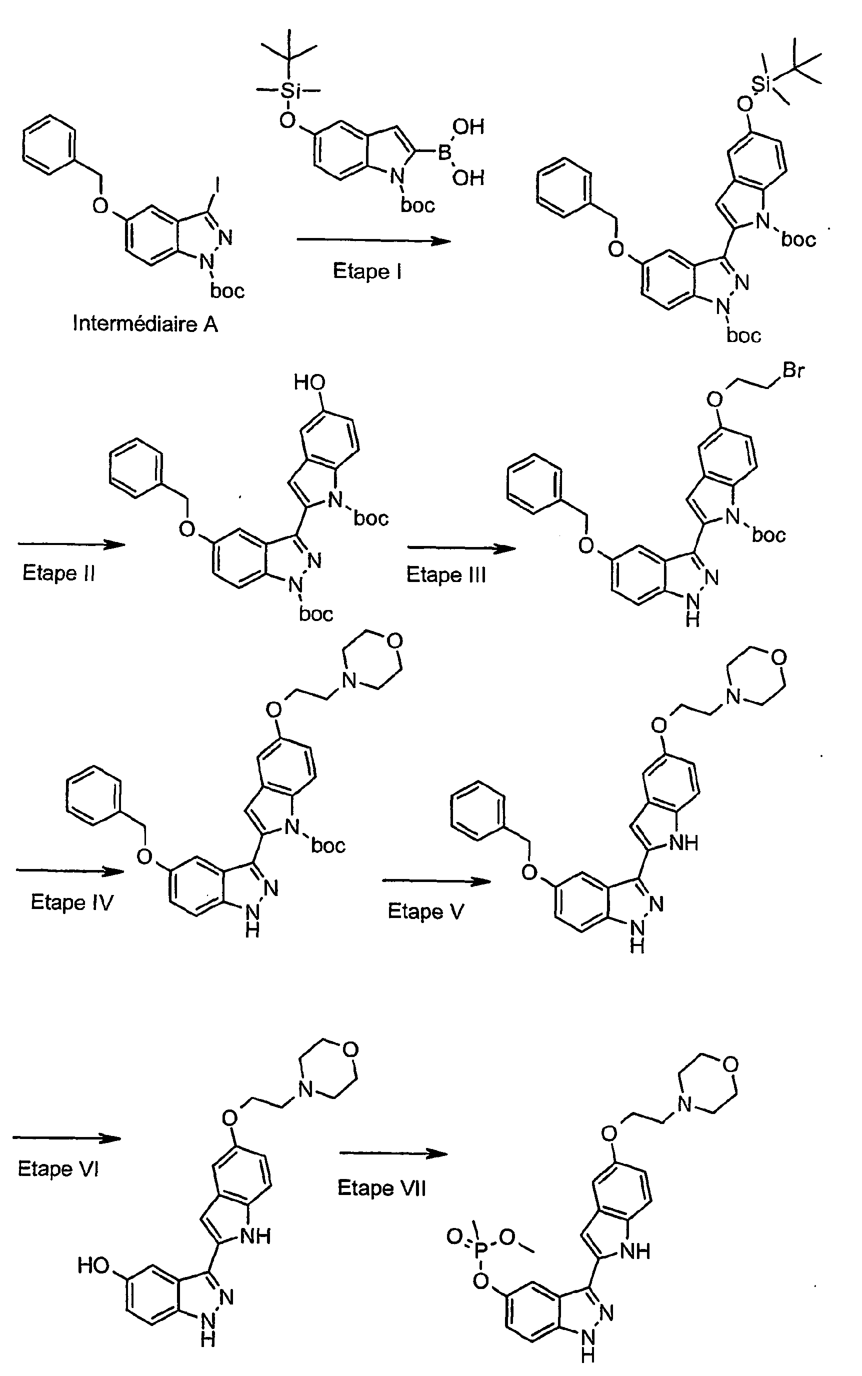

### Etape I: Préparation de 5-benzyloxy-3-[1-tert-butoxycarbonyl-5-(tert-butyl-dimethyl-silanyloxy)-1H-indol-2-yl]-indazole-1-carboxylic acid tert-butyl ester (Procédure M)

A une solution de 10 g de 5-benzyloxy-3-iodo-indazole-1-carboxylic acid tert-butyl ester (intermédiaire A) dans 156 ml de dioxanne sont ajoutés successivement 13 g d'acide 1-(tert-butyldimethylsilyloxy)-5-indole-2-boronique, 28.9 g de carbonate de césium, 906.5 mg de [1.1'-bis(diphenyl-phospino)ferrocene]dichloropalladium II en complexe avec le dichlorométhane puis 51 ml d'eau distillée. Le mélange réactionnel est chauffé pendant 45 minutes par un bain d'huile préalablement chauffé à 100°C. Le mélange est refroidi à température ambiante par un bain d'eau. Le milieu est décanté. La phase inférieure est écartée (environ 50 ml) et la phase organique est concentrée sous vide. La gomme brune obtenue est solubilisée dans 250 ml de dichlorométhane et la phase organique est lavée par 3 fois 50 ml d'eau distillée. La phase organique est séchée sur sulfate de magnésium et charbon activé puis filtrée sur papier et concentrée à l'évaporateur rotatif. Le brut réactionnel est purifié par chromatographie flash sur 500 g de silice 40-63 µm, avec pour éluant un mélange cyclohexane/dichlorométhane 40/60. 13.4 g de 5-benzyloxy-3-[1-tert-butoxycarbonyl-5-(tert-butyl-dimethyl-silanyloxy)-1H-indol-2-yl]-indazole-1-carboxylic acid tert-butyl ester sont isolés.

### Etape II : Préparation de 5-benzyloxy-3-(1-tert-butoxycarbonyl-5-hydroxy-1H-indol-2-yl)-indazole-1-carboxylic acid tert-butyl ester

Une solution de 13.4 g de 5-benzyloxy-3-[1-tert-butoxycarbonyl-5-(tert-butyl-dimethyl-silanyloxy)-1H-indol-2-yl]-indazole-1-carboxylic acid tert-butyl ester et 6.15 g de fluorure de tétrabutylammonium dans 140 ml de tétrahydrofurane anhydre est agitée à température ambiante pendant 30 minutes. Le solvant est évaporé sous vide. Le brut réactionnel est repris par 50 ml de dichlorométhane et la phase organique est lavée par 2 fois 25 ml d'eau distillée. La phase organique est séchée sur sulfate de magnésium. Après filtration et concentration sous vide, le brut réactionnel est purifié par chromatographie flash sur 450 g de silice 40-63 µm. On élue avec du dichlorométhane 100 % puis un mélange dichlorométhane/méthanol 98/2 puis 95/5. 8.54 g de 5-benzyloxy-3-(1-tert-butoxycarbonyl-5-hydroxy-1H-indol-2-yl)-indazole-1-carboxylic acid tert-butyl ester sont isolés.

LC/MS analytique : Tr = 4.75 min ; [M+H]⁺ = 446.34

### Etape III : Préparation de 5-benzyloxy-3-[5-(2-bromo-ethoxy)-1-tert-butoxycarbonyl-1 H-indol-2-yl]-indazole-1-carboxylic acid tert-butyl ester

2.22 g de 5-benzyloxy-3-(1-tert-butoxycarbonyl-5-hydroxy-1H-indol-2-yl)-indazole-1-carboxylic acid tert-butyl ester, 7.8 g de carbonate de césium dans 22 ml de dibromoéthane sont agités à 80°C (température du bain d'huile) pendant 48 heures. Le mélange réactionnel est filtré sur verre fritté, le solide est rincé par 20 ml de dichlorométhane. Le filtrat est concentré sous vide. Le brut réactionnel est purifié par chromatographie flash sur 160 g de silice. L'éluant est du dichlorométhane 100 %. 2 g de 5-benzyloxy-3-[5-(2-bromo-ethoxy)-1-tert-butoxycarbonyl-1H-indol-2-yl]-indazole-1-carboxylic acid tert-butyl ester sont isolés.

### Etape IV : Préparation de 5-benzyloxy-3-[1-tert-butoxycarbonyl-5-(2-morpholin-4-yl-ethoxy)-1 H-indol-2-yl]-indazole-1-carboxylic acid tert-butyl ester (Procédure N)

2.0 g de 5-benzyloxy-3-[5-(2-bromo-ethoxy)-1-tert-butoxycarbonyl-1H-indol-2-yl]-indazole-1-carboxylic acid tert-butyl ester sont agités dans 90 ml d'acétonitrile. 498 mg d'iodure de potassium sont ajoutés et la suspension est chauffée à 80°C. Après 7 heures 20 minutes, sont ajoutés successivement les réactifs suivants : 394 µl de morpholine, 1.24 g de carbonate de potassium, 150 mg d'iodure de potassium. La suspension est chauffée à 80°C pendant la nuit. L'insoluble est filtré et le filtrat est concentré sous vide. Le brut réactionnel est repris dans 50 ml de dichlorométhane. La phase organique est lavée par 2 fois 25 ml d'eau distillée. La phase organique est séchée sur sulfate de magnésium et filtrée. Le solvant est évaporé à l'évaporateur rotatif. 1.90 g de 5-benzyloxy-3-[1-tert-butoxycarbonyl-5-(2-morpholin-4-yl-ethoxy)-1 H-indol-2-yl]-indazole-1-carboxylic acid tert-butyl ester sont isolés.

LC/MS analytique : Tr = 3.99 min ; [M+H]⁺ = 669.43

### Etape V: Préparation de 5-benzyloxy-3-[5-(2-morpholin-4-yl-ethoxy)-1H-indol-2-yl]-1 H-indazole

1.0 g de 5-benzyloxy-3-[1-tert-butoxycarbonyl-5-(2-morpholin-4-yl-ethoxy)-1 H-indol-2-yl]-indazole-1-carboxylic acid tert-butyl ester sont agités à température ambiante pendant 18 heures dans 6 ml d'une solution d'acide chlorhydrique 4M dans le dioxanne. Le produit est filtré sur verre fritté, rincé au dioxanne. 692.4 mg de 5-benzyloxy-3-[5-(2-morpholin-4-yl-ethoxy)-1H-indol-2-yl]-1H-indazole sont recueillis.

LC/MS analytique : Tr = 3.05 min ; [M+H]⁺ = 469.34

### Etape VI: Préparation de 3-[5-(2-morpholin-4-yl-ethoxy)-1H-indol-2-yl]-1H-indazol-5-ol

1.12 g de 5-benzyloxy-3-[5-(2-morpholin-4-yl-ethoxy)-1H-indol-2-yl]-1H-indazole sont mis en solution dans 55 ml d'éthanol absolu puis on ajoute successivement 424 mg de palladium sur charbon, 587 mg de formiate d'ammonium, 227 µl de triéthylamine. Le milieu réactionnel est agité à 67°C (température interne de la suspension). Un fort dégagement gazeux est observé. Après une heure sous agitation, le milieu est filtré sur papier, le catalyseur est rincé à l'éthanol absolu. Le filtrat est concentré sous vide. 558 mg de 3-[5-(2-morpholin-4-yl-ethoxy)-1H-indol-2-yl]-1H-indazol-5-ol sont recueillis.

LC/MS analytique : Tr = 2.29 min ; [M+H]⁺ = 379.37

### Etape VII : Préparation de methyl-phosphonic acid methyl ester 3-[5-(2-morpholin-4-yl-ethoxy)-1H-indol-2-yl]-1H-indazol-5-yl ester

Une suspension de 170 mg methylphosphonic acid bis-(4-nitrophenyl)ester (préparé selon la procédure E) et 189.7 mg de 3-[5-(2-morpholin-4-yl-ethoxy)-1H-indol-2-yl]-1H-indazol-5-ol dans 17 ml de dichlorométhane (stabilisé sur amylène) est agitée à température ambiante. On ajoute goutte à goutte en 10 minutes une solution de 75 µl de DBU dans 500 µl de dichlorométhane. On maintient l'agitation pendant une nuit. 204 µl de méthanol sont additionnés avec 75 µl de DBU. La solution est agitée pendant 24 heures. Le milieu réactionnel est concentré sous vide puis le brut est repris par 25 ml d'acétate d'éthyle et lavé par 4 fois 25 ml d'eau distillée. La phase organique est séchée sur sulfate de magnésium, filtrée et concentrée sous vide. Le brut réactionnel est purifié par chromatographie flash sur 5 g de silice 40-63 µm. Eluants : dichlorométhane/méthanol 98/2 puis 95/5. 95 mg de methyl-phosphonic acid methyl ester 3-[5-(2-morpholin-4-yl-ethoxy)-1H-indol-2-yl]-1 H-indazol-5-yl ester sont recueillis.

LC/MS analytique : Tr = 2.25 min ; [M+H]⁺ = 471.11

### Exemple 29: Préparation de methyl-phosphonic acid 3-[6-(2-diethylamino-ethoxy)-1H-indol-2-yl]-1 H-indazol-5-yl ester methyl ester

Le composé est préparé en 10 étapes selon le schéma 7 suivant :

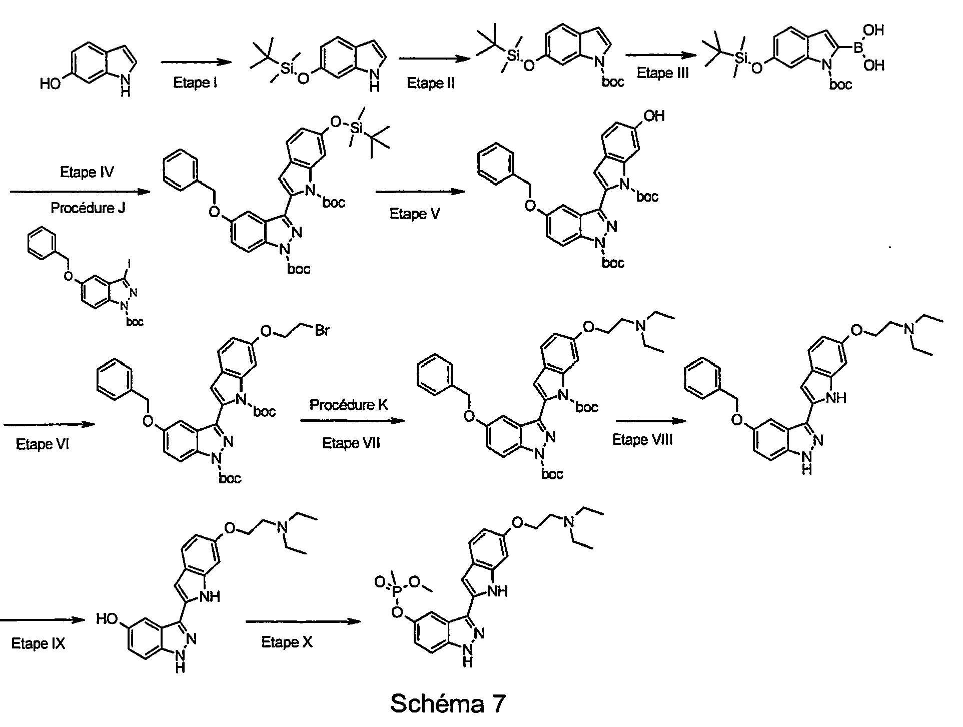

### Etape I : préparation de 6-(tert-butyl-dimethyl-silanyloxy)-1H-indole

Une solution de 3.52 g de 6-hydroxyindole, 4.78 g de chlorure de tert-butyldimethylsilyle, 4.5 g d'imidazole dans 16 ml de diméthylformamide est agitée à température ambiante pendant une nuit. Le milieu réactionnel est dilué avec de l'acétate d'éthyle puis la phase organique est lavée par 2 fois 50 ml d'eau distillée. La phase organique est séchée sur sulfate de magnésium, filtrée et le solvant est évaporé à l'évaporateur rotatif. 6.5 g de 6-(tert-butyl-dimethyl-silanyloxy)-1H-indole sont recueillis.

LC/MS analytique : Tr = 4.55 min ; [M+H]⁺ = 248.30

### Etape II : préparation de 6-(tert-butyl-dimethyl-silanyloxy)-indole-1-carboxylic acid tert-butyl ester

Une solution de 6.5 g 6-(tert-butyl-dimethyl-silanyloxy)-1H-indole, 9.23 g de di-tert-butyldicarbonate, 646 mg de 4-diméthylaminopyridine dans 65 ml de dichlorométhane est agitée à température ambiante. Après 4 heures d'agitation, le solvant est évaporé à l'évaporateur rotatif et le brut réactionnel est purifié par chromatographie flash sur silice 35-70 µm, éluant: cyclohexane. On isole 9.20 g de 6-(tert-butyl-dimethyl-silanyloxy)-indole-1-carboxylic acid tert-butyl ester sous forme d'huile jaune.

LC/MS analytique : Tr = 6.0 min ; [M+H]⁺ = 348.3

### Etape III : préparation de 6-(tert-butyl-dimethyl-silanyloxy)-indole-1-carboxylic acid tert-butyl 3-boronic acid

Une solution de 8.20 g de 6-(tert-butyl-dimethyl-silanyloxy)-indole-1-carboxylic acid tert-butyl ester dans 120 ml de tétrahydrofurane anhydre est refroidie à -78°C par un bain de carboglace dans l'acétone. 19 ml de tert-butyllithium 1.5M dans le pentane sont additionnés goutte à goutte en 40 minutes. La solution est agitée à -78°C pendant 30 minutes. On additionne alors 5.3 ml de triméthylborate. Après réchauffement du milieu à 0°C, la solution est agitée à cette température pendant 2 heures. 75 ml d'une solution aqueuse saturée de chlorure d'ammonium est ajoutée ainsi que 200 ml d'éther éthylique. Le milieu est agité pendant 20 minutes à température ambiante. Après acidification du milieu par 60 ml d'une solution aqueuse à 10 % de NaHSO₄ et 2 ml d'acide sulfurique concentré, la phase organique est décantée et lavée par 120 ml d'eau distillée et 120 ml d'une solution aqueuse saturée de chlorure de sodium. Après séchage sur sulfate de magnésium et filtration, le solvant est évaporé. Le solide obtenu est lavé par du cyclohexane et essoré sur verre fritté. On recueille 5.79 g de 6-(tert-butyl-dimethyl-silanyloxy)-indole-1-carboxylic acid tert-butyl 3-boronic acid.

### Etape IV : Préparation de 5-benzyloxy-3-[1-tert-butoxycarbonyl-6-(tert-butyl-dimethyl-silanyloxy)-1 H-indol-2-yl]-indazole-1-carboxylic acid tert-butyl ester

Le composé est préparé selon la procédure M à partir de 5.12 g d'intermédiaire A, 5.79 g de 6-(tert-butyl-dimethyl-silanyloxy)-indole-1-carboxylic acid tert-butyl 3-boronic acid, 466 mg de [1.1'-bis(diphenyl-phospino)ferrocene]dichloropalladium II en complexe avec le dichlorométhane, 14.82 g de carbonate de césium en suspension dans un mélange eau (30 ml) et dioxanne (70 ml). Le milieu réactionnel est chauffé à 105°C pendant 1 heure 30 minutes. Après traitement le brut réactionnel est purifié par chromatographie flash sur silice 35-70 µm, éluant : cyclohexane. On recueille 5.56 g de 5-benzyloxy-3-[1-tert-butoxycarbonyl-6-(tert-butyl-dimethyl-silanyloxy)-1H-indol-2-yl]-indazole-1-carboxylic acid tert-butyl ester.

### Etape V : préparation de 5-benzyloxy-3-(1-tert-butoxycarbonyl-6-hydroxy-1H-indol-2-yl)-indazole-1-carboxylic acid tert-butyl ester

1.50 g de 5-benzyloxy-3-[1-tert-butoxycarbonyl-6-(tert-butyl-dimethyl-silanyl-oxy)-1H-indol-2-yl]-indazole-1-carboxylic acid tert-butyl ester en solution dans 35 ml de tétrahydrofurane avec 770 mg de fluorure de tétrabutylammonium hydraté sont agités à température ambiante. Après 1 heure 30 minutes d'agitation, le milieu est dilué par du dichlorométhane puis la phase organique est lavée par de l'eau distillée. Après séchage sur sulfate de magnésium et filtration, le solvant est évaporé sous vide à l'évaporateur rotatif. On recueille 1.05 g de de 5-benzyloxy-3-(1-tert-butoxycarbonyl-6-hydroxy-1H-indol-2-yl)-indazole-1-carboxylic acid tert-butyl ester.

LC/MS analytique : Tr = 4.73 min ; [M+H]⁺ = 556.06

### Etape VI : Préparation de 5-benzyloxy-3-[6-(2-bromo-ethoxy)-1-tert-butoxy-carbonyl-1 H-indol-2-yl]-indazole-1-carboxylic acid tert-butyl ester

Une solution de 1.05 g de 5-benzyloxy-3-(1-tert-butoxycarbonyl-6-hydroxy-1H-indol-2-yl)-indazole-1-carboxylic acid tert-butyl ester dans 10.4 ml de dibromoéthane est agitée à température ambiante. 1.85 g de carbonate de césium sont additionnées et le milieu est chauffé à 80°C pendant 24 heures. Le solvant est évaporé et le brut est repris par un mélange eau/acétate d'éthyle. La phase organique est séchée sur sulfate de magnésium. Après filtration, le solvant est évaporé à l'évaporateur rotatif. Le brut réactionnel est purifié par chromatographie flash sur cartouche de 50 g de silice, par un gradient cyclohexane/acétate d'éthyle 95/5 à 65/35 en 60 minutes. On recueille 1.23 g 5-benzyloxy-3-[6-(2-bromo-ethoxy)-1-tert-butoxycarbonyl-1H-indol-2-yl]-indazole-1-carboxylic acid tert-butyl ester.

LC/MS analytique : Tr = 5.43 min ; [M+H]⁺ = 664.04

### Etape VII : Le composé 5-benzyloxy-3-[1-tert-butoxycarbonyl-6-(2-diethyl-amino-ethoxy)-1H-indol-2-yl]-indazole-1-carboxylic acid tert-butyl ester est préparé selon la procédure N à partir de1.23 g de 5-benzyloxy-3-[6-(2-bromo-ethoxy)-1-tert-butoxycarbonyl-1H-indol-2-yl]-indazole-1-carboxylic acid tert-butyl ester, 50 ml d'acétonitrile, 401 mg d'iodure de potassium, 204 mg de diéthylamine, et 770 mg de carbonate de césium. Après traitement le brut réactionnel est purifié par chromatographie sur cartouche de 20 g de silice ; éluant : dichlorométhane/méthanol 98/2, 95/5, 92/8. On recueille 390 mg de 5-benzyloxy-3-[1-tert-butoxycarbonyl-6-(2-diethylamino-ethoxy)-1H-indol-2-yl]-indazole-1-carboxylic acid tert-butyl ester.

LC/MS analytique : Tr = 4.62 min ; [M+H]⁺ = 655.45

Etape VIII : le composé {2-[2-(5-benzyloxy-1H-indazol-3-yl)-1H-indol-6-yloxy]-ethyl}-diethylamine est préparé de la manière suivante : 390 mg de 5-benzyl-oxy-3-[1-tert-butoxycarbonyl-6-(2-diethylamino-ethoxy)-1H-indol-2-yl]-indazole-1-carboxylic acid tert-butyl ester en solution dans 5 ml de dichlorométhane et 2 ml d'acide trifluoroacétique sont agités à température ambiante pendant 20 heures. Le solvant est évaporé sous vide à l'évaporateur rotatif. On recueille 480 mg de {2-[2-(5-benzyloxy-1H-indazol-3-yl)-1H-indol-6-yloxy]-ethyl}-diethyl-amine sous forme de sel de trifluoroacétate.

LC/MS analytique : Tr = 3.87 min ; [M+H]⁺ = 455.51

Etape IX : le composé 3-[6-(2-diethylamino-ethoxy)-1H-indol-2-yl]-1H-indazol-5-ol est préparé de la manière suivante : 990 mg de {2-[2-(5-benzyloxy-1H-indazol-3-yl)-1H-indol-6-yloxy]-ethyl}-diethylamine en solution dans 15 ml d'éthanol absolu en présence de 100 mg de palladium sur charbon et 1.1 g de formiate d'ammonium sont chauffés dans un four à micro-ondes à pression atmosphérique à 90°C pendant 30 minutes. Le brut réactionnel est filtré sur célite, le catalyseur est rincé à l'éthanol absolu et le filtrat est concentré sous vide. Le brut réactionnel est repris dans 80 ml d'acétate d'éthyle et lavé par 2 fois 50 ml d'une solution aqueuse saturée de bicarbonate de sodium. Après séchage sur sulfate de magnésium la phase organique est concentrée à sec. Le solide obtenu est lavé au dichlorométhane et à l'éther isopropylique. On recueille 280 mg de 3-[6-(2-diethylamino-ethoxy)-1H-indol-2-yl]-1H-indazol-5-ol.

Etape X : le composé methyl-phosphonic acid 3-[6-(2-diethylamino-ethoxy)-1H-indol-2-yl]-1H-indazol-5-yl ester methyl ester est préparé comme suit : 100mg de 3-[6-(2-diethylamino-ethoxy)-1H-indol-2-yl]-1H-indazol-5-ol en solution dans 4 ml de dichlorométhane avec 93 mg de methylphosphonic acid bis-(4-nitrophenyl)ester (préparé selon la procédure E) sont agités à température ambiante. Goutte à goutte est additionnée une solution de 41 µl de 1,8-diazabicyclo[5.4.0]undec-7-ene (DBU) dans 1 ml de dichlorométhane. La solution est agitée une nuit à température ambiante. On coule alors 41 µl de DBU puis 115 µl de méthanol. Cette opération est répétée après 4 heures 15 minutes d'agitation. Après une autre heure d'agitation à température ambiante le solvant est évaporé et le brut est purifié par chromatographie flash sur une cartouche de 25 g de silice, éluant dichlorométhane/méthanol 90/10 à 60/40 par paliers. Les fractions obtenues sont concentrées et purifiées par LC/MS préparative. On recueille 30 mg de methyl-phosphonic acid 3-[6-(2-diethylamino-ethoxy)-1 H-indol-2-yl]-1 H-indazol-5-yl ester methyl ester sous forme d'une huile incolore.

Spectre RMN : De 0,99 à 1,18 (m, 6H) ; 1,70 (d, J = 17,0 Hz, 3H) ; 2,63 (m large, 4H) ; 2,85 (m large, 2H) ; 3,78 (d, J = 11,0 Hz, 3H) ; 4,05 (m large, 2H) ; 6,78 (d large, J = 9,0 Hz, 1H); 6,97 (m large, 2H) ; 7,30 (d large, J = 9,0 Hz, 1 H) ; 7,48 (d, J = 9,0 Hz, 1 H) ; 7,60 (d, J = 9,0 Hz, 1H) ; 7,89 (m large, 1H) ; 11,4 (m large, 1 H) ; 13,3 (s large, 1 H).

### Exemple 30 : Préparation d'ethyl-phosphonic acid 3-(1H-indol-2-yl)-1H-indazol-5-yl ester methyl ester

L'intermédiaire ethylphosphonic acid bis(nitrophenyl)ester est préparé selon la procédure E à partir de 528 mg d'ethylphosphonic dichloride, 1 g de p-nitrophénol, 345 mg d'hydrure de sodium (50 % dans l'huile) et 10 ml de tétrahydrofurane. 1.29 g d'ethylphosphonic acid bis(nitrophenyl)ester sont recueillis.

Le composé ethyl-phosphonic acid 3-(1H-indol-2-yl)-1H-indazol-5-yl ester methyl ester est préparé selon la procédure F à partir de 282.5 mg d'ethylphosphonic acid bis(nitrophenyl), de 200 mg de 3-(1H-indol-2-yl)-H-indazole-5-ol (intermédiaire B) en solution dans 10 ml de dichlorométhane, 120 µl de 1,8-diazabicyclo[5.4.0]undec-7-ene (DBU). Après 7 heures à température ambiante, on ajoute 120 µl de DBU et 144.5 µl de méthanol et on laisse la nuit à température ambiante. Après concentration, le brut est purifié par chromatographie sur cartouche de 50 g de silice. Eluant : cyclohexane/acétate d'éthyle 9/1 par paliers jusqu'à acétate d'éthyle/méthanol 9/1. Le solide issu de la concentration des fractions contenant l'attendu est lavé à l'acétate d'éthyle puis à l'éther éthylique. 83 mg d'ethyl-phosphonic acid 3-(1H-indol-2-yl)-1H-indazol-5-yl ester methyl ester sont recueillis.

Spectre RMN 1 H à 400 MHz sur spectromètre BRUKER AVANCE DRX-400 avec les déplacements chimiques (δ en ppm) - dans le solvant diméthylsulfoxide - d6 (DMSO-d6) référencé à 2,50 ppm :

1,17 (td, J = 7,0 et 20,0 Hz, 3H) ; de 1,95 à 2,07 (m, 2H) ; 3,77 (d, J = 11,0 Hz, 3H) ; de 6,98 à 7,07 (m, 2H) ; 7,15 (t large, J = 8,0 Hz, 1 H) ; 7,33 (d large, J = 8,0 Hz, 1 H) ; 7,46 (d large, J = 8,0 Hz, 1 H) ; de 7,58 à 7,65 (m, 2H) ; 7,92 (s large, 1 H) ; 11,6 (m large, 1H) ; 13,45 (m étalé, 1 H).

### Exemple 31 : Préparation de methyl-phosphonothioic acid O-[3-(1H-indol-2-yl)-1H-indazol-5-yl] ester O-methyl ester

L'intermédiaire methyl-phosphonothioic acid O,O-bis-(4-nitro-phenyl) ester est préparé selon la procédure E à partir de 536 mg de methylphosphonothioic dichloride, 345 mg d'hydrure de sodium (50 % dans l'huile), 1 g de p-nitrophénol, 10 ml de tétrahydrofurane. On recueille 1.19 g de produit attendu.

Le composé methyl-phosphonothioic acid O-[3-(1H-indol-2-yl)-1H-indazol-5-yl] ester O-methyl ester est préparé selon la procédure F à partir 200 mg de 3-(1H-indol-2-yl)-H-indazole-5-ol (intermédiaire B) en solution avec 284 mg de methyl-phosphonothioic acid O,O-bis-(4-nitro-phenyl) ester dans 6 ml de dichlorométhane (stabilisé sur amylène) à laquelle on ajoute 120 µl de 1,8-diazabicyclo[5.4.0]undec-7-ene (DBU) en solution dans 1 ml de dichlorométhane. Après agitation 4 heures 30 minutes à température ambiante, on ajoute 325 µl de méthanol et 120 µl de DBU en solution dans 1 ml de dichlorométhane. Après une nuit à température ambiante, le solvant est évaporé et le brut est purifié par chromatographie flash sur de la silice 35-70 µm, éluant : cyclohexane/acétate d'éthyle 90/10 jusqu'à 50/50. Les fractions obtenues sont concentrées et purifiées par LC/MS préparative. 47.7 mg de methyl-phosphonothioic acid O-[3-(1H-indol-2-yl)-1 H-indazol-5-yl] ester O-methyl ester sont recueillis.

Spectre RMN 1 H à 400 MHz sur spectromètre BRUKER AVANCE DRX-400 avec les déplacements chimiques (δ en ppm) - dans le solvant diméthylsulfoxide - d6 (DMSO-d6) référencé à 2,50 ppm :

2,11 (d, J = 15,0 Hz, 3H) ; 3,81 (d, J = 14,0 Hz, 3H) ; 7,02 (t large, J = 9,0 Hz, 1 H) ; de 7,07 à 7,14 (m, 2H) ; 7,28 (d large, J = 9,0 Hz, 1 H) ; 7,44 (d large, J = 9,0 Hz, 1H) ; de 7,58 à 7,65 (m, 2H ) ; 7,49 (m, 1 H) ; 11,6 (m large, 1 H) ; 13,35 (s large, 1H).

### Exemple 32: Préparation de cyclohexyl-phosphonic acid 3-(1H-indol-2-yl)-1 H-indazol-5-yl ester methyl ester

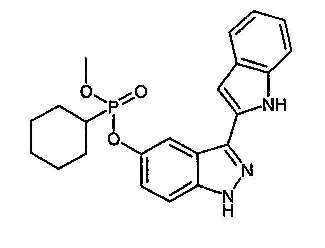

L'intermédiaire cyclohexylphosphonic acid bis(nitrophenyl)ester est préparé selon la procédure E à partir de 722 mg de cyclohexylphosphonic dichloride, 1 g de p-nitrophénol, 345 mg d'hydrure de sodium (50 % dans l'huile) et 10 ml de tétrahydrofurane. 1.47 g de composés attendus sont recueillis.
Rendement = quantitatif

Le composé cyclohexyl-phosphonic acid 3-(1H-indol-2-yl)-1H-indazol-5-yl ester methyl ester est préparé selon la procédure F à partir 200 mg de 3-(1 H-indol-2-yl)-H-indazole-5-ol (intermédiaire B) en solution avec 325 mg de cyclohexylphosphonic acid bis(nitrophenyl)ester dans 6 ml de dichlorométhane (stabilisé sur amylène) à laquelle on ajoute 120 µl de 1,8-diazabicyclo[5.4.0]undec-7-ene (DBU) en solution dans 1 ml de dichlorométhane. Après agitation 4 heures 30 minutes à température ambiante, on ajoute 325 µl de méthanol en solution dans 1 ml de dichlorométhane. Après une nuit à température ambiante, le solvant est évaporé et le brut est purifié par chromatographie flash sur de la silice 35-70 µm, éluant : cyclohexane/acétate d'éthyle 90/10 jusqu'à 50/50. Les fractions obtenues sont concentrées et purifiées par LC/MS préparative. 64.5 mg de cyclohexyl-phosphonic acid 3-(1H-indol-2-yl)-1H-indazol-5-yl ester methyl ester sont recueillis.

Spectre RMN : déplacements chimiques (δ en ppm) - dans le solvant diméthylsulfoxide - d6 (DMSO-d6) référencé à 2,50 ppm :

De 1,20 à 1,50 (m, 6H) ; de 1,62 à 1,83 (m, 3H) ; de 1,93 à 2,15 (m, 2H) ; 3,75 (d, J = 11,0 Hz, 3H) ; de 6,96 à 7,05 (m, 2H) ; 7,11 (t large, J = 8,0 Hz, 1 H) ; 7,31 (d large, J = 9,0 Hz, 1 H) ; 7,45 (d large, J = 9,0 Hz, 1 H) ; de 7,59 à 7,64 (m, 2H) ; 7,40 (m, 1 H) ; 11,6 (m large, 1 H) ; 13,5 (m étalé, 1 H) .

### Exemple 33 : Préparation du composé phenyl-phosphonic acid mono-[3-(1H-indol-2-yl)-1H-indazol-5-yl] ester

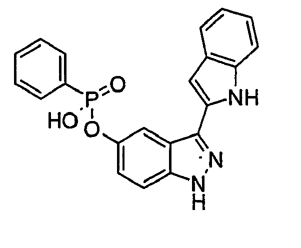

En vue de la préparation du composé [3-(1H-Indol-2-yl)-1H-indazol-5-ylmethyl]-phenyl-phosphinic acid N ethyl amide, une solution de 150 mg phenyl-phosphonic acid 3-(1H-indol-2-yl)-1H-indazol-5-yl ester 4-nitro-phenyl ester (préparé selon la procédure E) et 1.5 ml de diéthylamine (solution 2M dans le tétrahydrofurane) dans le dichlorométhane (stabilisé sur amylène) est agitée à température ambiante. On ajoute 44 µl de 1,8-diazabicyclo[5.4.0] undec-7-ene (DBU) et le milieu est agité une nuit à température ambiante. Le solvant est évaporé à l'évaporateur rotatif et le brut réactionnel est purifié par LC/MS préparative pour isoler un composé de masse [M+H]⁺ = 390, ne correspondant pas au produit attendu. On recueille 23 mg d'un solide gris identifié comme le phenyl-phosphonic acid mono-[3-(1H-indol-2-yl)-1H-indazol-5-yl] ester.

LC/MS analytique : Tr = 2.72 min ; [M+H]⁺ = 390.27

Spectre RMN : déplacements chimiques (δ en ppm) - dans le solvant diméthylsulfoxide - d6 (DMSO-d6) référencé à 2,50 ppm :

Pour le produit principal du mélange (70 %), nous avons :

6,82 (s large, 1 H) ; 7,02 (t large, J = 8,5 Hz, 1 H) ; 7,11 (t large, J = 8,5 Hz, 1 H) ; 7,21 (d large, J = 9,0 Hz, 1 H) ; 7,43 (d large, J = 9,0 Hz, 1 H) ; de 6,90 à 7,62 (m, 5H) ; 7,76 (s large, 1H); 7,82 (dd large, J = 8,5 et 12,5 Hz, 2H) ; 11,55 (m large, 1 H) ; 13,3 (s large, 1 H).

### Protocoles expérimentaux sur les tests biochimiques

### 1. FAK

L'activité inhibitrice des composés sur FAK est déterminée par une mesure de l'inhibition de l'autophosphorylation de l'enzyme en utilisant un test de fluorescence résolue dans le temps (HTRF).

L'ADNc complet de FAK humain, dont l'extrémité N-terminale a été marquée à l'histidine, a été cloné dans un vecteur d'expression baculovirus pFastBac HTc. La protéine a été exprimée et purifiée à environ 70 % d'homogénéité.

L'activité kinase est déterminée en incubant l'enzyme (4.6 µg/ml) avec différentes concentrations de composé à tester dans un tampon 50 mM Hepes pH = 7,5, 5 % glycérol, 0,03 % Triton x-100, 50 mM NaCl, 1 mM DTT, 5 mM MgCl₂, 5 µM d'ATP, 1 % DMSO final, pendant 10 minutes à 37°C. La réaction enzymatique est stoppée par l'addition de 320 mM EDTA, et le marquage est effectué dans un tampon 100 mM Hepes pH = 7,5 contenant 0.8 mM KF, 0,2 % BSA, pendant une nuit à 4°C, par l'addition dans ce tampon d'un anticorps anti-Histidine marqué avec XL665 et d'un anticorps monoclonal phosphospécifique de la tyrosine conjugué à du cryptate d'europium (Eu-K). Les caractéristiques des deux fluorophores sont disponibles dans G. Mathis et al., Anticancer Research, 1997, 17, pages 3011-3014. Le transfert d'énergie entre le cryptate d'europium excité vers le XL665 accepteur est proportionnel au degré d'autophosphorylation de FAK. Le signal de longue durée spécifique de XL-665 est mesuré dans un compteur de plaques Packard Discovery. Tous les essais sont effectués en double exemplaire et la moyenne des deux essais est calculée. L'inhibition de l'activité d'autophosphorylation de FAK avec des composés de l'invention est exprimée en pourcentage d'inhibition par rapport à un contrôle dont l'activité est mesurée en l'absence de composé test. Pour le calcul du % d'inhibition, le ratio [signal à 665 nm/signal à 620 nm] est considéré.

### 2. KDR

L'effet inhibiteur des composés est déterminé dans un test de phosphorylation de substrat par l'enzyme KDR *in vitro* par une technique de scintillation (plaque 96 puits, NEN).

Le domaine cytoplasmique de l'enzyme KDR humaine a été cloné sous forme de fusion GST dans le vecteur d'expression baculovirus pFastBac. La protéine a été exprimée dans les cellules SF21 et purifiée à environ 60 % d'homogénéité.

L'activité kinase de KDR est mesurée dans 20 mM MOPS, 10 mM MgCl₂, 10 mM MnCl2, 1 mM DTT, 2.5 mM EGTA, 10 mM b-glycérophosphate, pH = 7.2, en présence de 10 mM MgCl₂, 100 µM Na₃VO₄, 1 mM NaF. 10 µl du composé sont ajoutés à 70 µl de tampon kinase contenant 100 ng d'enzyme KDR à 4°C. La réaction est lancée en ajoutant 20 µl de solution contenant 2 µg de substrat (fragment SH2-SH3 de la PLCγ exprimée sous forme de protéine de fusion GST), 2 µCi γ ³³P[ATP] et 2 µM ATP froid. Après 1 heure d'incubation à 37°C, la réaction est stoppée en ajoutant 1 volume (100 µl) de 200 mM EDTA. Le tampon d'incubation est retiré, et les puits sont lavés trois fois avec 300 µl de PBS. La radioactivité est mesurée dans chaque puits en utilisant un compteur de radioactivité Top Count NXT (Packard).

Le bruit de fond est déterminé par la mesure de la radioactivité dans quatre puits différents contenant l'ATP radioactif et le substrat seul.

Un contrôle d'activité totale est mesuré dans quatre puits différents contenant tous les réactifs (γ³³P-[ATP], KDR et substrat PLCγ) mais en l'absence de composé.

L'inhibition de l'activité KDR avec le composé de l'invention est exprimée en pourcentage d'inhibition de l'activité contrôle déterminée en l'absence de composé.

Le composé SU5614 (Calbiochem) (1 µM) est inclus dans chaque plaque comme contrôle d'inhibition.

### 3. Aurora2

L'effet inhibiteur de composés vis-à-vis de la kinase Aurora2 est déterminé par un test de scintillation par radioactivité utilisant du nickel chélate.

Une enzyme Aurora2 recombinante complète, dont l'extrémité N-terminale a été marquée à l'histidine, a été exprimée dans *E. coli* et purifiée jusqu'à une qualité proche de l'homogénéité.

Le fragment C-terminal (Q1687-H2101) d'une NuMA (protéine Nucléaire qui s'associe avec l'Appareil Mitotique) exprimé dans *E. coli*, et dont l'extrémité N-terminale a été marquée à l'histidine, a été purifié par chromatographie au nickel chélate et utilisé comme substrat dans le test de la kinase Aurora2.

Pour déterminer l'activité kinase, le substrat NuMA est équilibré par chromatographie sur une colonne PD10 Pharmacia, dans un tampon (50 mM Tris-HCl, pH7.5, 50 mM NaCl, 10 mM MgCl₂) additionné de 10 % (v/v) de glycérol et de 0.05 % (w/v) de NP40.

L'activité kinase d'Aurora2 est mesurée par scintillation avec du nickel chélate (New England Nuclear, modèle SMP107). Chaque puits contient 100 µl de la solution suivante : 0.02 µM d'Aurora2 ; 0.5 µM de substrat NuMA; 1 µM d'ATP additionné de 0.5 µCi d'ATP-[³³P]. Les solutions sont incubées pendant 30 minutes à 37°C. Le tampon du test est ensuite éliminé et les puits sont rincés deux fois avec 300 µl de tampon kinase. La radioactivité est mesurée dans chaque puits à l'aide d'un appareil Packard Model Top Count NXT.

Le bruit de fond est déduit de la mesure de radioactivité par mesure en double exemplaire dans des puits contenant de l'ATP radioactif seul contenant de la kinase tamponnée traitée de la même manière que les autres échantillons.

L'activité du contrôle est effectuée en mesurant en double exemplaire la radioactivité dans le mélange complet du test (ATP, Aurora2 et le substrat NuMA), en l'absence de composé test.

L'inhibition de l'activité de Aurora2 avec un composé de l'invention est exprimée en pourcentage d'inhibition de l'activité de contrôle en l'absence de composé test. De la staurosporine est ajoutée dans chaque plaque comme contrôle d'inhibition.

### 4. CDK2/cycline E :

### Purification du complexe CDK2/CyclineE-(His)₆ par IMAC (Immobilized Metal Affinity Chromatography)

Deux baculovirus recombinants portant les séquences humaines codant respectivement pour CDK2 et la CyclineE (cette dernière comportant un tag hexa-histidine en C terminal) sont utilisés pour co-infecter des cellules d'insecte Sf21. Deux à trois jours après le début de la co-infection, les cellules sont récoltées par centrifugation, puis conservées à -40°C jusqu'à leur utilisation. Après décongélation et lyse mécanique des cellules, le complexe présent dans le surnageant de lyse est purifié par chromatographie d'affinité sur Nickel (IMAC), et conservé à -80°C.

### Essai Flashplate CDK2/CyclinE en format 96 puits.

Un format en plaques 96 puits coatés à la streptavidine est utilisé pour tester l'activité des composés sur l'activité kinase de CDK2/Cycline E.

Pour réaliser cet essai, le substrat peptidique biotynilé, fragment de la protéine pRb, (biotinyl-SACPLNLPLQNNHTAADMYLSPVRSPKKKGSTTR-OH) est solubilisé à la concentration de 1 mM dans du tampon kinase (HEPES/ NaOH 50 mM, NaCl 1 mM, MgCl₂ 5 mM, pH 7.5) afin de constituer une solution-stock conservée à -20°C sous forme d'aliquots de 110 µl. Le jour de l'expérience, un aliquot de cette solution est décongelé et dilué dans du tampon kinase contenant 1 mM de Dithiothréitol, ajouté au tampon extemporanément, afin d'obtenir une concentration de 14.3 µM. 70 µl de cette solution sont ajoutés dans chaque puits de la Flashplate afin d'obtenir une concentration finale en substrat de 10 µM lors de la réaction enzymatique conduite dans un volume final du milieu réactionnel de 100 µl (cf. ci-après).

Des dilutions intermédiaires d'inhibiteurs (produits de l'invention) à différentes concentrations sont préparées dans le DMSO à partir de solutions stock à 10 mM dans des tubes séparés. On réalise ainsi des dilutions à 1000 µM, 333.3 µM, 111.1 µM, 37.03 µM, 12.35 µM, 4.11 µM et 1.37 µM. Un µl de chacune de ces solutions (ou 1 µl de DMSO pour les contrôles) est transferré dans les puits de la plaque de test.

Dans chaque puits, sont ensuite ajoutés 19 µl d'une solution d'un mélange d'adénosinetriphosphate (ATP) et d'ATPy³³P dans le tampon kinase à la concentration de 5,26 µM d'ATP total et de 52,6 µCi/ml de ³³P. La réaction enzymatique est déclenchée par addition de 10 µl par puits d'une solution de CDK2/Cycline E à 200 nM dans le tampon kinase contenant 1 mM de dithiothréitol (ou 10µl de tampon kinase contenant 1 mM de dithiothréitol pour les blancs réactionnels).

Après addition de chacun des réactifs, le volume final de chaque puits est de 100µl, la concentration finale de substrat est de 10 µM, les concentrations finales en inhibiteurs sont de10 µM, 3,33 µM, 1,11 µM, 0,37 µM, 0,123 µM, 0,041 µM et 0,014 µM (selon la concentration de la dilution intermédiaire), la concentration finale en ATP est de 1 µM, la quantité finale de ³³P est de 1µCi/puits, la concentration finale de complexe CDK2/Cycline E est de 20 nM.

Après l'addition de tous les réactifs, la plaque de test incubée à 30 °C sous agitation orbitale à 650 rpm.

Lorsque l'incubation est terminée, la plaque est lavée trois fois par 300 µl par puits de PBS (Phosphate Buffered Saline, pH=7,4 sans calcium ni magnésium, référence 10010-015, Gibco BRL). L'incorporation de ³³P au peptide est quantifiée par comptage par scintillation avec un appareil Packard Topcount.NXT. L'activité inhibitrice des produits de l'invention est évaluée par mesure de la concentration d'inhibiteur permettant une diminution de l'activité enzymatique de 50 % (CI50).

### 5. Tie2

La séquence codante de Tie2 humain correspondant aux acides aminés du domaine intracellulaire 776-1124 a été générée par PCR en utilisant le cDNA isolé de placenta humain comme modèle. Cette séquence a été introduite dans un vecteur d'expression *baculovirus* pFastBacGT sous forme de protéine de fusion GST.

L'effet inhibiteur des molécules est déterminé dans un test de phosphorylation de PLC par Tie2 en présence de GST-Tie2 purifiée à environ 80 % d'homogénéité. Le substrat est composé des fragments SH2-SH3 de la PLC exprimée sous forme de protéine de fusion GST.

L'activité kinase de Tie2 est mesurée dans un tampon MOPS 20mM pH 7.2, contenant 10 mM MgCl₂, 10 mM MnCl₂, 1 mM DTT, 10 mM de glycérophosphate. Dans une plaque 96 puits FlashPlate maintenue sur glace, on dépose un mélange réactionnel composé de 70 µl de tampon kinase contenant 100 ng d'enzyme GST-Tie2 par puits. Ensuite 10 µl de la molécule à tester diluée dans du DMSO à une concentration de 10 % maximum sont ajoutés. Pour une concentration donnée, chaque mesure est effectuée en quatre exemplaires. La réaction est initiée en ajoutant 20 µl de solution contenant 2 µg de GST-PLC, 2 µM d'ATP froid et 1 µCi d'³³P[ATP]. Après 1 heure d'incubation à 37°C, la réaction est stoppée en ajoutant 1 volume (100 µl) d'EDTA à 200 mM. Après élimination du tampon d'incubation, les puits sont lavés trois fois avec 300 µl de PBS. La radioactivité est mesurée sur un MicroBeta1450 Wallac.

L'inhibition de l'activité Tie2 est calculée et exprimée en pourcentage d'inhibition par rapport à l'activité contrôle déterminée en l'absence de composé.

Les produits des exemples selon l'invention présentent en général une activité sur les différentes kinases et particulièrement sur Tie2 et Aurora-2 estimée par la concentration inhibant 50 % de l'activité de la kinase comprise entre 3 nM et 500 nM.

| Exemple | Au2 IC50 (nM) | CDK2 IC50 (nM) | FAK IC50 (nM) | KDR IC50 (nM) | TIE2 IC50 (nM) |
|---|---|---|---|---|---|
| 01 | 37 | 234 | 279 | 158 | 40 |
| 02 | 75 | 991 | 3525 | 508 | 518 |
| 03 | 20 | 142 | 816 | 310 | 3 |
| 04 | 99 | 586 | 2896 | 1634 | 22 |
| 05 | 15 | 394 | 48 | 141 | 11 |
| 06 | 15 | 184 | 740 | 288 | 48 |
| 07 | 24 | 959 | 9639 | 2048 | 162 |
| 08 | 53 | 3700 | 8300 | 5400 | 130 |
| 09 | 27 | 54 | 3019 | 344 | 138 |
| 10 | 9130 | >10000 | >10000 | | >10000 |
| 11 | 90 | 199 | 1136 | 1250 | 229 |
| 12 | 140 | 290 | 10000 | 2033 | 2307 |
| 13 | 69 | | | | |
| 14 | 76 | 780 | 5881 | 1660 | 290 |
| 15 | 108 | 2260 | >10000 | >10000 | 4084 |
| 16 | 2200 | >10000 | >10000 | >10000 | 2211 |
| 17 | 770 | | | | |
| 18 | 20 | 33 | 7570 | 228 | 33 |
| 19 | 55 | 113 | | 121 | 33 |
| 20 | 96 | 7772 | | 1415 | 96 |
| 21 | 74 | 4467 | | 133 | 214 |
| 22 | 64 | 152 | | 174 | 44 |
| 23 | 24 | 249 | | 442 | 30 |
| 24 | 24 | 142 | | 364 | 22 |
| 25 | 41 | 578 | | | |
| 26 | 730 | 3236 | | | |
| 27 | 134 | 7561 | | 1883 | 353 |
| 28 | 25 | 74 | 1444 | 205 | 119 |
| 29 | 17 | 128 | | 122 | 13 |
| 30 | 15 | 87 | 4539 | 215 | 90 |
| 31 | 19 | 125 | 9990 | 296 | 99 |
| 32 | 26 | 1496 | 10000 | 999 | 119 |
| 33 | 10 | 291 | 316 | 577 | 17 |

## Revendications

1. Produit de formule générale (I) suivante : 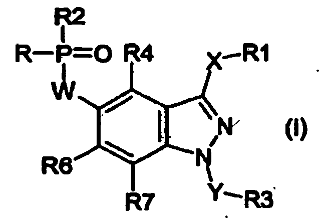 dans laquelle :
- W représente un groupe choisi parmi une liaison covalente ou O ;
- X représente une liaison covalente, un groupe -C=O-NRₐ-, NRₐ-C=O,
- (CH₂)ₙ-, -CH=CH-, -C≡C-, -NRₐ-, S, O, -SO₂-, -SO, -CO, -COO dans lequel Rₐ représente H ouun groupe (C₁-C₄)alkyle qui peut éventuellement former un cycle avec R1, et dans lequel n = 0, 1, 2, 3, 4, 5, 6, 7, 8, 9, 10, 11, ou 12 ;
- R₁ représente H (sauf quand X = -SO₂-, -SO-), alkyle, cycloalkyle, aryle, hétéroaryloe;
- R et R₂, identiques ou différents représentent H ou un groupe choisi parmi les radicaux alkyle, cycloalkyle, hétérocycloalkyle, aryle, hétéroaryle, hydroxy, alkoxy et aryloxy,
- Y représente une liaison covalente ou un radical choisi parmi: -C=O-NRₐ-,
- C=O-O-, -C=O-, -(CH₂)ₙ-, -SO₂-, dans lequel Rₐ est sélectionné dans le groupe constitué par H, (C₁-C₄)alkyle, et (C₁-C₄)alkyle lié à R3 pour former un cycle ;
- R₃ est sélectionné dans le groupe constitué par H (sauf quand Y est -C=O-O-, ou -SO₂-), alkyle, cycloalkyle, aryle, et hétéroaryle;
- R₄, R₆ et R₇, Identiques ou différents, peuvent être indépendamment choisis parmi : H, halogène, (C₁-C₄)alkyle, (C₁-C₄)alkoxy, cyano, -N(R_{b})R_{c}, -C=ON(R_{b})R_{c}, -N(R_{b})-CO-R_{c}, dans lequel R_{b} et R_{c} sont indépendamment choisis parmi H, (C₁-C₄)alkyle, et (C₃-C₆)cycloalkyle.

2. Produit selon la revendication 1, **caractérisé en ce que** W est O.

3. Produit selon l'une quelconque des revendications précédentes, **caractérisé en ce que** les radicaux aryle et hétéroaryle sont indépendamment choisis parmi:
(i) des radicaux monocycliques comportant de zéro à quatre hétéroatomes choisis parmi O, N et S, et
(ii) des radicaux bicycliques condensés comprenant :
(a) un radical monocyclique comportant 5, 6, 7 ou 8 chaînons et comportant de zéro à quatre hétéroatomes choisis parmi O, N et S, condensé à
(b) un autre cycle comportant 5 ou 6 chaînons, et comportant de zéro à trois hétéroatomes choisis parmi O, N et S.

4. Produit selon la revendication 3, **caractérisé en ce que** les radicaux aryle ou hétéroaryle sont Indépendamment sélectionnés dans le groupe constitué par : phényle, pyridyle, pyrimidyle, triazinyle, pyrrolyle, imidazolyle, thiazolyle, furyle, thiényle, indolyle, indazolyle, azaindazolyle, isobenzofuranyle, isobenzothlényle, benzimidazolyle, benzoxazolyle, benzothiazolyle, aryivinyiène, arylamido, arylcarboxamide, aralkylamine, quinoleyle, isoquinoleyle, cinnolyle, qulnazolyle, naphtyridyle, triazolyle ou tétrazolyle.

5. Produit selon la revendication 4, **caractérisé en ce que** les radicaux aryle ou hétéroaryle sont indépendamment sélectionnés dans le groupe constitué par: phényle, pyrrolyle, indolyle éventuellement substitué, et arylvinylène.

6. Produit selon la revendication 1 à 5, dans lequel X représente une liaison covalente et R₁ représente un radical hétérocyclique notamment Indolyle.

7. Produit selon la revendication 1 à 6, dans lequel R₂ représente un radical (C₁-C₄)alkyle.

8. Produit selon la revendication 1 à 7, dans lequel Y est liaison et R₃ est H.

9. Produit de formule générale (I) suivante : 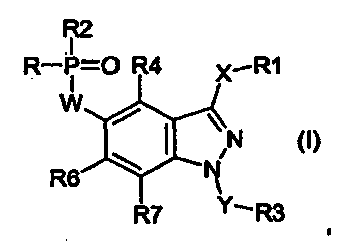 dans laquelle
- W représente un groupe choisi parmi une liaison covalente ou O ;
- X représente une liaison covalente, un groupe -C=O-NRₐ-, NRₐ-C=O,
- (CH₂)ₙ-, -CH=CH-, -C≡C-, -NRₐ-, S, O, -SO₂-, -SO, -CO, -COO dans lequel Rₐ représente H or un groupe alkyle (C₁-C₄) qui peut éventuellement former un cycle avec R1, et dans lequel n est choisi dans l'intervalle [0 à 12], ces deux bornes incluses ;
- R₁ représente H (sauf quand X = -SO₂-, -SO-), alkyle, cycloalkyle, aryle, hétéroaryle;
- R et R₂, identiques ou différents représentent H ou un groupe choisi parmi les radicaux alkyle, cycloalkyle, aryle, hétéroaryle, hydroxy, alkoxy et aryloxy,
- Y représente une liaison covalente ou un radical choisi parmi: -C=O-NRₐ-,-C=O-O-, -C=O-, -(CH₂)ₙ-, -SO₂-, dans lequel Rₐ est sélectionné dans le groupe constitué par H, (C₁-C₄)alkyle, et (C₁-C₄)alkyle lié à R3 pour former un cycle 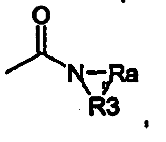
- R₃ est sélectionné dans le groupe constitué par H (sauf quand Y=C=O-O, SO₂), alkyle, cycloalkyle, aryle, et hétéroaryle;
- R₄, R₆ et R₇, identiques ou différentes, peuvent être indépendamment choisis parmi : H, halogène, (C₁-C₄)alkyle, (C₁-C₄)alkoxy, cyano, -N(R_{b})R_{c}, -C=ON(R_{b})R_{c}, -N(R_{b})-CO-R_{c}, dans lequel R_{b} et R_{c} sont Indépendamment choisis parmi H, (C₁-C₄)alkyle, et (C₃-C₆)cycloalkyle ;
en tant que médicament.

10. Produit choisi dans la liste suivante
• methyl-phenyl-phosphinic acid 3-((E)-styryl)-1H-indazol-5-yl ester
• diphenyl-phosphinic acid 3-((E)-styryl)-1H-indazol-5-yl ester
• methyl-phenylphosphinic acid 3-(1H-indol-2-yl)-1H-indazol-5-yester
• diphenyl-phosphinic acid 3-(1H-indol-2-yl)-1H-indazol-5-yl ester
• methyl-phenyl-phosphinic acid 3-[5-(2-morpholin-4-yl-ethoxy)-1H-indol-2-yl]-1H-indazol-5-yl ester
• phenyl-phosphonic acid 3-(1H-indol-2-yl)-1H-indazol-5-yl ester methyl ester
• phenyl-phosphonic acid 3-(1H-indol-2-yl)-1H-indazol-5-yl ester isopropyl ester
• phenyl-phosphonic acid benzyl ester 3-(1H-indol-2-yl)-1H-indazol-5-yl ester
• methyl-phosphonic acid 3-(1H-indol-2-yl)-1H-indazol-5-yl ester methyl ester
• phenyl-phosphonic acid methyl ester 3-(1H-pyrrol-2-yl)-1H-indazol-5-yl ester sous forme de trifluoroacétate.
• [3-((E)-styryl)-1H-indazol-5-yl]-phosphonic acid dimethyl ester
• [3-((E)-styryl)-1H-indazol-5-yl]-phosphonic acid monomethyl ester
• [3-((E)-styryl)-1H-indazol-5-yl]-phosphonic acid diethyl ester
• [3-(1H-indol-2-yl)-1H-indazol-5-yl]-phosphonic acid monomethyl ester
• (3-thiophen-2-yl-1H-indazol-5-yl)-phosphonic acid monomethyl ester
• [3-(1H-pyrrol-2-yl)-1H-indazol-5-yl]-phosphonic acid monomethyl ester
• dimethyl-phosphinic acid 3-(1H-indol-2-yl)-1H-indazol-5-yl ester
• methyl-phosphoric acid methylester 3-[5-(2-piperidin-1-yl-ethoxy)-1H-indol-2-yl]-1H-indazol-5-yl
• methyl-phosphonic acid mono-{3-[5-(2-piperazin-1-yl-ethoxy)-1H-indol-2-yl]-1H-indazol-5-yl} ester
• methyl-phosphonic acid mono-{3-[5-(2-diethylamino-ethoxy)-1H-indol-2-yl]-1H-indazol-5-yl} ester
• methyl-phosphonic acid 3-[5-(2-diethylamino-ethoxy)-1H-indol-2-yl]-1H-indazol-5-yl ester methyl ester
• phenyl-phosphonic acid ethyl ester 3-(5-methoxy-1H-pyrrolo[3,2-b]pyridin-2-yl)-1H-indazol-5-yl ester
• phenyl-phosphonic acid 3-(5-methoxy-1H-pyrrolo[3,2-b]pyridin-2-yl)-1H-indazol-5-yl ester methyl ester
• phenyl-phosphonic acid methyl ester 3-styryl-1H-indazol-5-yl ester
• phenyl-phosphonic acid 3-thiophen-2-yl-1H-indazol-5-yl ester methyl ester
• phenyl-phosphonic acid 3-benzo[b]thiophen-2-yl-1H-indazol-5-yl ester methyl ester
• methyl-phosphonic acid methyl ester 3-[5-(2-morpholin-4-yl-ethoxy)-1H-indol-2-yl]-1H-indazol-5-yl ester
• methyl-phosphonic acid 3-[6-(2-diethylamino-ethoxy)-1H-indol-2-yl]-1H-indazol-5-yl ester methyl ester
• ethyl-phosphonic acid 3-(1H-indol-2-yl)-1H-indazol-5-yl ester methyl ester
• methyl-phosphonothioic acid O-[3-(1H-indol-2-yl)-1H-indazol-5-yl] ester O-methyl ester
• cyclohexyl-phosphonic acid 3-(1H-indol-2-yl)-1H-indazol-5-yl ester methyl ester
• phenyl-phosphonic acid mono-[3-(1H-indol-2-yl)-1H-indazol-5-yl] ester

## Claims

1. Product of formula (I) below: 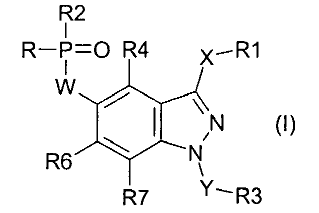 in which
- W represents a group chosen from a covalent bond or O;
- X represents a covalent bond, a group -C=O-NRₐ-, NRₐ-C=O, -(CH2)n-, -CH=CH-, -C≡C-, -NRₐ-, S, O, -SO2-, -SO,
- CO or -COO in which Rₐ represents H or a (C1-C4)alkyl group which can optionally form a ring with R1, and in which n = 0, 1, 2, 3, 4, 5, 6, 7, 8, 9, 10, 11 or 12;
- R₁ represents H (except when X = -SO2- or -SO-), alkyl, cycloalkyl, aryl or heteroaryl;
- R and R₂, which may be identical or different, represent H or a group chosen from alkyl, cycloalkyl, heterocycloalkyl, aryl, heteroaryl, hydroxyl, alkoxy and aryloxy radicals;
- Y represents a covalent bond or a radical chosen from: -C=O-NRₐ-, -C=O-O-, -C=O-, -(CH2)n- or -SO2-, in which Rₐ is selected from the group consisting of H, (C1-C4)alkyl and (C1-C4)alkyl linked to R3 so as to form a ring ;
- R₃ is selected from the group consisting of H (except when Y is -C=O-O-, or -SO₂-), alkyl, cycloalkyl, aryl, and heteroaryl;
- R₄, R₆ and R₇, which may be identical or different, can be independently chosen from: H, halogen, (C₁-C₄)alkyl, (C₁-C₄)alkoxy, cyano, -N(R_{b})R_{c}, -C=O-N(R_{b})R_{c} and
- N(R_{b})-CO-R_{c}, in which R_{b} and R_{c} are independently chosen from H, (C₁-C₄)alkyl and (C₃-C₆)cycloalkyl.

2. Product according to Claim 1, wherein W is O.

3. Product according to either one of the preceding claims, wherein the aryl and heteroaryl radicals are independently chosen from:
(i) monocyclic radicals containing from zero to four hetero atoms chosen from O, N and S, and
(ii) condensed bicyclic radicals comprising:
(a) a monocyclic radical containing 5, 6, 7 or 8 ring members and containing from zero to four hetero atoms chosen from O, N and S, condensed with
(b) another ring containing 5 or 6 ring members, and containing from zero to three hetero atoms chosen from O, N and S.

4. Product according to Claim 3, wherein the aryl or heteroaryl radicals are independently selected from the group consisting of: phenyl, pyridyl, pyrimidyl, triazinyl, pyrrolyl, imidazolyl, thiazolyl, furyl, thienyl, indolyl, indazolyl, azaindazolyl, isobenzofuranyl, isobenzothienyl, benzimidazolyl, benzoxazolyl, benzothiazolyl, arylvinylene, arylamido, arylcarboxamide, aralkylamine, quinoleyl, isoquinoleyl, cinnolyl, quinazolyl, naphthyridyl, triazolyl or tetrazolyl.

5. Product according to Claim 4, wherein the aryl or heteroaryl radicals are independently selected from the group consisting of: phenyl, pyrrolyl, optionally substituted indolyl, and arylvinylene.

6. Product according to Claims 1 to 5, in which X represents a covalent bond and R₁ represents a heterocyclic radical, in particular indolyl.

7. Product according to Claims 1 to 6, in which R₂ represents a (C₁-C₄)alkyl radical.

8. Product according to Claims 1 to 7, in which Y is a bond and R₃ is H.

9. Product of formula (I) bellow : 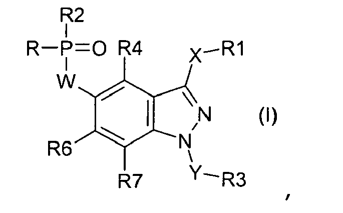 in which
- W represents a group chosen from a covalent bond or O;
- X represents a covalent bond, a group -C=O-NRₐ-, NRₐ-C=O, -(CH2)n-, -CH=CH-, -C≡C-, -NRₐ-, S, O, -SO2-, -SO,
- CO or -COO in which Rₐ represents H or a (C1-C4)alkyl group which can optionally form a ring with R1, and in which n is chosen from the range [0 to 12], these two limits inclusive;
- R₁ represents H (except when X = -SO2- or -SO-), alkyl, cycloalkyl, aryl or heteroaryl;
- R and R₂, which may be identical or different, represent H or a group chosen from alkyl, cycloalkyl aryl, heteroaryl, hydroxyl, alkoxy and aryloxy radicals;
- Y represents a covalent bond or a radical chosen from: -C=O-NRₐ-, -C=O-O-, -C=O-, -(CH2)n- or -SO2-, in which Rₐ is selected from the group consisting of H, (C1-C4)alkyl, and (C1-C4)alkyl linked to R3 so as to form a ring 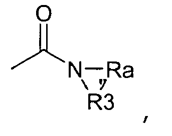
- R₃ is selected from the group consisting of H (except when Y=C=O-O or SO₂), alkyl, cycloalkyl, aryl and heteroaryl;
- R₄, R₆ and R₇, which may be identical or different, can be independently chosen from: H, halogen, (C₁-C₄)alkyl, (C₁-C₄)alkoxy, cyano, -N(R_{b})R_{c}, -C=O-N(R_{b})R_{c} and
- N(R_{b})-CO-R_{c}, in which R_{b} and R_{c} are independently chosen from H, (C₁-C₄) alkyl and (C₃-C₆) cycloalkyl; as a medicinal product.

10. Product chosen from the following list :
• methylphenylphosphinic acid 3-((E)-styryl)-1H-indazol-5-yl ester
• diphenylphosphinic acid 3-((E)-styryl)-1H-indazol-5-yl ester
• methylphenylphosphinic acid 3-(1H-indol-2-yl)-1H-indazol-5-yl ester
• diphenylphosphinic acid 3-(1H-indol-2-yl)-1H-indazol-5-yl ester
• methylphenylphosphinic acid 3-[5-(2-morpholin-4-yl-ethoxy)-1H-indol-2-yl]-1H-indazol-5-yl ester
• phenylphosphonic acid 3-(1H-indol-2-yl)-1H-indazol-5-yl ester methyl ester
• phenylphosphonic acid 3-(1H-indol-2-yl)-1H-indazol-5-yl ester isopropyl ester
• phenylphosphonic acid benzyl ester 3-(1H-indol-2-yl)-1H-indazol-5-yl ester
• methylphosphonic add 3-(1H-indol-2-yl)-1H-indazol-5-yl ester methyl ester
• phenylphosphonic acid methyl ester 3-(1H-pyrrol-2-yl)-1H-indazol-5-yl ester in trifluoroacetate form
• [3-((E)-styryl)-1H-indazol-5-yl]phosphonic acid dimethyl ester
• [3-((E)-styryl)-1H-indazol-5-yl]phosphonic acid monomethyl ester
• [3-((E)-styryl)-1H-indazol-5-yl]phosphonic acid diethyl ester
• [3-(1H-indol-2-yl)-1H-indazol-5-yl]phosphonic acid monomethyl ester
• (3-thiophen-2-yl-1H-indazol-5-yl)-phosphonic acid monomethyl ester
• [3-(1H-pyrrol-2-yl)-1H-indazol-5-yl]phosphonic acid monomethyl ester
• dimethylphosphinic acid 3-(1H-indol-2-yl)-1H-indazol-5-yl ester
• methylphosponic acid methyl ester 3-[5-(2-piperidin-1-yl-ethoxy)-1H-indol-2-yl]-1H-lndazol-5-yl ester
• methylphosphonic acid mono-{3-[5-(2-piperazin-1-ylethoxy)-1H-indol-2-yl]-1H-indazol-5-yl} ester
• methylphosphonic acid mono-{3-[5-(2-diethylaminoethoxy)-1H-indol-2-yl]-1H-indazol-5-yl} ester
• methylphosphonic acid 3-[5-(2-diethylaminoethoxy)-1H-indol-2-yl]-1H-indazol-5-yl ester methyl ester
• phenylphosphonic acid ethyl ester 3-(5-methoxy-1H-pyrrolo[3,2-b]pyridin-2-yl)-1H-indazol-5-yl ester
• methylphosphonic acid 3-[5-(2-diethylaminoethoxy)-1H-indol-2-yl]-1H-indazol-5-yl ester methyl ester
• phenylphosphonic acid ethyl ester 3-(5-methoxy-1H-pyrrolo[3,2-b]pyridin-2-yl)-1H-indazol-5-yl ester
• phenylphosphonic acid 3-(5-methoxy-1H-pyrrolo[3,2-b]pyridin-2-yl)-1H-indazol-5-yl ester methyl ester
• phenylphosphonic acid methyl ester 3-styryl-1H-indazol-5-yl ester
• phenylphosphonic acid 3-thiophen-2-yl-1H-indazol-5-yl ester methyl ester
• phenylphosphonic acid 3-benzo[b]thiophen-2-yl-1H-indazol-5-yl ester methyl ester
• methylphosphonic add methyl ester 3-[5-(2-morpholin-4-yl-ethoxy)-1H-indol-2-yl]-1H-indazol-5-yl ester
• methylphosphonic acid 3-[6-(2-diethylaminoethoxy)-1H-indol-2-yl]-1H-indazol-5-yl ester methyl ester
• othylphosphonic acid 3-(1H-indol-2-yl)-1H-indazol-5-yl ester methyl ester
• methylphosphonothioic acid O-[3-(1H-indol-2-yl)-1H-indazol-5-yl] ester O-methyl ester
• cyclohexylphosphonic acid 3-(1H-indol-2-yl)-1H-indazol-5-yl ester methyl ester
• phenylphosphonic acid mono-[3-(1H-indol-2-yl)-1H-indazol-5-yl] ester

## Patentansprüche

1. Produkt der folgenden allgemeinen Formel (I): 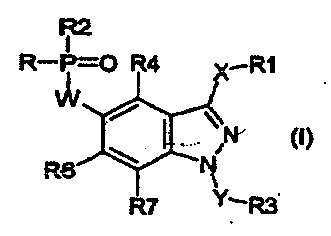 worin:
- W für eine unter einer kovalenten Bindung oder O ausgewählte Gruppe steht;
- X für eine kovalente Bindung, eine -C=O-NRₐ-, NRₐ-C=O-, -(CH₂)ₙ-, -CH=CH-, -C≡C-, -NRₐ-, S-, O-, -SO₂-, -SO-, -CO- oder -COO-Gruppe steht, wobei Rₐ für H oder eine (C₁-C₄)-Alkylgruppe, die gegebenenfalls mit R₁ einen Ring bilden kann, steht und n = 0, 1, 2, 3, 4, 5, 6, 7, 8, 9, 10, 11 oder 12;
- R₁ für H (außer wenn X = -SO₂- oder -SO-), Alkyl, Cycloalkyl, Aryl oder Heteroaryl steht;
- R und R₂ gleich oder verschieden sind und für H oder eine unter Alkyl-, Cycloalkyl-, Heterocycloalkyl-, Aryl-, Heteroaryl-, Hydroxyl-, Alkoxy- und Aryloxyresten ausgewählte Gruppe stehen;
- Y für eine kovalente Bildung oder einen unter -C=O-NRₐ-, -C=O-O-, -C=O-, -(CH₂)ₙ- oder -SO₂- ausgewählten Rest steht, wobei Rₐ aus der Gruppe bestehend aus H, (C₁-C₄)-Alkyl und unter Bildung eines Rings mit R3 verknüpftem (C₁-C₄)-Alkyl ausgewählt ist;
- R₃ aus der Gruppe bestehend aus H (außer wenn Y -C=O-O- oder -SO₂- bedeutet), Alkyl, Cycloalkyl, Aryl und Heteroaryl ausgewählt ist;
- R₄, R₆ und R₇ gleich oder verschieden sind und unabhängig voneinander unter H, Halogen, (C₁-C₄)-Alkyl, (C₁-C₄)-Alkoxy, Cyano, -N(R_{b})R_{c}, -C=O-N(R_{b})R_{c} und -N(R_{b})-CO-R_{c} ausgewählt sein können, wobei R_{b} and R_{c} unabhängig voneinander unter H, (C₁-C₄)-Alkyl und (C₃-C₆)-Cycloalkyl ausgewählt sind.

2. Produkt nach Anspruch 1, **dadurch gekennzeichnet, daß** W für O steht.

3. Produkt nach einem der vorhergehenden Ansprüche, **dadurch gekennzeichnet, daß** die Aryl- und Heteroarylreste unabhängig voneinander unter
(i) monocyclischen Resten mit null bis vier unter O, N und S ausgewählten Heteroatomen und
(ii) kondensierten bicyclischen Resten mit:
(a) einem 5-, 6-, 7- oder 8-gliedrigen monocylischen Rest mit null bis vier unter O, N und S ausgewählten Heteroatomen, kondensiert mit
(b) einem anderen 5- oder 6-gliedrigen Ring mit null bis drei unter O, N und S ausgewählten Heteroatomen,
ausgewählt sind.

4. Produkt nach Anspruch 3, **dadurch gekennzeichnet, daß** die Aryl- oder Heteroarylreste unabhängig voneinander aus der Gruppe bestehend aus Phenyl, Pyridyl, Pyrimidyl, Triazinyl, Pyrrolyl, Imidazolyl, Thiazolyl, Furyl, Thienyl, Indolyl, Indazolyl, Azaindazolyl, Isobenzofuranyl, Isobenzothienyl, Benzimidazolyl, Benzoxazolyl, Benzothiazolyl, Arylvinylen, Arylamido, Arylcarboxamid, Aralkylamin, Chinoleyl, Isochinoleyl, Cinnolyl, Chinazolyl, Naphthyridyl, Triazolyl oder Tetrazolyl ausgewählt sind.

5. Produkt nach Anspruch 4, **dadurch gekennzeichnet, daß** die Aryl- oder Heteroarylreste unabhängig voneinander aus der Gruppe bestehend aus Phenyl, Pyrrolyl, gegebenenfalls substituiertem Indolyl und Arylvinylen ausgewählt sind.

6. Produkt nach Anspruch 1 bis 5, worin X für eine kovalente Bindung steht und R₁ für einen heterocyclischen Rest, insbesondere Indolyl, steht.

7. Produkt nach Anspruch 1 bis 6, worin R₂ für einen (C₁-C₄)-Alkylrest steht.

8. Produkt nach Anspruch 1 bis 7, worin Y für eine Bindung steht und R₃ für H steht.

9. Produkt der folgenden allgemeinen Formel (I): 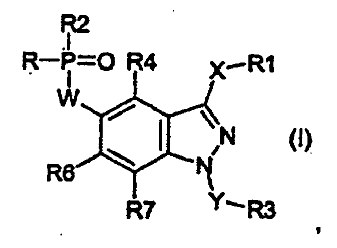 worin:
- W für eine unter einer kovalenten Bindung oder O ausgewählte Gruppe steht;
- X für eine kovalente Bindung, eine -C=O-NRₐ-, NRₐ-C=O-, -(CH₂)ₙ-, -CH=CH-, -C≡C-, -NRₐ-, S-, O-, -SO₂-, -SO-, -CO- oder -COO-Gruppe steht, wobei Rₐ für H oder eine (C₁-C₄) -Alkylgruppe, die gegebenenfalls mit R₁ einen Ring bilden kann, steht und n aus dem Intervall [0 bis 12] einschließlich der beiden Grenzen ausgewählt ist;
- R₁ für H (außer wenn X = -SO₂- oder -SO-), Alkyl, Cycloalkyl, Aryl oder Heteroaryl steht;
- R und R₂ gleich oder verschieden sind und für H oder eine unter Alkyl-, Cycloalkyl-, Aryl-, Heteroaryl-, Hydroxyl-, Alkoxy- und Aryloxyresten ausgewählte Gruppe stehen;
- Y für eine kovalente Bildung oder einen unter -C=O-NRₐ-, -C=O-O-, -C=O-, - (CH₂)ₙ- oder -SO₂- ausgewählten Rest steht, wobei Rₐ aus der Gruppe bestehend aus H, (C₁-C₄)-Alkyl und unter Bildung eines Rings 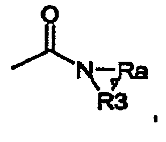 mit R3 verknüpftem (C₁-C₄)-Alkyl ausgewählt ist;
- R₃ aus der Gruppe bestehend aus H (außer wenn Y=C=O-O, SO₂), Alkyl, Cycloalkyl, Aryl und Heteroaryl ausgewählt ist;
- R₄, R₆ und R₇ gleich oder verschieden sind und unabhängig voneinander unter H, Halogen, (C₁-C₄)-Alkyl, (C₁-C₄)-Alkoxy, Cyano, -N(R_{b})R_{c}, -C=O-N(R_{b})R_{c} und -N(R_{b})-CO-R_{c} ausgewählt sein können, wobei R_{b} und R_{c} unabhängig voneinander unter H, (C₁-C₄)-Alkyl und (C₃-C₆)-Cycloalkyl ausgewählt sind,
als Medikament.

10. Produkt, ausgewählt aus der folgenden Liste:
• Methylphenylphosphinsäure-3((E)-styryl)-1H-indazol-5-ylester
• Diphenylphosphinsäure-3((E)-styryl)-1H-indazol-5-ylester
• Methylphenylphosphinsäure-3-(1H-indol-2-yl)-1H-indazol-5-ylester
• Diphenylphosphinsäure-3-(1H-indol-2-yl)-1H-indazol-5-ylester
• Methylphenylphosphinsäure-3-[5-(2-morpholin-4-ylethoxy)-1H-indol-2-yl]-1H-indazol-5-ylester
• Phenylphosphonsäure-3-(1H-indol-2-yl)-1H-indazol-5-ylestermethylester
• Phenylphosphonsäure-3-(1H-indol-2-yl)-1H-indazol-5-ylesterisopropylester
• Phenylphosphonsäurebenzylester-3-(1H-indol-2-yl)-1H-indazol-5-ylester
• Methylphosphonsäure-3-(1H-indol-2-yl)-1H-indazol-5-ylestermethylester
• Phenylphosphonsäuremethylester-3-(1H-pyrrol-2-yl)-1H-indazol-5-ylester in Trifluoracetat-Form
• [3((E)-Styryl)-1H-indazol-5-yl]phosphonsäure-dimethylester
• [3((E)-Styryl)-1H-indazol-5-yl]phosphonsäure-monomethylester
• [3((E)-Styryl)-1H-indazol-5-yl]phosphonsäure-diethylester
• [3-(1H-indol-2-yl)-1H-indazol-5-yl]phosphon-säuremonomethylester
• (3-Thiophen-2-yl-1H-indazol-5-yl)phosphonsäure-monomethylester
• [3-(1H-Pyrrol-2-yl)-1H-indazol-5-yl)phosphon-säuremonomethylester
• Dimethylphosphinsäure-3-(1H-indol-2-yl)-1H-indazol-5-ylester
• Methylphosphonsäuremethylester-3-[5-(2-piperidin-1-ylethoxy)-1H-indol-2-yl]-1H-indazol-5-ylester
• Methylphosphonsäuremono-{3-[5-(2-piperazin-1-ylethoxy)-1H-indol-2-yl]-1H-indazol-5-yl}ester
• Methylphosphonsäuremono-{3-[5-(2-diethylamino-ethoxy)-lH-indol-2-yl]-1H-indazol-5-yl}ester
• Methylphosphonsäure-{3-[5-(2-diethylamino-ethoxy)-1H-indol-2-yl]-1H-indazol-5-yl}ester-methylester
• Phenylphosphonsäureethylester-3-(5-methoxy-1H-pyrrolo[3,2-b]pyridin-2-yl)-1H-indazol-5-yl-ester
• Phenylphosphonsäure-3-(5-methoxy-1H-pyrrolo-[3,2-b]pyridin-2-yl)-1H-indazol-5-ylester-methylester
• Phenylphosphonsäuremethylester-3-styryl-1H-indazol-5-ylester
• Phenylphosphonsäure-3-thiophen-2-yl-1H-indazol-5-ylestermethylester
• Phenylphosphonsäure-3-benzo[b]thiophen-2-yl-1H-indazol-5-ylestermethylester
• Methylphosphonsäuremethylester-3-[5-(2-morpholin-4-ylethoxy)-1H-indol-2-yl]-1H-indazol-5-ylester
• Methylphosphonsäure-3-[6-(2-diethylamino-ethoxy)-1H-indol-2-yl]-1H-indazol-5-ylester-methylester
• Ethylphosphonsäure-3-(1H-indol-2-yl)-1H-indazol-5-ylestermethylester
• Methylthiophosphonsäure-O-[3-(1H-indol-2-yl)-1H-indazol-5-yl]ester-O-methylester
• Cyclohexylphosphonsäure-3-(1H-indol-2-yl)-1H-indazol-5-ylestermethylester
• Phenylphosphonsäuremono-[3-(1H-indol-2-yl)-1H-indazol-5-yl]ester.
